# EUROPEAN PATENT APPLICATION

(11) **EP 4 151 210 A2**
(43) Date of publication of application: **22.03.2023**
(21) Application number: 22188840.7
(22) Date of filing: 10.01.2021
(51) Int. Cl.: A61K 31/4245, A61K 31/444, A61K 31/4745, A61K 31/4985, A61K 31/506, A61K 31/517, A61K 31/519, A61P 3/04, A61P 25/18, A61P 43/00, A61K 47/55, A61K 45/06, C07D 471/04

(54) **PYRIDINE-CARBOLINE DERIVATIVES AS MCHR1 ANTAGONISTS FOR USE IN THERAPY**

(30) Priority: 10.01.2020 US 202062959534 P; 10.01.2020 US 202062959769 P; 21.02.2020 US 202062980053 P; 09.05.2020 US 202063022484 P
(62) Divisional of application: 21738983.2
(71) Applicant: Harmony Biosciences, LLC, Plymouth Meeting, PA 19462 (US)
(72) Inventor: CUI, Wenge, Rensselaer, New York (US); LIU, Shuang, Rensselaer, New York (US); MAKI, John, Rensselaer, New York (US); GUZZO, Peter, R., Rensselaer, New York (US)
(74) Representative: Garner, Stephen

(57) **Abstract**

Provided are therapeutic combinations or formulations of drugs comprising triple monoamine reuptake inhibitors, melanin concentrating hormone receptor 1 (MCHR1) antagonists and diazoxide or its formulations and various combinations thereof, these in combination with other drugs or active agents. Provided are methods for the treatment of various conditions, including genetic confirmed syndromes, and diseases, using therapeutic combinations and formulations of drugs as provided herein. Provided are methods for administering triple monoamine reuptake inhibitors (TRIs), melanin concentrating hormone receptor 1 (MCHR1) antagonists and diazoxide or diazoxide or its formulations, whose dosages are determined using a method as provided herein including empirical methods for safe and predictable titration and to determine the initial therapeutic dose; model -based methods for safe and predictable titration and to determine the initial therapeutic dose and to determine the lowest therapeutic dose or to determine an optimal effective dose, including use of Bayesian pharmacometric models.

## Description

### RELATED APPLICATIONS

This Patent Convention Treaty (PCT) International Application claims the benefit of priority under 35 U.S.C. § 119(e) of U.S. Provisional Application Nos. USSN 62/959,534, filed January 10, 2020; USSN 62/959,769, filed January 10, 2020; USSN 62/980,053, filed February 21, 2020; and, USSN 63/022,484, filed May 9, 2020. The aforementioned applications are expressly incorporated herein by reference in their entirety and for all purposes. All publications, patents, patent applications cited herein are hereby expressly incorporated by reference for all purposes.

### TECHNICAL FIELD

This invention generally relates to pharmacology and pharmacokinetics. In alternative embodiments, provided are therapeutic combinations or formulations of drugs comprising triple monoamine reuptake inhibitors (TRIs) (which inhibits the reuptake of serotonin, dopamine and norepinephrine via blocking central serotonin transporter (SERT), dopamine transporter (DAT) and norepinephrine transporter (NET)), melanin concentrating hormone receptor 1 (MCHR1) antagonists and diazoxide or diazoxide or its formulations, and various combinations thereof, and these in combination with other drugs or active agents. In alternative embodiments, provided are methods for administering triple monoamine reuptake inhibitors (TRIs), melanin concentrating hormone receptor 1 (MCHR1) antagonists or diazoxide or its formulations, whose dosages are determined using a method as provided herein including empirical methods for safe and predictable titration and to determine the initial therapeutic dose; model-based methods for safe and predictable titration and to determine the initial therapeutic dose and to determine the lowest therapeutic dose or to determine an optimal effective dose, including use of Bayesian pharmacometric models. In alternative embodiments, provided are methods for the treatment of various conditions, including genetic confirmed syndromes, and diseases, using therapeutic combinations and formulations of drugs as provided herein. In alternative embodiments, provided are PK-guided, precision dosing methods using the above mentioned pharmacokinetic modeling and simulation methods, or similar modeling and simulation methods, and a population PK/PD model of formula I to treat hyperphagia, hyperphagia in PWS, disruptive mood dysregulation disorder (DMDD), oppositional defiant disorder (ODD), obesity in hypothalamic-injury induced obesity, and binge eating disorder (BED) with an oral dosage of Formula I or deuterated versions thereof.

### BACKGROUND

Prader-Willi syndrome (PWS) is a rare, serious, complex genetic neurodevelopment disorder present in 1/15,000 to 1/30,000 individuals. PWS is caused by the loss of paternally expressed genes on chromosome 15q11 - 13. In approximately 70% of cases, PWS is due to a paternal deletion at 15q11-q13. Approximately 25% of PWS cases are due to maternal uniparental disomy (mUPD), or when both copies of chromosome 15 are inherited from the mother, and 5% to translocations or imprinting center mutations.

Today, the only therapy currently approved by the United States Food and Drug Administration (FDA) to treat PWS is human growth hormone, which is used to increase linear growth. There are no therapies approved to treat the hallmark features of PWS, specifically hyperphagia, anxiety, and obsessive and compulsive symptoms. There is no approved pharmaceutical treatment for hyperphagia or associated behavioral symptoms associated with PWS.

Hypothalamic obesity or Hypothalamic injury-induced obesity (HO or HIO) is caused by injury to the hypothalamus. The most common cause is related to a rare non-cancerous tumor called a craniopharyngioma. When this tumor is removed, the hypothalamus can get damaged leading to the symptoms of hypothalamic obesity, a disorder sharing many aspects of the phenotype of PWS, such as hyperphagia, excessive day time sleepiness, ADHD, autonomic imbalance; growth hormone-, gonadotropins and thyroid-stimulating hormone deficiency.

Narcolepsy, a chronic, disabling neurologic disorder of hypersomnolence affects an estimated 20-67 people per 100,000 worldwide. The onset of narcolepsy most commonly occurs in the second decade of life, though diagnosis is often delayed by several years. Symptoms of narcolepsy include excessive daytime sleepiness (EDS), which, although not specific to narcolepsy, is a characteristic of the disorder present in all patients, as it is a requirement for diagnosis. Cataplexy, an involuntary loss of muscle tone during wakefulness that is typically evoked by strong emotions, occurs in up to 60% of patients. Other symptoms are disturbed night-time sleep; hypnagogic and hypnopompic hallucinations, which occur while falling asleep and waking up, respectively; and sleep paralysis. There is no cure for narcolepsy. Various medications modulating the monoamine systems such as serotonin, dopamine, norepinephrine and histamine, and γ-aminobutyric acid B (GABA_{B}) receptors have been used in clinic to manage the symptoms. It is believed that narcolepsy especially narcolepsy with cataplexy is attributed to the deficiency of orexin/hypocretin.

Getting the optimal dose for an individual patient is very important to achieve the clinical benefit and minimize adverse events. Due to the individual variance in body weight, body composition, metabolic enzyme CYP P450 polymorphism, drug transporters such as PGP polymorphism, drug-drug integration with the use of multiple drugs, it is challenging for physicians to prescribe the optimal dose to a patient and often involve empirical titration which is inefficient to find the optimal dose. It is more challenging for pediatric, intellectually disabled and clinically fragile patients.

The case of tesofensine underscores the importance of dosing to achieve clinical benefit and minimize adverse events. Initial clinical studies with tesofensine using standard dosing showed some promise in managing weight in HIO and hyperphagia in PWS (Saniona Corporation Presentation June 2020). However, disclosed data also showed that the plasma drug concentrations can vary significantly in PWS patients (Saniona Corporation Presentation March 2019). By just using a standard mg/day dosing, 2/3 patients had plasma drug levels exceeding the safety levels and discontinued the treatment due to adverse events (excessive increase of dopamine due to DAT inhibition can cause CNS side effect or exacerbate existing CNS disorders in patients), while the patients with the appropriate plasma drug level had the hyperphagia controlled completely. On the other hand, also in the case of tesofensine, using standard mg/day dosing, lowering the dosing for all patients led to no efficacy.

New paradigms are needed to ensure safe and effective dosage regimens for these high risk patients.

### SUMMARY

In alternative embodiments, provided are therapeutic combinations, pharmaceutical dosage forms, drug delivery devices or products of manufacture, comprising one or more of any of the active agents or drugs comprising:
(a)
   (i) a triple monoamine reuptake inhibitor (TRI) and a melanin concentrating hormone receptor 1 (MCHR1) antagonist or inhibitor,
   (ii) a triple monoamine reuptake inhibitor (TRI) and a diazoxide (or PROGLYCEM^{™}) or a diazoxide Choline Controlled-Release (DCCR formulation),
   (iii) a melanin concentrating hormone receptor 1 (MCHR1) antagonist or inhibitor and a diazoxide (or PROGLYCEM^{™}) or a diazoxide Choline Controlled-Release (DCCR formulation), or
   (iv) a triple monoamine reuptake inhibitor (TRI) a melanin concentrating hormone receptor 1 (MCHR1) antagonist or inhibitor, and, a diazoxide (or PROGLYCEM^{™}) or a diazoxide Choline Controlled-Release (DCCR formulation); or
(b)
   (i) a triple monoamine reuptake inhibitor (TRI),
   (ii) a melanin concentrating hormone receptor 1 (MCHR1) antagonist or inhibitor,
   (iii) a diazoxide Choline Controlled-Release (DCCR formulation), or
(c): (a) or (b) formulated with any one or several of the following drugs or small molecules:
   (1) an unacylated ghrelin (UAG) analog and/or livolitide (also known as AZP-531),
   (2) carbetocin, or DURATOCIN^{™}, PABAL^{™} or LONACTENE^{™},
   (3) oxytocin and/or the precursor oxytocin-neurophysin,
   (4) liraglutide, or VICTOZA^{™} or SAXENDA^{™},
   (5) exenatide, or BYETTA^{™}, BYDUREON^{™}, BYDUREON^{™} or BCISE^{™},
   (6) setmelanotide (also known as IMCIVREE^{™}, RM-493, BIM-22493, IRC-022493, N2-Acetyl-L-arginyl-L-cysteinyl-D-alanyl-L-histidyl-D-phenylalanyl-L-arginyl-L-tryptophyl-L-cysteinamide),
   (7) rimonabant (also known as SR141716) and/or ACOMPLIA^{™}, ZIMULTI^{™},
   (8) a beta adrenergic blocker, wherein optionally the beta adrenergic blocker is or comprises: metoprolol (or LOPRESSOR^{™}, METOLAR XR^{™}, TOPROL X^{™}), atenolol (or TENORMIN^{™}), propranolol (or INDERAL^{™}) and/or nadolol (or CORGARD^{™}), or any combination thereof,
   (9) a melatonin receptor agonist, wherein optionally the melatonin receptor agonist is or comprises: melatonin or N-acetyl-5-methoxy tryptamine, ramelteon (or ROZEREM^{™}) and/or tesimelteon (or HETLIOZ^{™}), or any combination thereof,
   (10) a histamine receptor 1 antagonist, wherein the histamine receptor 1 antagonist is or comprises: doxepin (or SINEQUAN^{™}, QUITAXON^{™} or APONAL^{™}), or doxepin low dose (wherein optionally the low dose is 3 mg or 6 mg per dose, trazadone (or DESYREL^{™}, DESYREL DIVIDOSE^{™} or OLEPTRO^{™}), amitriptyline (or ELAVIL^{™}, PROTANOL^{™}, QUALITRIPTINE^{™}, REDOMEX^{™} or SAROIEN^{™}) or amitriptyline with chlordiazepoxide (MORELIN^{™}, RISTRYL^{™} or SEDANS^{™}), and/or mirtazapine (or REMERON^{™}), or any combination thereof,
   (11) a histamine H3 receptor antagonist or inverse agonist, wherein optionally the H3 receptor antagonist or inverse agonist is or comprises: pitolisant (or tiprolisant, ciproxidine or WAKIX^{™}), thioperamide, clobenpropit, ciproxifan, conessine (or nerine, roquessine, wrightine) and/or betahistine (or SERC^{™}), SUVN-G3031 (Suven Life Sciences Ltd), or any combination thereof,
   (12) modafinil (or PROVIGIL^{™}, ALERTEC^{™} or MODAVIGIL^{™}), and/or amodafonil (or NUVIGIL^{™}),
   (13) human growth hormone (hGH) (or somatotropin) or recombinant forms thereof (or OMNITROPE^{™}, JINTROPIN^{™}, NUTROPIN^{™} or NUTROPIN DEPOT^{™}, HUMATROPE^{™}, GENOTROPIN^{™}, NORDITROPIN^{™} or SAIZEN^{™}), or any combination thereof,
   (14) testosterone and/or 17β-Hydroxyandrost-4-en-3-one,
   (15) progesterone and/or pregn-4-ene-3,20-dione,
   (16) estrogen, estrone, estradiol or estriol, or any combination thereof,
   (17) a thyroid hormone or derivative thereof, wherein optionally the thyroid hormone or derivative thereof is or comprises: triiodothyronine (T3), thyroxine (T4), levothyroxine or L-thyroxine, or any combination thereof,
   (18) chorionic gonadotropin (hCG) hormone or recombinant forms thereof, or NOVAREL^{™} or PREGNYL^{™}, or any combination thereof,
   (19) metformin (or GLUCOPHAGE^{™}) and/or repaglinide (or PRANDIN)^{™},
   (20) an insulin or human insulin or recombinant or analog forms thereof (or ACTRAPID^{™}, HUMALOG^{™}, NOVORAPID^{™}, APIDRA^{™}, LANTUS^{™} or LEVEMIR^{™}) or any combination thereof,
   (21) a glucocorticoid receptor antagonist or an anticorticosteroid, or mifepristone (or RU-486, or MIFEGYNE^{™} or MIFEPREX^{™}), metyrapone (or METOPIRONE^{™}), ketoconazole (or NIZORAL^{™}) or aminoglutethimide (or ELIPTEN^{™}, CYTADREN^{™} or ORIMETEN^{™}),
   (22) a histamine receptor 2 antagonist, wherein optionally the histamine receptor 2 antagonist is or comprises: cimetidine (or TAGAMET^{™}), ranitidine (or ZANTAC^{™}) and/or famotidine (or PEPCID^{™}), or any combination thereof,
   (23) a mood stabilizer, wherein optionally the mood stabilizer is or comprises: gabapentin (or NEURONTIN^{™}); clonazepam (or KLONOPIN^{™} or RIVOTRILF^{™}); valproate, valproic acid, sodium valproate or valproate semisodium (or CONVULEX^{™}, DEPAKOTE^{™}, EPILIM^{™} or STAVZOR^{™}); oxcarbazepine (or TRILEPTAL^{™} or OXTELLAR XR^{™}); lithium or lithium carbonate (or LITHOBID^{™} or LITHOMAX^{™}); topiramate (or TOPAMAX^{™}, TROKENDI XR^{™} or QUDEXY XR^{™}) and/or lamotrigine (or LAMICTAL^{™}), or any combination thereof,
   (24) a neuroleptic, wherein optionally the neuroleptic comprises: risperidone (or Risperdal), aripiprazole (or ABILIFY^{™}), quetiapine (or SEROQUEL^{™}), olanzapine (or ZYPREXA^{™}), ziprasidone (or GEODON^{™}) and/or haloperidol (or HALDOL^{™} or SERENACE^{™}), or any combination thereof,
   (25) quetiapine, or SEROQUEL^{™} or TEMPROLIDE^{™},
   (26) naltrexone (or FEBIA^{™} or VIVITROL^{™}),
   (27) γ-aminobutyric acid (GABA) B (GABA_{B}) receptors modulator such as sodium oxybate (Xyrem^{™},), or controlled-release sodium oxybate (or FT218), or a low sodium oxybate, optionally JZP-258, or baclofen,
   (28) solriamfetol or SUNOS^{™},
   (29) hypocretin/orexin 2 receptor-selective agonists, optionally TAK-925, TAK-994 or TAK-988,
   (30) a selective norepinephrine reuptake inhibitor (NRI), or a selective serotonin reuptake inhibitor (SSRI), or a selective serotonin norepinephrine inhibitor (SNRI), wherein optionally the SSRI or SNRI inhibitor is or comprises reboxetine (or AXS-12, or Edronax^{™}), atomoxetine (or Strattera^{™}), venlafaxine (Effexor^{™} or Effexor XR^{™}), fluoxetine (Prozac^{™}), citalopram (Celexa^{™}), escitalopram (Lexapro), paroxetine (Paxil^{™}), sertraline (Zoloft^{™}), or duloxetine (Cymbalta^{™}),
   (31) an amphetamine, wherein optionally the amphetamine or comprises amphetamine, dextroamphetamine (or Adderall^{™}), dextroamphetamine-amphetamine (Mydayis^{™}) or lisdexamfetamine (or Vyvanse^{™}).
   (32) methylphenidate or Ritalin, Ritalin LA, Concerta, Metadate CD, Methylin, Methylin ER, Daytrana, Quillivant XR, Quillichew ER, Aptensio XR, Cotempla XR-ODT, Jomay PM, or Adhansia XR,
   (33) a tricyclic antidepressant (TCA) wherein optionally the TCA is or comprises imipramine (Tofranil^{™}), or amitriptyline (Elavil^{™}), clomipramine (Anafranil^{™}) or another TCA,
   (34) a monoamine oxidase inhibitor (MAOI) wherein optionally the MAOI is or comprises selegiline (Emsam^{™}), isocarboxazid (Marplan^{™}), phenelzine (Nardil^{™}), and tranylcypromine (Parnate^{™}) or another MAOI,
   (35) THN102, or a combination of modafinil and flecainide,
   (36) an inhibitor of astroglial connexins inhibitor, wherein optionally the inhibitor of astroglial connexins inhibitor is or comprises flecainide,
   (37) a prostaglandin DP1 receptor antagonist, wherein optionally the prostaglandin DP1 receptor antagonist is or comprises ONO-4127Na,
   (38) an opioid, wherein optionally the opioid comprises morphine,
   (39) an anti-obesity medication, wherein optionally the anti-obesity medication comprises lorcaserin (Belviq^{™}), orlistat (Alli^{™}), phentermine and topiramate (Qsymia^{™}), ornaltrexone HCl or bupropion HCl (Contrave^{™}),
   (40) a famesoid X receptor (FXR) agonist, wherein optionally the FXR agonist comprises Obeticholic Acid (OCA), EYP001 (ENYO Pharma), TQA3526, Px-102, Px-104, or tropifexor,
   (41) a PPAR agonist, optionally a PPAR α/δ agonist, or a PPAR-α/γ agonist, wherein optionally the PPAR-α/γ agonist comprises pioglitazone, elafibranor or lanifibranor (or IVA337, Inventiva),
   (42) a CC chemokine receptor CCR2/CCR5 inhibitor, wherein optionally the CC chemokine receptor comprises tropifexor, a combination of tropifexor and cenicriviroc, or cenicriviroc,
   (43) a mitochondrial pyruvate carrier (MPC) inhibitor, wherein optionally the MPC inhibitor comprises MSDC-0602K (Cirius Therapeutics),
   (44) a Fibroblast Growth Factor 19 (FGF19) analogue, wherein optionally the FGF19 analogue comprises aldafermin (or NGM282, NGM Biopharmaceuticals)
   (45) a Fibroblast Growth Factor 21 (FGF21) analogue, wherein optionally the FGF21 analogue comprises a PEGylated Fibroblast Growth Factor 21 analogue, optionally pegbelfermin,
   (46) a thyroid hormone receptor beta (THR-β) agonist, wherein optionally the THR-β agonist comprises resmetirom (MGL-3196, Magrigal Pharmaceuticals) or VK-2890,
   (47) a Stearoyl-CoA desaturase-1 (SCD1) inhibitor, wherein optionally the SCD1 inhibitor comprises aramchol (Galmed Pharmaceuticals),
   (48) an apoptosis signal-regulating kinase 1 (ASK1) inhibitor, wherein optionally the ASK1 inhibitor comprises selonsertib (Gilead Sciences),
   (49) an acetyl-CoA carboxylase (ACC) inhibitor, wherein optionally the ACC inhibitor comprises firsocostat (GS-0976) or MK-4074, or
   (50) any combination of (1) to (49).

In alternative embodiments, the triple monoamine reuptake inhibitor is or comprises:
(a) tesofensine, tesomet or tesofen (Saniona, Ballerup, Denmark),
(b) a [1,2,4]triazolo[1,5-a]pyridinyl-6-yl-substituted tetrahydroisoquinoline, or a salt, solvate, racemate, crystalline form or derivative thereof, optionally as described in USPN 8,802,696, or 7-([1,2,4]triazolo[1,5-a]pyridin-6-yl)-4-(3,4-dichlorophenyl)-1,2,3,4-tetrahydroisoquinoline or a salt, solvate, racemate, crystalline form or derivative thereof, or a compound having the formula (Formula I): 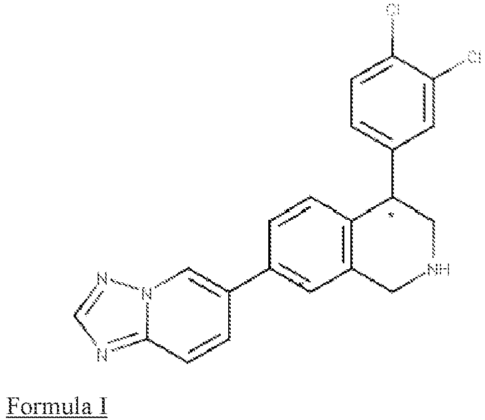 or a pharmaceutically acceptable salt thereof, or an S-enantiomer, (+)-stereoisomer or a (-)-stereoisomer thereof, or a compound in the S or R configuration, or a (S)(+)-stereoisomer or a (R)(-)-stereoisomer thereof,
(c) a deuterated form of a compound or drug of (a) or (b), wherein 1, 2, 3, 4, 5, 6, 7, 8, 9 or 10 or more hydrogen residues are deuterated.

In alternative embodiments, the melanin concentrating hormone receptor 1 (MCHR1) antagonist or inhibitor is or comprises:
(a) GW8564649 (GSK), AZD-1979 (AstraZeneca), AMG-076 (Amgen), BMS-830216 (BMS), ATC-0065 or ATC-0175 (see Chaki et al (2005) CNS Drug Reviews vol 11(4):341-52), GW-803430 or GW-3430 (see Gehlert et al (2009) J Pharm and Experimental Therapeutics, vol 329(2):429-38), NGD-4715 (Ligand Pharmaceuticals), SNAP-7941 (see Klemenhagen et al (2007) J Pharm and Experimental Therapeutics, vol 321 (1): 237-48), T-226 or T-296 (see Takekawa et al (2002) Eur J Pharm vol 438(3):129-35), or any combination thereof,
(b) a compound (designated Formula II) having the formula: 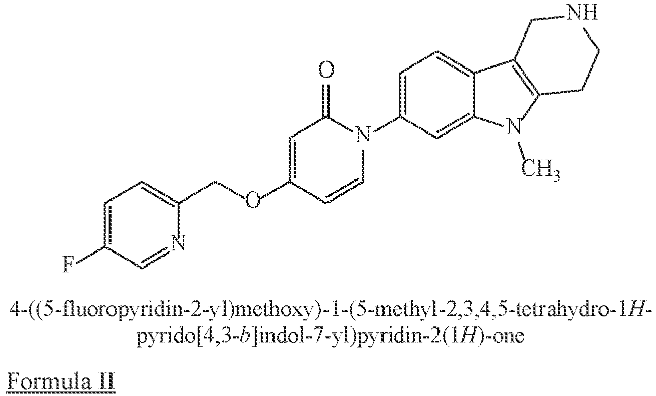 , or
   a (1-azinone)-substituted pyridoindole as described in USPN 8,716, 308, or a compound having the formula: 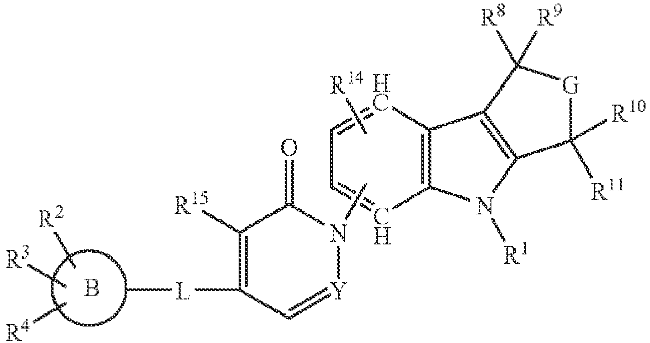
   wherein R₁ is H or optionally substituted alkyl;
   R₂, R₃, R₄ are each independently selected from H, -O-alkyl, -S-alkyl, alkyl, halo, -CF₃, and -CN;
   G is -CR¹²R¹³-NR⁵- or -NR⁵-CR¹²R¹³; R⁵ is H, optionally substituted alkyl, optionally substituted heterocycle, -C(=O)-R⁶,-C(=O)-O-R⁷. or -C(=O)-NR¹⁹R²⁰; R⁶ and R⁷ are each optionally substituted alkyl or optionally substituted heterocycle;
   R₈, R₉, R₁₀, R₁₁, R₁₂, R₁₃, R₁₉ and R₂₀ are each independently selected from H or optionally substituted alkyl; R¹⁴ and R¹⁵ are each independently H or halogen; Y is CH; L is -CH₂-O-, -CH₂CH₂-, -CH=CH- or a bond; and B is aryl or heteroaryl or cycloalkyl; with the proviso that, when L is a direct bond, B cannot be unsubstituted heteroaryl or heteroaryl monosubstituted with fluorine, or
(c) a deuterated form of a compound or drug of (a) or (b),
   and optionally the therapeutic combination, pharmaceutical dosage form, drug delivery device or product of manufacture further comprises a N-acetyltransferase 2 (NAT2) or arylamine N-acetyltransferase inhibitor, optionally acetaminophen, N-acetyl-para-aminophenol (APAP), or paracetamol (or TYLENOL^{™} or PANADOL^{™}), in combination with any compound of (b) or a deuterated form of a compound or drug of (b), wherein optionally in this combination (b) is Formula II.

In alternative embodiments, two or three or more of the drugs or active agents are formulated as separate compositions, or two or three or more of the drugs or active agents are formulated into one composition or drug formulation (two or more drugs or active agents are formulated together).

In alternative embodiments, one or two or more of the drugs or active agents are packaged individually, or are packaged together, or packaged in any combination, in a single package, a plurality of packages or packettes, or a blister packet, lidded blister or blister card or packets, or a shrink wrap.

In alternative embodiments, one or two or more or all of the drugs or active agents are formulated or manufactured as a parenteral formulation, an aqueous solution, a liposome, an injectable solution, a tablet, a pill, a lozenge, a capsule, a caplet, a spray, a sachet, an inhalant, a powder, a freeze-dried powder, an inhalant, a patch, a gel, a geltab, a nanosuspension, a nanoparticle, a nanoliposome, a microgel, a pellet, a suppository or any combination thereof, and optionally the drug delivery device or product of manufacture is or comprises an implant.

In alternative embodiments, the one or two or more or all of the drugs or active agents are formulated or manufactured together in one parenteral formulation, one aqueous solution, one liposome, one injectable solution, one freeze-dried powder, one feed, one food, one food supplement, one pellet, one lozenge, one liquid, one elixir, one aerosol, one inhalant, one adhesive, one spray, one powder, one freeze-dried powder, one patch, one tablet, one pill, one capsule, one gel, one geltab, one lozenge, one caplet, one nanosuspension, one nanoparticle, one nanoliposome, one microgel or one suppository.

In alternative embodiments, the one or two or more or all of the drugs or active agents are packaged in dosages that match a chrono-dosing regimen to match an optimal dose for the time of day.

In alternative embodiments, the drugs or active agents comprise:
(a) a triple monoamine reuptake inhibitor or a [1,2,4]triazolo[1,5-a]pyridinyl-6-yl-substituted tetrahydroisoquinoline derivative, optionally a compound of Formula I, and a histamine receptor 1 antagonist, optionally doxepin;
(b) a triple monoamine reuptake inhibitor or a [1,2,4]triazolo[1,5-a]pyridinyl-6-yl-substituted tetrahydroisoquinoline derivative, optionally a compound of Formula I , and naltrexone;
(c) a triple monoamine reuptake inhibitor or a [1,2,4]triazolo[1,5-a]pyridinyl-6-yl-substituted tetrahydroisoquinoline derivative, optionally a compound of Formula I, and a histamine receptor 1 antagonist, optionally doxepin, and naltrexone;
(d) a triple monoamine reuptake inhibitor or a [1,2,4]triazolo[1,5-a]pyridinyl-6-yl-substituted tetrahydroisoquinoline derivative, optionally a compound of Formula I , and a diazoxide (or PROGLYCEM^{™}) or a diazoxide Choline Controlled-Release (DCCR formulation);
(e) a triple monoamine reuptake inhibitor or a [1,2,4]triazolo[1,5-a]pyridinyl-6-yl-substituted tetrahydroisoquinoline derivative, optionally a compound of Formula I , and a diazoxide (or PROGLYCEM^{™}) or a diazoxide Choline Controlled-Release (DCCR formulation), and a melatonin receptor agonist, optionally melatonin or N-acetyl-5-methoxy tryptamine, ramelteon (or ROZEREM^{™}) and/or tesimelteon (or HETLIOZ^{™}), or any combination thereof,
(f) a triple monoamine reuptake inhibitor or a [1,2,4]triazolo[1,5-a]pyridinyl-6-yl-substituted tetrahydroisoquinoline derivative, optionally a compound of Formula I, and a melanin concentrating hormone receptor 1 (MCHR1) antagonist or inhibitor, optionally a compound of Formula II, or any combination thereof;
(g) a triple monoamine reuptake inhibitor or a [1,2,4]triazolo[1,5-a]pyridinyl-6-yl-substituted tetrahydroisoquinoline derivative, optionally a compound of Formula I, and a beta blocker, optionally, atenolol (TENORMIN^{™}), bisoprolol (CARDICOR^{™}, EMCOR^{™}), or metoprolol (BETALOC^{™}, LOPRESOR^{™}, TOPROL XL^{™}), or any combination thereof; and/or
(h) any combination of (a) to (g).

In alternative embodiments, provided are pharmaceutical compositions, drug or formulations comprising a compound having the formula: 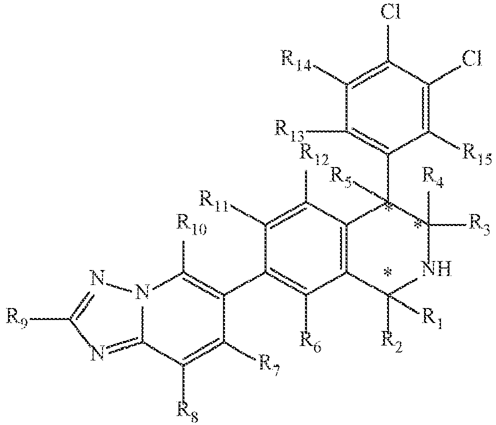
wherein at least one of R₁ through R₁₅ is -D (deuterium), or all of **R₁** through R₁₅ is -D,
and optionally the carbon atom designated ^{∗} is in an R or an S configuration if it is a stereocenter;
or a pharmaceutically acceptable salt thereof.

In alternative embodiments, provided herein is a compound having the formula:
(a) a 7-([1,2,4]triazolo[1,5-a]pyridin-6-yl)-4-(3,4-dichlorophenyl)-1,2,3,4-tetrahydroisoquinoline-1,1,3,3,4,8-d6 compound having the formula: 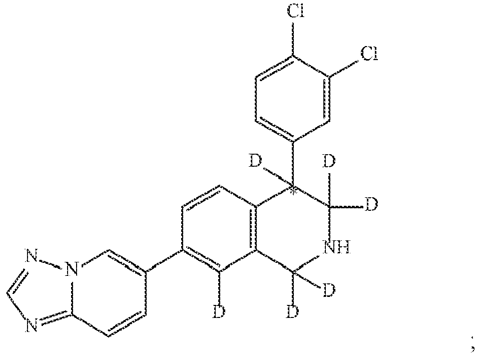
(b) a 7-([1,2,4]triazolo[1,5-a]pyridin-6-yl)-4-(3,4-dichlorophenyl)-1,2,3,4-tetrahydroisoquinolme-1,1,3,3,4-d5 compound having the formula: 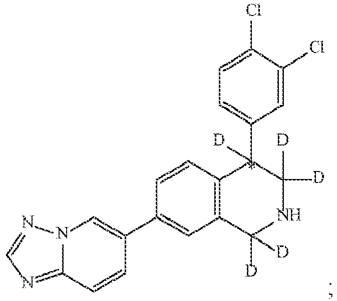
(c) a7-([1,2,4]triazolo[1,5-a]pyridin-6-yl)-4-(3,4-dichlorophenyl)-1,2,3,4-tetrahydroisoquinoline-1,1-d2 compound having the formula: 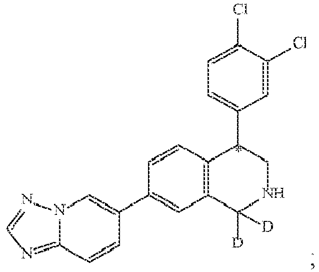
(d) a 7-([1,2,4]triazolo[1,5-a]pyridin-6-yl)-4-(3,4-dichlorophenyl)-1,2,3,4-tetrahydroisoquinoline-3,3-d2 compound having the formula: 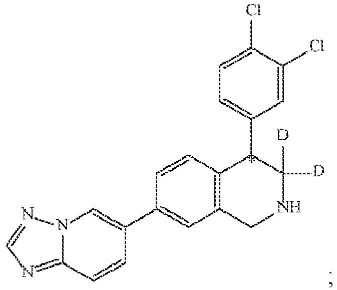
(e) a 7-([1,2,4]triazolo[1,5-a]pyridin-6-yl)-4-(3,4-dichlorophenyl)-1,2,3,4-tetrahydroisoquinoline-4-d compound having the formula: 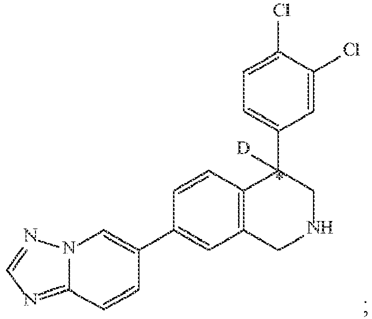
(f) a 7-([1,2,4]triazolo[1,5-a]pyridin-6-yl)-4-(3,4-dichlorophenyl)-1,2,3,4-tetrahydroisoquinoline-8-d compound having the formula: 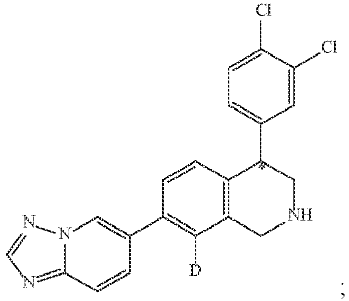 or,
(g) a 7-([1,2,4]triazolo[1,5-a]pyridin-6-yl)-4-(3,4-dichlorophenyl)-1,2,3,4-tetrahydroisoquinoline-3,3,4-d3 compound having the formula:

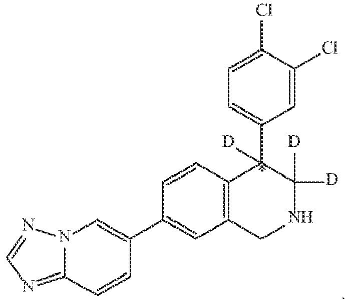

In alternative embodiments, provided are pharmaceutical compositions, drugs or formulations comprising a compound having the formula: 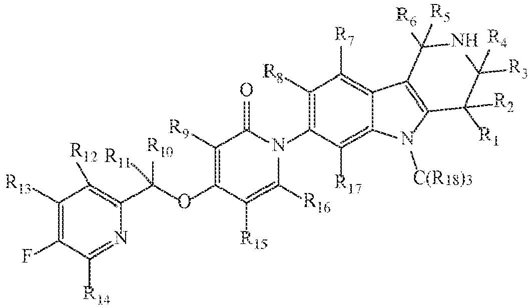
wherein at least one of R₁ through R₁₈ is -D (deuterium), or all of R₁ through R₁₈ is -D;
or a pharmaceutically acceptable salt thereof.

In alternative embodiments, provided are pharmaceutical compositions, drugs or formulations comprising a compound having the formula:

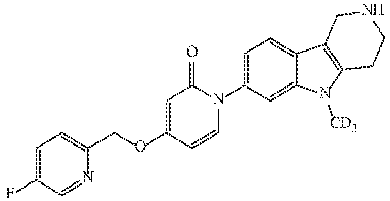

### 4-((5-fluoropyridin-2-yl)methoxy)-1-(5-(methyl-d₃)-2,3,4,5-tetrahydro-1H-pyrido[4,3-b]indol-7-yl)pyridin-2(1H)-one ,

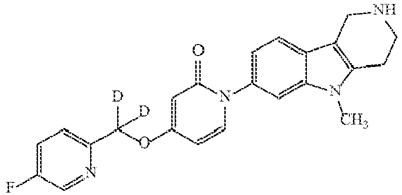

### 4-((5-fluoropyridin-2-yl)methoxy-d₂)-1-(5-methyl-2,3,4,5-tetrahydro-1H-pyrido[4,3-b]indol-7-yl)pyridin-2(1H)-one ,

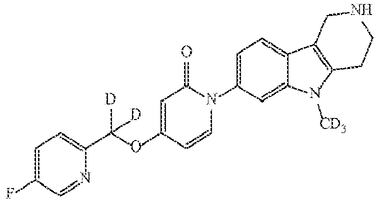

### 4-((5-fluoropyridin-2-yl)methoxy-d₂)-1-(5-(methyl-d₃)-2,3,4,5-tetrahydro-1H-pyrido[4,3-b]indol-7-yl)pyridin-2(1H)-one ,

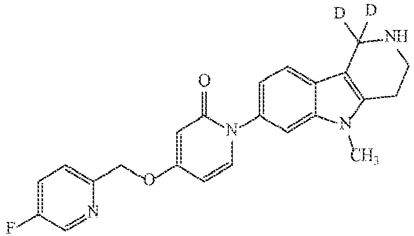

### 4-((5-fluoropyridin-2-yl)methoxy)-1-(5-methyl-2,3,4,5-tetrahydro-1H-pyrido[4,3-b]indol-7-yl-1,1-d₂)pyridin-2(1H)-one ,

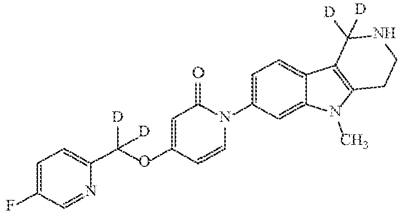

### 4-((3-fluoropyridin-2-yl)methoxy-d₂)-1-(5-methyl-2,3,4,5-tetrahydro-1H-pyrido[4,3-b]indol-7-yl-1,1-d₂)pyridin-2(1H)-one ,

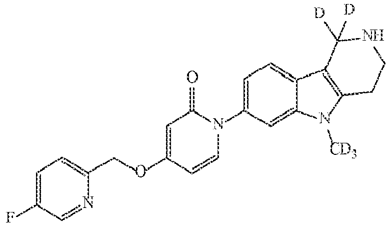

### 4-((5-fluoropyridin-2-yl)methoxy)-1-(5-(methyl-d₃)-2,3,4,5-tetrahydro-1H-pyrido[4,3-b]indol-7-yl-1,1-d₂)pyridin-2(1H)-one

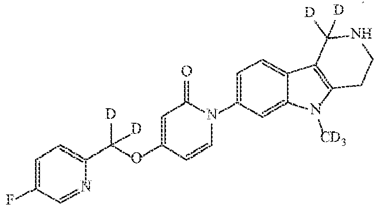

### 4-((5-fluoropyridin-2-yl)methoxy-d₂)-1-(5-(methyl-d₃)-2,3,4,5-tetrahydro-1H-pyrido[4,3-b]indol-7-yl-1,1-d₂)pyridin-2(1H)-one ,

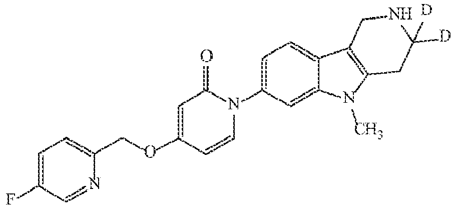

### 4-((5-fluoropyridin-2-yl)methoxy)-1-(5-methyl-2,3,4,5-tetrahydro-1H-pyrido[4,3-b]indol-7-yl-3,3-d₂)pyridin-2(1H)-one ,

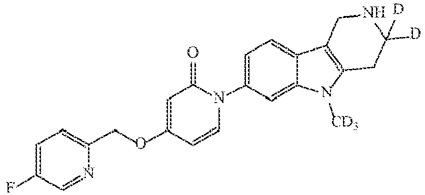

### 4-((5-fluoropyridin-2-yl)methoxy)-1-(5-(methyl-d₃)-2,3,4,5-tetrahydro-1H-pyrido[4,3-b]indol-7-yl-3,3-d₂)pyridin-2(1H)-one ,

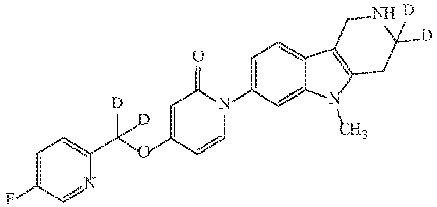

### 4-((5-fluoropyridin-2-yl)methoxy-d₂)-1-(5-methyl-2,3,4,5-tetrahydro-1H-pyrido[4,3-b]indol-7-yl-3,3-d₂)pyridin-2(1H)-one ,

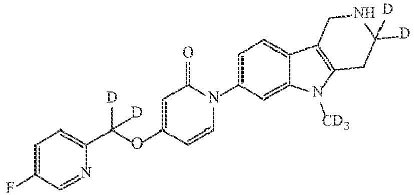

### 4-((5-fluoropyridin-2-yl)methoxy-d₂)-1-(5-(methyl-d₃)-2,3,4,5-tetrahydro-1H-pyrido[4,3-b]indol-7-yl-3,3-d₂)pyridin-2(1H)-one ,

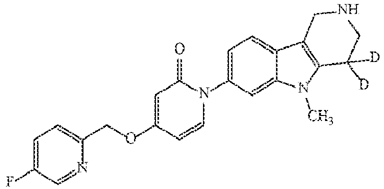

### 4-((5-fluoropyridin-2-yl)methoxy)-1-(5-methyl-2,3,4,5-tetrahydro-1H-pyrido[4,3-b]indol-7-yl-4,4-d₂)pyridin-2(1H)-one ,

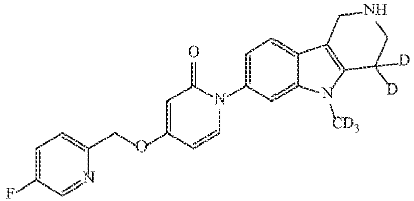

### 4-((5-fluoropyridin-2-yl)methoxy)-1-(5-(methyl-d₃)-2,3,4,5-tetrahydro-1H-pyrido[4,3-b]indol-7-yl-4,4-d₂)pyridin-2(1H)-one

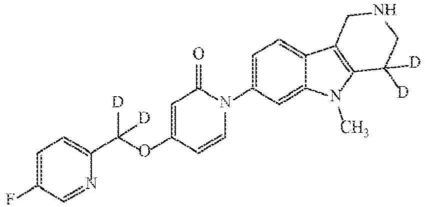

### 4-((5-fluoropyridin-2-yl)methoxy-d₂)-1-(5-methyl-2,3,4,5-tetrahydro-1H-pyrido[4,3-b]indol-7-yl-4,4-d₂)pyridin-2(1H)-one ,

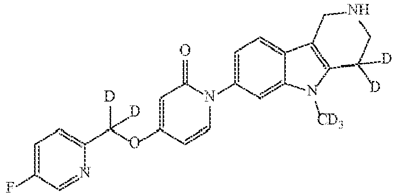

### 4-((5-fluoropyridin-2-yl)methoxy-d₂)-1-(5-(methyl-d₃)-2,3,4,5-tetrahydro-1H-pyrido[4,3-b]indol-7-yl-4,4-d₂)pyridin-2(1H)-one ,

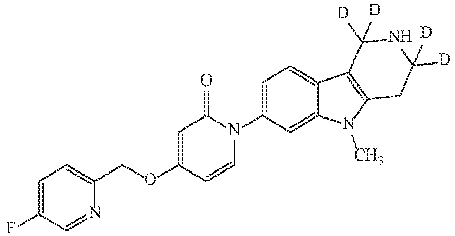

### 4-((5-fluoropyridin-2-yl)methoxy)-1-(5-methyl-2,3,4,5-tetrahydro-1H-pyrido[4,3-b]indol-7-yl-1,1,3,3-d₄)pyridin-2(1H)-one

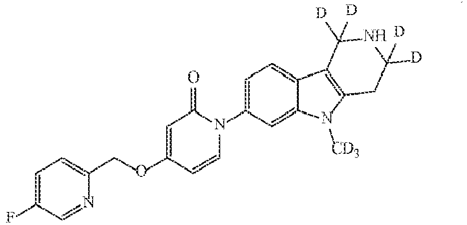

### 4-((5-fluoropyridin-2-yl)methoxy)-1-(5-(methyl-d₃)-2,3,4,5-tetrahydro-1H-pyrido[4,3-b]indol-7-yl-1,1,3,3-d₄)pyridin-2(1H)-one ,

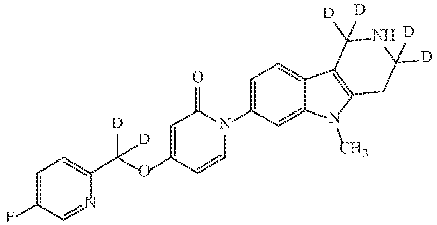

### 4-((5-fluoropyridin-2-yl)-methoxy-d₂)-1-(5-methyl-2,3,4,5-tetrahydro-1H-pyrido[4,3-b]indol-7-yl-1,1,3,3-d₄)pyridin-2(1H)-one ,

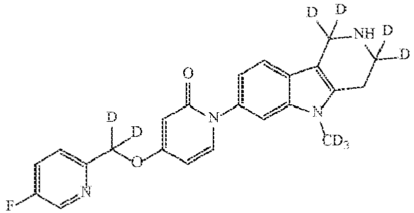

### 4-((5-fluoropyridin-2-yl)methoxy-d₂)-1-(5-(methyl-d₃)-2,3,4,5-tetrahydro)-1H-pyrido[4,3-b]indol-7-yl-1,1,3,3-d₄)pyridin-2(1H)-one ,

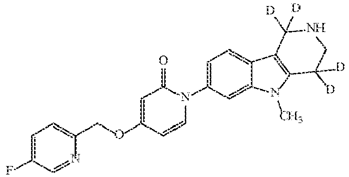

### 4-((5-fluoropyridin-2-yl)methoxy)-1-(5-methyl-2,3,4,5-tetrahydro-1H-pyrido[4,3-b]indol-7-yl-1,1,4,4-d₄)pyridin-2(1H)-one ,

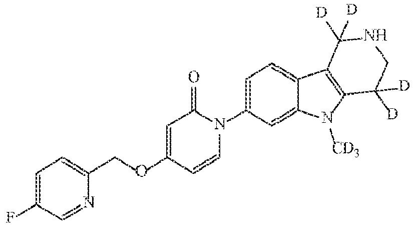

### 4-((5-fluoropyridin-2-yl)methoxy)-1-(5-(methyl-d₃)-2,3,4,5-tetrahydro-1H-pyrido[4,3-b]indol-7-yl-1,1,4,4-d₄)pyridin-2(1H)-one ,

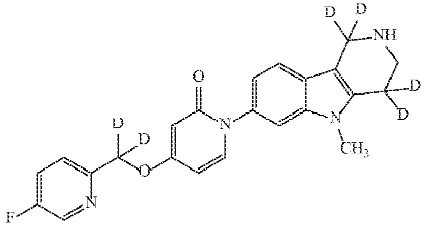

### 4-((5-fluoropyridin-2-yl)methoxy-d₂)-1-(5-methyl-2,3,4,5-tetrahydro-1H-pyrido[4,3-b]indol-7-yl-1,1,4,4-d₄)pyridin-2(1H)-one ,

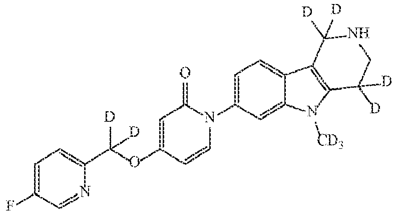

### 4-((5-fluoropyridin-2-yl)methoxy-d₂)-1-(5-(methyl-d₃)-2,3,4,5-tetrahydro-1H-pyrido[4,3-b]indol-7-yl-1,1,4,4-d₄)pyridin-2(1H)-one ,

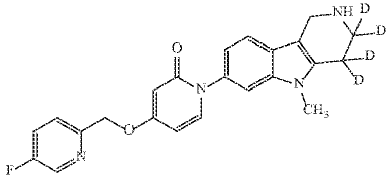

### 4-((5-fluoropyridin-2-yl)methoxy)-1-(5-methyl-2,3,4,5-tetrahydro-1H-pyrido[4,3-b]indol-7-yl-3,3,4,4-d₄)pyridin-2(1H)-one ,

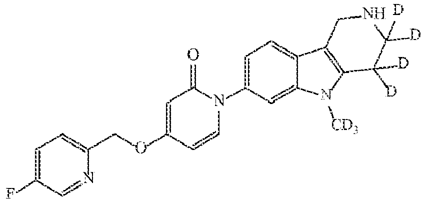

### 4-((5-fluoropyridin-2-yl)methoxy)-1-(5-(methyl-d₃)-2,3,4,5-tetrahydro-1H-pyrido[4,3-b]indol-7-yl-3,3,4,4-d₄)pyridin-2(1H)-one ,

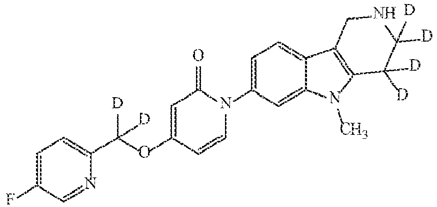

### 4-((5-fluoropyridin-2yl)methoxy-d₂)-1-(5-methyl-2,3,4,5-tetrahydro-1H-pyrido[4,3-b]indol-7-yl-3,3,4,4-d₄)pyridin-2(1H)-one ,

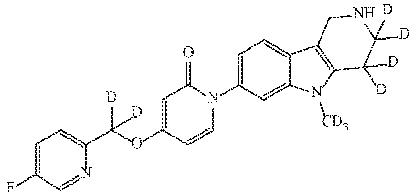

### 4-((5-fluoropyridin-2-yl)methoxy-d₂)-1-(5-(methyl-d₃)-2,3,4,5-tetrahydro-1H-pyrido[4,3-b]indol-7-yl-3,3,4,4-d₄)pyridin-2(1H)-one ,

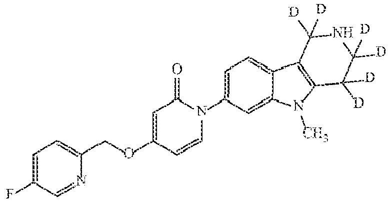

### 4-((5-fluoropyridin-2-yl)methoxy)-1-(5-methyl-2,3,4,5-tetrahydro-1H-pyrido[4,3-b]indol-7-yl-1,1,3,3,4,4-d₆)pyridin-2(1H)-one ,

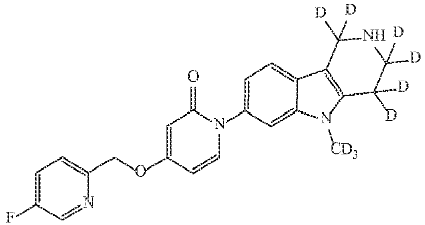

### 4-((5-fluoropyridin-2-yl)methoxy)-1-(5-(methyl-d₃)-2,3,4,5-tetrahydro-1H-pyrido[4,3-b]indol-7-yl-1,1,3,3,4,4-d₆)pyridin-2(1H)-one ,

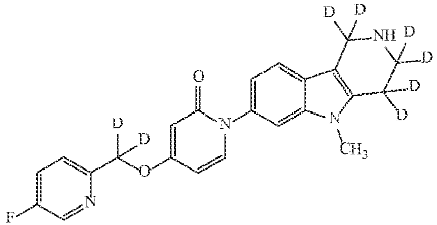

### 4-((5-fluoropyridin-2-yl)methoxy-d₂)-1-(5-methyl-2,3,4,5-tetrahydro-1H-pyrido[4,3-b]indol-7-yl-1,1,3,3,4,4-d₆)pyridin-2(1H)-one ,

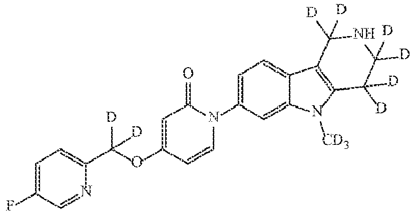

### 4-((5-fluoropyridin-2-yl)methoxy-d₂)-1-(5-(methyl-d₃)-2,3,4,5-tetrahydro-1H-pyrido[4,3-b]indol-7-yl-1,1,3,3,4,4-d₆)pyrido-2(1H)-one , or

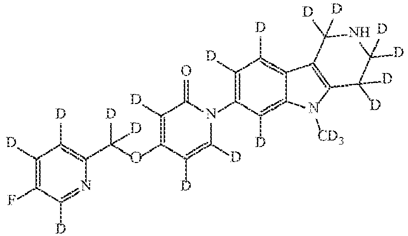

### 4-((5-fluoropyridin-2-yl-3,4,6-d₃)methoxy-d₂)-1-(5-(methyl-d₃)-2,3,4,5-tetrahydro-1H-pyrido[4,3-b]indol-7-yl-1,1,3,3,4,4,6,8,9-d₉)pyridin-2(1H)-one-3,5,6-d₃.

In alternative embodiments, provided are methods for treating, ameliorating, slowing the progress of, reducing the symptoms of, reducing adverse events associated with, or preventing, a disease or condition comprising or associated with:
hyperphagia (optionally as assessed by HQ-CT),
moderate to severe binge eating disorder (BED),
bulimia nervosa,
management of obesity, optionally further comprising management of weight loss and maintenance of weight loss, or optionally further comprising as an adjunct to a reduced-calorie diet or for increased physical activity for chronic weight management,
early onset morbid obesity,
non-Alcoholic Steatohepatitis (NASH),
non-alcoholic fatty liver disease (NAFLD),
Primary sclerosing cholangitis (PSC),
Primary biliary cholangitis liver (PBC),
inflammatory bowel disease (IBD), or irritable bowel syndrome (IBS), type II diabetes,
hypothalamic injury-induced obesity,
obsessive-compulsive disorder (OCD),
disruptive mood dysregulation disorder (DMDD),
oppositional defiant disorder (ODD),
excoriation,
trichotillomania,
intermittent explosive disorder (IED),
excessive daytime sleepiness (EDS),
excessive daytime sleepiness associated with narcolepsy and sleep apnea,
excessive daytime sleepiness associated with narcolepsy,
excessive daytime sleepiness associated with central or obstructive sleep apnea,
narcolepsy,
narcolepsy type 1 (NT1) according to the International Classification of Sleep Disorders-Third Edition (ICSD-3),
narcolepsy with cataplexy,
narcolepsy type 2 (NT2) according to ICSD-3,
narcolepsy without cataplexy,
cataplexy,
idiopathic hypersomnia,
rapid eye movement (REM) sleep behavior disorder,seizures,
epilepsy, an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
major depressive disorder (MDD),
treatment resistant depression (TRD),
negative symptoms of schizophrenia,
Parkinson's Disease,
MDD Associated with Parkinson's Disease,
general anxiety,
Social Anxiety Disorder (Social Phobia),
Fibromyalgia (FM),
diabetic neuropathy.
lower back pain,
chronic fatigue syndrome,
attention deficit hyperactivity disorder (ADHD),
autism,
Asperger spectrum, a genetically confirmed disease or syndrome, wherein optionally the genetically confirmed disease or syndrome is or comprises Prader-Willi Syndrome, Bardet Biedl Syndrome, Smith-Magenis Syndrome, 1p36 deletion syndrome, 16p11.2 deletion syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,
wherein optionally reducing adverse events comprises reducing psychosis, serotonin syndrome, tachycardia or postural orthostatic tachycardia syndrome,
the method comprising:
(a)
   (i) providing or having provided a therapeutic combination, pharmaceutical dosage form, drug delivery device or product of manufacture as provided herein, or a pharmaceutical composition as provided herein, and
   (ii) administering to or implanting into an individual in need thereof the therapeutic combination, pharmaceutical dosage form, drug delivery device or product of manufacture, or
(b) administering to or implanting into an individual in need thereof a therapeutically effective dose of the therapeutic combination, pharmaceutical dosage form, drug delivery device or product of manufacture as provided herein.

In alternative embodiments of methods as provided herein:
- the drug, or therapeutic combination, pharmaceutical dosage form, is administered orally, parenterally, by inhalation spray, nasally, topically, intrathecally, intrathecally, intracerebrally, epidurally, intracranially or rectally, and optionally parenteral administration comprises administration intrathecally, intracerebrally or epidurally (into a intrathecal, intracerebral, epidural space), subcutaneously, intravenously, intramuscularly and/or intraarterially,
- the drug, or therapeutic combination, pharmaceutical dosage form, is administered to achieve a therapeutic level range of plasma concentration at a steady state, and wherein:
   the therapeutic level range of plasma concentration for the [1,2,4]triazolo[1,5-a]pyridinyl-6-yl-substituted tetrahydroisoquinoline derivative, or a compound of Formula I, is between about: 50 to 4000 ng/ml; 100 to 3000 ng/ml; 250 to 1600 ng/ml; 500 to 1400 ng/ml, 600 to 1300 ng/ml, 800 to 1250 ng/ml, 1000 ng/ml to 1250 ng/ml, 1250 to 1500 ng/ml, 1500 to 2000 ng/ml, 2000 to 2500 ng/ml; 2500 to 3000 ng/ml; 3000 to 3500 ng/ml; or, 3500 to 4000 ng/ml;
   the therapeutic level range of plasma concentration for the (1-azinone)-substituted pyridoindole, or a compound of Formula II, is between about: 5 to 4000 ng/ml, 10 to 1000 ng/ml, 10 to 500 ng/ml, 25 to 500 ng/ml, 25 to 250 ng/ml, 50 to 500 ng/ml, 50 to 1000 ng/ml, 50 to 500 ng/ml, 100 to 1000 ng/ml, 100 to 500 ng/ml, 200 to 1000 ng/ml, 500 to 1000 ng/ml, 1000 to 2000 ng/ml, 2000 to 3000 ng/ml, or 3000 to 4000 ng/ml,
   the therapeutic level range of plasma concentration for tesofensine is between about: 2 ng to 50 ng/ml, 5 to 20 ng/ml, 6.5 to 15 ng/ml, 8 to 12 ng/ml, or about 10 ng/ml,
   the therapeutic level range of plasma concentration for diazoxide is between about: 10 ng/ml to 100 ng/ml, 20 ng/ml to 80 ng/ml, 30 to 50 ng/ml, or about 40 ng/ml;
- in alternative embodiments of methods as provided herein, the steady-state concentration is the time during which the concentration of the drug in the body stays consistent; for most drugs, the time to reach steady state is three to five half-lives if the drug is given at regular intervals,
- in alternative embodiments of methods as provided herein, the plasma concentration for the drug, or therapeutic combination, pharmaceutical dosage form is:
   (a) the trough level or trough concentration (C_{trough}), or the lowest concentration reached by the drug, or therapeutic combination or pharmaceutical dosage form before a second or next dose is administered, or
   (b) determined from blood samples taken between about 0.5 hours to 24 hours, or 4 to 12 hours, or 1, 2, 3, 4, 5, 6, 7, 8, 9, or 10 or more hours, after the last dose or administration of the drug, or therapeutic combination, pharmaceutical dosage form,
- the drug, therapeutic combination, or pharmaceutical dosage form, is administered to achieve a therapeutic level range of plasma concentration (optionally human plasma concentration) at a steady state, and wherein:
   (a) the therapeutic level range of plasma concentration for a compound of Formula I, is between about: 250 to 1600 ng/ml; 500 to 1400 ng/ml, 600 to 1300 ng/ml, 800 to 1250 ng/ml, 1000 ng/ml to 1250 ng/ml, 1250 to 1500 ng/ml, 1500 to 2000 ng/ml, 2000 to 2500 ng/ml; or 2500 to 3000 ng/ml;
   (b) the therapeutic level range of plasma concentration for a compound of Formula I, is between about: 600 to 1300 ng/ml, 800 to 1250 ng/ml, 1000 ng/ml to 1250 ng/ml, 1250 to 1500 ng/ml, 1500 to 2000 ng/ml, or 2000 to 2500 ng/ml; or
   (c) the therapeutic level range of plasma concentration for a compound of Formula I, is between about: 600 to 1300 ng/ml, 800 to 1250 ng/ml, 1000 ng/ml to 1250 ng/ml, or 1250 to 1500 ng/ml;
- in alternative embodiments,the drug, or therapeutic combination, pharmaceutical dosage form, is administered to achieve a daytime therapeutic level range between about 8 to 12 hour time-averaged plasma concentration, wherein the plasma concentration is measured after achieving steady state, and wherein:
   the therapeutic level range of daytime 12-hour time-averaged plasma concentration for the [1,2,4]triazolo[1,5-a]pyridinyl-6-yl-substituted tetrahydroisoquinoline derivative, or a compound of Formula I, is between about: 50 to 4000 ng/ml; 100 to 3000 ng/ml; 250 to 1600 ng/ml; 500 to 1400 ng/ml, 600 to 1300 ng/ml, 800 to 1250 ng/ml, 1000 ng/ml to 1250 ng/ml, 1250 to 1500 ng/ml, 1500 to 2000 ng/ml, 2000 to 2500 ng/ml; 2500 to 3000 ng/ml; 3000 to 3500 ng/ml; or, 3500 to 4000 ng/ml;
   the therapeutic level range of daytime 12-hour time-average plasma concentration for the (1-azinone)-substituted pyridoindole, or a compound of Formula II, is between about: 5 to 4000 ng/ml, 10 to 1000 ng/ml, 10 to 500 ng/ml, 25 to 500 ng/ml, 25 to 250 ng/ml, 50 to 500 ng/ml, 50 to 1000 ng/ml, 50 to 500 ng/ml, 100 to 1000 ng/ml, 100 to 500 ng/ml, 200 to 1000 ng/ml, 500 to 1000 ng/ml, 1000 to 2000 ng/ml, 2000 to 3000 ng/ml, or 3000 to 4000 ng/ml,
   the therapeutic level range of daytime 12-hour time-averaged plasma concentration for tesofensine is between about: 2 ng to 50 ng/ml, 5 to 20 ng/ml, 6.5 to 15 ng/ml, 8 to 12 ng/ml, or about 10 ng/ml, or
   the therapeutic level range of daytime 12-hour time-average plasma concentration for diazoxide is between about: 10 ng/ to 100 ng/ml, 20 to 80 ng/ml, 30 to 50 ng/ml, or about 40ng/ml;
- each drug or active agent of the therapeutic combination, pharmaceutical dosage form, drug delivery device or product of manufacture is delivered to the individual simultaneously, or separately, wherein optionally one or each drug or active agent of the therapeutic combination, pharmaceutical dosage form, drug delivery device or product of manufacture is administered in a chronodosed regimen, and/or
- the therapeutic combination, pharmaceutical dosage form, drug delivery device or product of manufacture comprises Formula I, or a deuterated derivative of Formula I.

In alternative embodiments, provided are uses of a therapeutic combination, pharmaceutical dosage form, drug delivery device or product of manufacture as provided herein for treating, ameliorating, slowing the progress of, reducing the symptoms of, reducing adverse events associated with, or preventing, a disease or condition comprising or associated with:
hyperphagia (optionally as assessed by HQ-CT),
moderate to severe binge eating disorder (BED),
bulimia nervosa,
management of obesity, optionally further comprising management of weight loss and maintenance of weight loss, or optionally further comprising as an adjunct to a reduced-calorie diet or for increased physical activity for chronic weight management,
early onset morbid obesity,
non-Alcoholic Steatohepatitis (NASH), non-alcoholic fatty liver disease (NAFLD),
Primary sclerosing cholangitis (PSC),
Primary biliary cholangitis liver (PBC),inflammatory bowel disease (IBD), or irritable bowel syndrome (IBS),
type II diabetes,
hypothalamic injury-induced obesity,
obsessive-compulsive disorder (OCD),
disruptive mood dysregulation disorder (DMDD),
oppositional defiant disorder (ODD),
excoriation,
trichotillomania,
intermittent explosive disorder (IED),
excessive daytime sleepiness (EDS),
excessive daytime sleepiness associated with narcolepsy and sleep apnea, excessive daytime sleepiness associated with narcolepsy,
excessive daytime sleepiness associated with central or obstructive sleep apnea,
narcolepsy,
narcolepsy type 1 (NT1) according to the International Classification of Sleep Disorders-Third Edition (ICSD-3),
narcolepsy with cataplexy,
narcolepsy type 2 (NT2) according to ICSD-3,
narcolepsy without cataplexy,
cataplexy,
idiopathic hypersomnia,
rapid eye movement (REM) sleep behavior disorder,
seizures,
epilepsy, an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
major depressive disorder (MDD),
treatment resistant depression (TRD),
negative symptoms of schizophrenia,
Parkinson's Disease,
MDD Associated with Parkinson's Disease,
general anxiety,
Social Anxiety Disorder (Social Phobia),
Fibromyalgia (FM),
diabetic neuropathy.
lower back pain,
chronic fatigue syndrome,
attention deficit hyperactivity disorder (ADHD),
autism,
Asperger spectrum,
a genetically confirmed disease or syndrome, wherein optionally the genetically confirmed disease or syndrome is or comprises Prader-Willi Syndrome, Bardet Biedl Syndrome, Smith-Magenis Syndrome, lp36 deletion syndrome, 16p11.2 deletion syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,
wherein optionally reducing adverse events comprises reducing psychosis, serotonin syndrome, tachycardia or postural orthostatic tachycardia syndrome.

In alternative embodiments, the therapeutic combination, pharmaceutical dosage form, drug delivery device or product of manufacture comprises Formula I, or a deuterated derivative of Formula I.

In alternative embodiments, provided are therapeutic combinations, pharmaceutical dosage forms, drug delivery devices or product of manufactures as provided herein for use in treating, ameliorating, slowing the progress of, reducing the symptoms of, reducing adverse events associated with, or preventing, a disease or a condition comprising or associated with:
hyperphagia (optionally as assessed by HQ-CT),
moderate to severe binge eating disorder (BED),
bulimia nervosa,
management of obesity, optionally further comprising management of weight loss and maintenance of weight loss, or optionally further comprising as an adjunct to a reduced-calorie diet or for increased physical activity for chronic weight management,
early onset morbid obesity,
non-Alcoholic Steatohepatitis (NASH), non-alcoholic fatty liver disease (NAFLD),
Primary sclerosing cholangitis (PSC),
Primary biliary cholangitis liver (PBC),
inflammatory bowel disease (IBD), or irritable bowel syndrome (IBS),
type II diabetes,
hypothalamic injury-induced obesity,
obsessive-compulsive disorder (OCD),
Disruptive Mood Dysregulation Disorder (DMDD),
Oppositional Defiant Disorder (ODD),
excoriation,
trichotillomania,
intermittent explosive disorder (IED),
excessive daytime sleepiness (EDS),
excessive daytime sleepiness associated with narcolepsy and sleep apnea,
excessive daytime sleepiness associated with narcolepsy
excessive daytime sleepiness associated with central or obstructive sleep apnea,
narcolepsy,
narcolepsy type 1 (NT1) according to the International Classification of Sleep Disorders-Third Edition (ICSD-3)
narcolepsy with cataplexy
narcolepsy type 2 (NT2) according to ICSD-3
narcolepsy without cataplexy
cataplexy
seizures,
epilepsy,
an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
major depressive disorder (MDD),
treatment resistant depression (TRD),
negative symptoms of schizophrenia,
Parkinson's Disease,
MDD Associated with Parkinson's Disease,
general anxiety,
Social Anxiety Disorder (Social Phobia),
Fibromyalgia (FM),
diabetic neuropathy.
lower back pain,
chronic fatigue syndrome,
attention deficit hyperactivity disorder (ADHD),
autism,
Asperger spectrum,
a genetically confirmed disease or syndrome, wherein optionally the genetically confirmed disease or syndrome is or comprises Prader-Willi Syndrome, Bardet Biedl Syndrome, Smith-Magenis Syndrome, lp36 deletion syndrome, 16p11.2 deletion syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,

wherein optionally reducing adverse events comprises reducing psychosis, serotonin syndrome, tachycardia or postural orthostatic tachycardia syndrome. In alternative embodiments, provided are methods for administering:
   (i) a triple monoamine reuptake inhibitor (TRI),
   (ii) a melanin concentrating hormone receptor 1 (MCHR1) antagonist or inhibitor,
   (iii) a diazoxide or diazoxide formulation,
   (iv) a therapeutic combination of any of (i) to (iii),
   (iv) a therapeutic combination as used in a method as provided herein, or
   (v) a pharmaceutical composition, drug or formulation as provided herein, including a deuterated compound or composition as provided herein,
wherein the dosaging is determined by:
   (a) an empirical method for safe and predictable titration and to determine the initial therapeutic dose, or to determine the lowest therapeutic dose, or to determine an optimal effective dose; the method comprising:
      1. administrating a test dose (for example, no adverse events observed dose amount or a minimal adverse events observed dose amount or a safe dose amount) daily until a patient achieves a steady state plasma drug concentration,
      2. prior to the next scheduled dose, drawing a blood sample and assessing the test plasma drug concentration,
      3. calculating the initial therapeutic dose/day by:
         A. for drugs with a linear dose to plasma concentration, divide the initial target therapeutic plasma drug concentration by the test plasma drug concentration, and then multiply the dividend by the test dose amount, and
         B. for drugs with a non-linear dose to plasma concentration, divide the initial target therapeutic plasma drug concentration by the test plasma drug concentration, then multiply the dividend by the test dose amount and then multiply the product by a non-linear index factor, optionally, the initial target therapeutic plasma drug concentration is the concentration corresponding to: (i) certain pharmacodynamic efficacy marker levels, such as occupancy of serotonin transporter (SERT or 5-HTT), dopamine transporter (DAT) or norepinephrine transporter (NET) determined by imaging method such as positron emission tomography (PET); or, (ii) the lowest effective therapeutic dose determined by a clinical study.
      4. optionally comprising:
         i. administrating the initial therapeutic dose, daily, until a patient achieves a steady state plasma drug concentration,
         ii. prior to the next scheduled dose, drawing a blood sample and assessing the current therapeutic plasma drug concentration,
         iii. calculating the next therapeutic target dose/day by:
            A. For drugs with a linear dose to plasma concentration, divide the next target therapeutic plasma drug concentration by the current therapeutic plasma drug concentration and then multiply the dividend by the current therapeutic dose amount, and
            B. For drugs with a non-linear dose to plasma concentration, divide next target therapeutic plasma drug concentration by the current therapeutic plasma drug concentration, then multiply the dividend by the current therapeutic dose amount and then multiply the product by a non-linear index factor, and
         iv. optionally, reaching steady state after two subsequent measured plasma drug concentration results at least one week apart and within the next therapeutic plasma drug concentration target range,
         v. and optionally continuing the process until the lowest therapeutic dose or optimal effective dose is achieved; or
   (b) a model-based method for safe and predictable titration, and to determine the initial therapeutic dose and to determine the lowest therapeutic dose, or to determine an optimal effective dose, and method comprising:
      1. Administrating a test dose amount daily for between about 1 to 28 days, 1 to 21 days, 1 to 14 days, 1 to 10 days, 1 to 7 days, 1 to 3 days, or about 1 day,
      2. Drawing a first blood sample at between about 1 hour to 3 days, 6 hours to 2 days or about 1 day, after administering the first test dose,
      3. Drawing a second or subsequent blood samples at between about 1 hour to 28 days, 6 hours to 14 days, 1 day to 7 days, or at about 2 days, after drawing of the first blood samples or subsequent blood samples,
      4. calculating the initial therapeutic starting dose/day by:
         i. using the initial target therapeutic plasma drug concentration, the test dose amount, the time at the administration of the test dose(s), the plasma concentration of the blood samples and the time the draws of the blood samples and a pharmacometric model,
         ii. and optionally, using the initial target therapeutic plasma drug concentration, the test dose amount, the time at the administration of the test dose(s), the plasma concentration of the blood samples, the time of the draws of the blood samples, human PK data and a Bayesian pharmacometric model,
         and optionally comprising:
         i. administrating the initial therapeutic starting dose, daily until an individual or a patient achieves a steady state plasma drug concentration,
         ii. prior to the next scheduled dose, drawing a blood sample and assessing the current therapeutic plasma drug concentration,
         iii. calculating the next therapeutic target dose/day:
            A. for drugs with a linear dose to plasma concentration, divide the next target therapeutic plasma drug concentration by the current therapeutic plasma drug concentration and then multiply the dividend by the current therapeutic dose amount, and
            B. for drugs with a non-linear dose to plasma concentration, divide he next target therapeutic plasma drug concentration by the current therapeutic plasma drug concentration and then multiply the dividend by the current therapeutic dose amount and then multiply the product by the a non-linear index factor, and
         iv. continuing the process until the lowest therapeutic dose or optimal effective dose is achieved or
   (c) a model-based Bayesian adaptive control method for safe and predictable titration, and to determine the initial therapeutic dose and to determine the lowest therapeutic dose, or to determine an optimal effective dose, and method comprising:
      1. Administrating a test dose amount once or twice daily for between about 1 to 28 days, 1 to 21 days, 1 to 14 days, 1 to 10 days, 1 to 7 days, 1 to 3 days, or about 1 day,
      2. Drawing a first blood sample at between about ½ hour to 28 days, 1 hour to 3 days, 6 hours to 2 days and 1 day after administering the first test dose,
      3. Optionally, drawing a second or subsequent blood samples at between about 1 hour to 28 days, 6 hours to 14 days, 1 day to 7 days, or at about 2 days, after drawing of the first blood samples or subsequent blood samples,
      4. Calculating the initial therapeutic starting dose/day by:
         i. Selecting the initial target trough plasma drug concentration or other corresponding PK parameters for example CMAX and other plasma concentration(s) prior to trough, AUC, among others),
         ii. Recording information that may include but not limited to the test dose amount, the time at the administration of the test dose (recorded in a patient paper or electronic diary), the measured plasma drug concentration and times of the draws of the blood samples, patient information (for example age, body weight, height, gender, a patient's genetic information such as CYP450 profile, a patient's non-heritable information, a patient's medications, a patient's health status, among other factors), the available dose strengths,
         iii. Developing a population PK model by:
            A. using data from a PK study of a human population, and
            B. using a nonlinear mixed effect modeling software (for example NONMEM (version 7.2, ICON, Ellicott City, MD), Phoenix NLME (version 8.1,CERTARA, St. Louis, MO ) or other modeling software),
            C. applying estimation methods such as first-order conditional estimation with interaction method (FOCE-I), Bayesian, and other similar estimation methods,
            D. exploring different structural, allometric and other types of models,
            E. Selecting the model that adequately describe the data, defined as goodness- of-fit diagnostic plots, comparisons based on the minimum objective function value (OFV) and evaluation of the estimates of population fixed and random effect parameters,
            F. Parametrizing the PK model in terms of values of clearance (CL), volume of distribution (V) and absorption rate constant (Ka),
         iv. Entering information from i, ii, and iii, into the appropriate section of a pharmacokinetic clinical decision support softwares such as, MWPharm ++ (Mediware, Prague, Czech Republic), InsightRx (InsightRx, San Francisco, CA), DoseMeRx (DoseMe, Queensland, Australia), among other software systems),
         v. using the Bayesian estimator, generating individual parameter estimates and selecting the available dose strength that most closely reaches the initial trough plasma drug concentration target or optionally, other PK parameter targets that characterize a target therapeutic plasma drug concentration target,
         vi. optionally, reaching steady state of the current dose as defined by two subsequent measured plasma drug concentration results, optionally about one week apart, are within the current target plasma concentration range,
      5. optionally, calculating the next and subsequent dose amount by:
         i. Selecting the next target trough plasma drug concentration or other corresponding PK parameters for example CMAX and other plasma concentration(s) prior to trough, AUC, among others),
         ii. Recording information that may include but not limited to current dose amount, the time at the administration of the test dose (recorded in a patient paper or electronic diary), the measured plasma drug concentration and times of the draws of the blood samples, patient information (for example age, body weight, height, gender, a patient's genetic information such as CYP450 profile, a patient's non-heritable information, a patient's medications, a patient's health status,, the available dose strengths
         iii. Entering i and ii above into the appropriate section of a pharmacokinetic clinical decision support softwares such as, MWPharm ++ (Mediware, Prague, Czech Republic), InsightRx (InsightRx, San Francisco, CA), DoseMeRx (DoseMe, Queensland, Australia), among other software systems Using the Bayesian estimator, generating individual parameter estimates and selecting the available dose strength that most closely reaches the next trough plasma drug concentration target or optionally, other PK parameter targets that characterize a target therapeutic plasma drug concentration,
         iv. optionally, reaching steady state after two subsequent measured plasma drug concentration results, optionally about one week apart, are within the target range,
         v. optionally, continuing the process until the lowest therapeutic dose or optimal effective dose at steady state is achieved.

In alternative embodiments, the therapeutic combination, pharmaceutical dosage form, drug delivery device or product of manufacture comprises Formula I, or a deuterated derivative of Formula I.

In alternative embodiments, provided as methods for delivering or administering a triple monoamine reuptake inhibitor (TRI) to an individual in need thereof, wherein the TRI comprises Formula I, or a deuterated derivative of Formula I, and optionally the TRI comprises tesofensine.

In alternative embodiments, provided are methods for administering and maintain an effective dose of:
(i) a triple monoamine reuptake inhibitor (TRI),
(ii) a melanin concentrating hormone receptor 1 (MCHR1) antagonist or inhibitor,
(iii) a diazoxide or diazoxide formulation,
(iv) a therapeutic combination of any of (i) to (iii), or
(iv) a therapeutic combination as used in a method as provided herein, or
(v) a pharmaceutical composition, drug or formulation as provided herein, including a deuterated compound or composition as provided herein,
wherein the dosaging is determined by a method comprising:
(a) recording the maintenance dose amount and the maintenance therapeutic plasma drug concentration associated with the lowest effective therapeutic dose or optimal therapeutic dose, and
(b) at the early of between about 1 to 12 months, 3 to 9 month 4 to 8 months or 6 months or change in body weight, medication or health status then:
   prior to the next scheduled dose, draw a blood sample and assess the current plasma drug concentration,
   and optionally if the current plasma drug concentration is different from the maintenance therapeutic plasma drug concentration by more than about 20%, optionally by more than about 50%, then calculate a new therapeutic dose/day by a method comprising:
      i. For drugs with a linear dose to plasma concentration, divide the maintenance therapeutic plasma drug concentration by the current plasma drug concentration, and then and multiple the dividend by the maintenance dose amount,
         A. Optionally, reaching steady state after two subsequent measured plasma drug concentration results at least one week apart and within about 20% of the maintenance therapeutic plasma concentration, or
         B. Optionally, continuing the process until a maintenance therapeutic dose at steady state is achieved, and
      ii. For drugs with a non-linear dose to plasma concentration, divide the maintenance therapeutic plasma drug concentration by the current plasma drug concentration, then multiply the dividend by the maintenance dose amount, and then multiply the product by a non-linear index; or
      iii. and optionally using a Bayesian adaptive model-based method comprising:
         (A) recording a maintenance therapeutic trough plasma drug concentration, the actual dose amounts, the time at the administration of the dose(s), the measured current trough plasma concentration of the blood samples and/or the time of the draws of the blood samples, patient factors,
         (B) entering the above into an appropriate section of a pharmacokinetic clinical software, and
         (C) using a Bayesian estimator and generating individual parameter(s) and selecting a dose amount that would most closely reach a maintenance therapeutic trough plasma drug concentration, or
            D. optionally, reaching steady state after two subsequent measured plasma drug concentration results at least one week apart and within about 20% of the maintenance therapeutic plasma concentration, or
            E. optionally, continuing the process until a maintenance therapeutic dose at steady state is achieved.

In alternative embodiments, the TRI comprises Formula I, or a deuterated derivative of Formula I, or the TRI comprises tesofensine.

In alternative embodiments, provided are methods for treating hyperphagia, hyperphagia in PWS, Disruptive Mood Dysregulation Disorder (DMDD), Oppositional Defiant Disorder (ODD), obesity in hypothalamic-injury induced obesity, or binge eating disorder (BED) comprising administering to an individual in need thereof Formula I, or a deuterated derivative of Formula I.

In alternative embodiments, provided are methods for administering Formula I, or a deuterated derivative of Formula I, for treating hyperphagia, hyperphagia in PWS, Disruptive Mood Dysregulation Disorder (DMDD), Oppositional Defiant Disorder (ODD), obesity in hypothalamic-injury induced obesity, or binge eating disorder (BED).

In alternative embodiments, provided are methods treating hyperphagia, hyperphagia in PWS, Disruptive Mood Dysregulation Disorder (DMDD), Oppositional Defiant Disorder (ODD), obesity in hypothalamic-injury induced obesity, and binge eating disorder (BED) with an oral dosage form of Formula I, or an deuterated derivative of Formula I of the proceeding claims, the method comprises administering an oral dosage form that provides trough plasma concentration, a 24-hour time-averaged plasma concentration, or a daytime 12-hour time-averaged plasma concentration between 250 ng/mL to 500 ng/ml, 500 to 1000 ng/ml, 1000 to 1500 ng/ml, 1500 to 2000 ng/ml, or 1500 to 2500 ng/ml of Formula I, or an deuterated derivative of Formula I of the proceeding claims, when administered once daily, or twice daily, and measured at about one week, about two weeks, or steady state.

In alternative embodiments, provided are methods for treating hyperphagia, hyperphagia in PWS, Disruptive Mood Dysregulation Disorder (DMDD), Oppositional Defiant Disorder (ODD), obesity in hypothalamic-injury induced obesity, and binge eating disorder (BED) with an oral dosage form of Formula I, or an deuterated derivative of Formula I of the proceeding claims, the method comprises administering an oral dosage form that provides trough plasma concentration, a 24-hour time-averaged plasma concentration, or a daytime 12-hour time-averaged plasma concentration between 150 to 3000 ng/ml of Formula I, or an deuterated derivative of Formula I of the proceeding claims, when administered once daily, or twice daily, and measured at about one week, about two weeks, or steady state.

The details of one or more exemplary embodiments of the invention are set forth in the accompanying drawings and the description below. Other features, objects, and advantages of the invention will be apparent from the description and drawings, and from the claims.

All publications, patents, patent applications cited herein are hereby expressly incorporated by reference in their entireties for all purposes.

### DESCRIPTION OF DRAWINGS

The patent or application file contains at least one drawing executed in color. Copies of this patent or patent application publication with color drawing(s) will be provided by the Office upon request and payment of the necessary fee.

The drawings set forth herein are illustrative of exemplary embodiments provided herein and are not meant to limit the scope of the invention as encompassed by the claims.

Figures are described in detail herein.
FIG. 1 illustrates an exemplary protocol for synthesizing the exemplary compound 7-([1,2,4]triazolo[1,5-a]pyridin-6-yl)-4-(3,4-dichlorophenyl)-1,2,3,4-tetrahydroisoquinoline-1,1,3,3,4-d5, as discussed in Example 2, below.
FIG. 2 illustrates an exemplary protocol for synthesizing the exemplary compound 7-([1,2,4]triazolo[1,5-a]pyridin-6-yl)-4-(3,4-dichlorophenyl)-1,2,3,4-tetrahydroisoquinoline-1,1-d2, as discussed in Example 3, below.
FIG. 3 illustrates an exemplary protocol for synthesizing the exemplary compound 7-([1 ,2,4]triazolo[1,5-a]pyridin-6-yl)-4-(3,4-dichlorophenyl)-1,2,3,4-tetrahydroisoquinoline-3,3,4-d3, as discussed in Example 4, below.
FIG. 4. illustrates an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1-d2)pyridin-2(1H)-one, as discussed in Example 5, below.
FIG. 5. illustrates an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1,3,3-d4)pyridin-2(1H)-one, as discussed in Example 6, below.
FIG. 6. illustrates an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-3,3-d2)pyridin-2(1H)-one, as discussed in Example 7, below.
FIG. 7 illustrates: Relationships between plasma concentrations of the compound of formula I versus striatal occupancies at dopamine transporters (DAT) after multiple doses of 10 to 60 mg the compound of formula I.
FIG. 8 and FIG. 9 illustrate the relationships between plasma concentrations of the compound of formula I versus occupancies at serotonin transporters (SERT) in striatum and thalamus after multiple doses of 3-60 mg the compound of formula I. Like reference symbols in the various drawings indicate like elements.
FIG. 10 schematically illustrates an exemplary, alternative protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1,3,3-d4)pyridin-2(1H)-one.
FIG. 11 schematically illustrates an exemplary, alternative protocol for synthesizing the exemplary compound P, as discussed in detail in Example 8, below.
FIG. 12 schematically illustrates the proton NMR spectra of compound P, as discussed in detail in Example 8, below.
FIG. 13 schematically illustrates the LCMS spectra of compound P, as discussed in detail in Example 8, below.

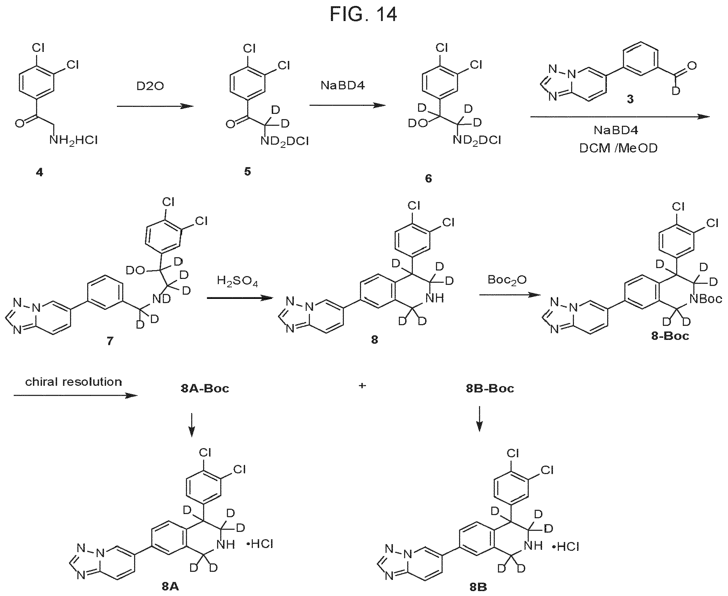

FIG. 14 illustrates an exemplary protocol for synthesizing the exemplary compounds: 7-([1,2,4]triazolo[1,5-a]pyridin-6-yl)-4-(3,4-dichlorophenyl)-1,2,3,4-tetrahydroisoquinoline-1,1,3,3,4-d5, 8A and 8B, as discussed in Example 4.1, below.

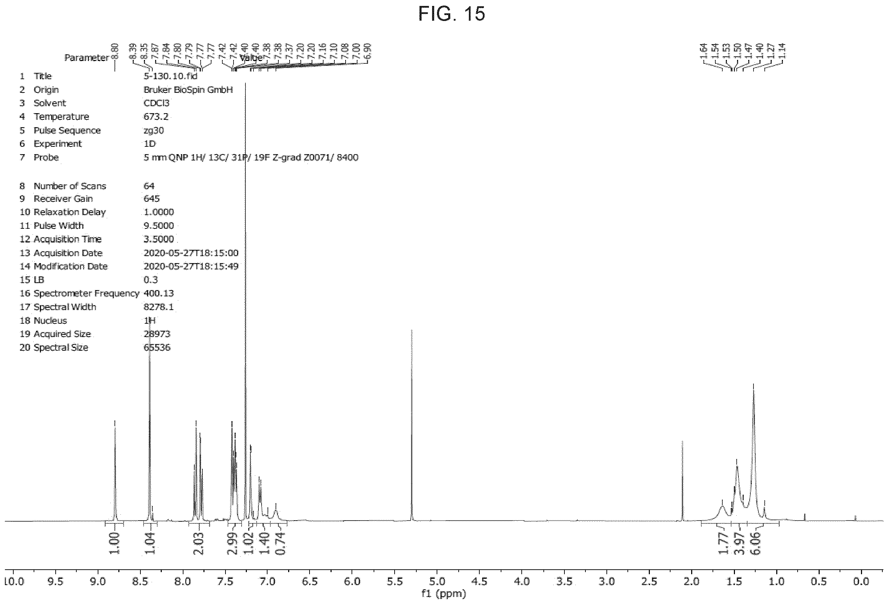

FIG. 15 schematically illustrates the proton NMR spectra of compound 8-Boc, as discussed in detail in Example 4.1, below.

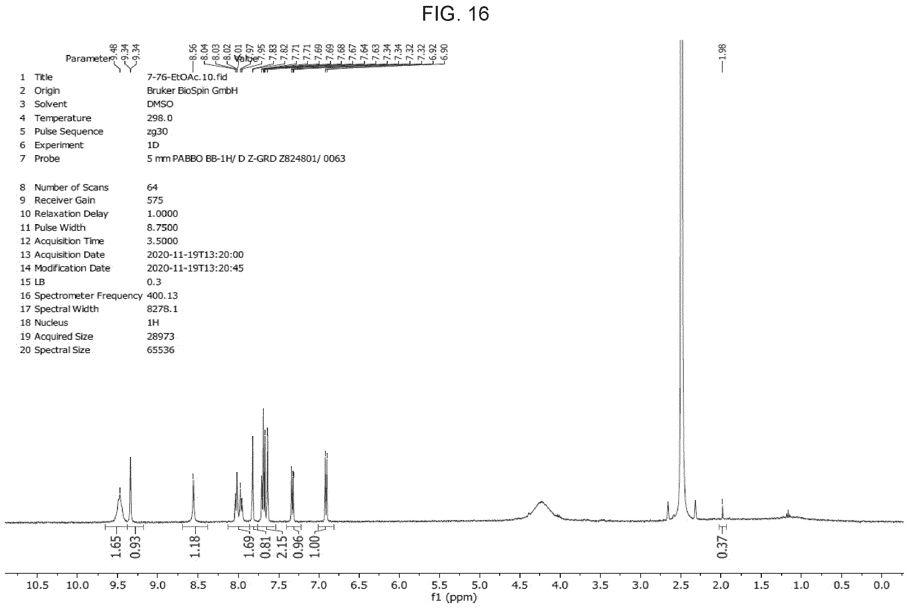

FIG. 16 schematically illustrates the proton NMR spectra of compound 8A, as discussed in detail in Example 4.1, below.

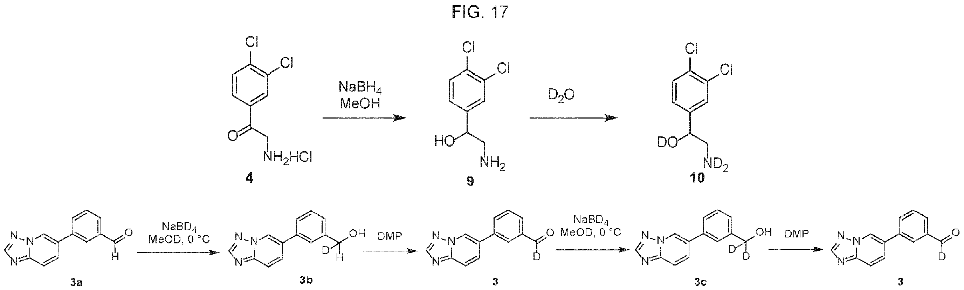

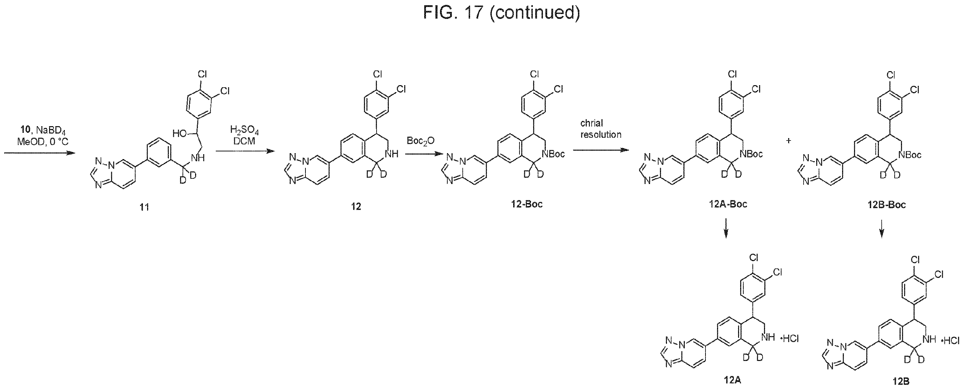

FIG. 17 illustrates an exemplary protocol for synthesizing the exemplary compounds: 7-([1,2,4]triazolo[1,5-a]pyridin-6-yl)-4-(3,4-dichlorophenyl)-1,2,3,4-tetrahydroisoquinoline-1,1-d2, 12A and 12B, as discussed in Example 5.1, below.

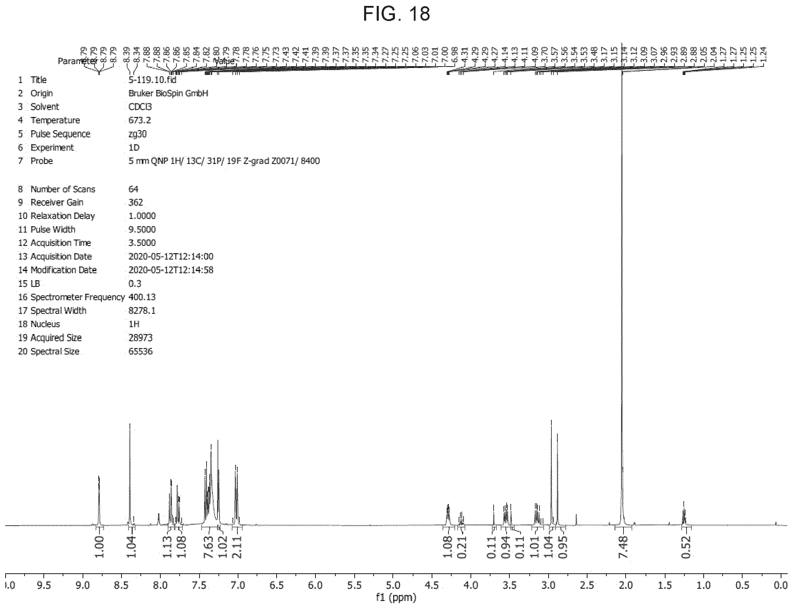

FIG. 18 schematically illustrates the proton NMR spectra of compound 12, as discussed in detail in Example 5.1, below.

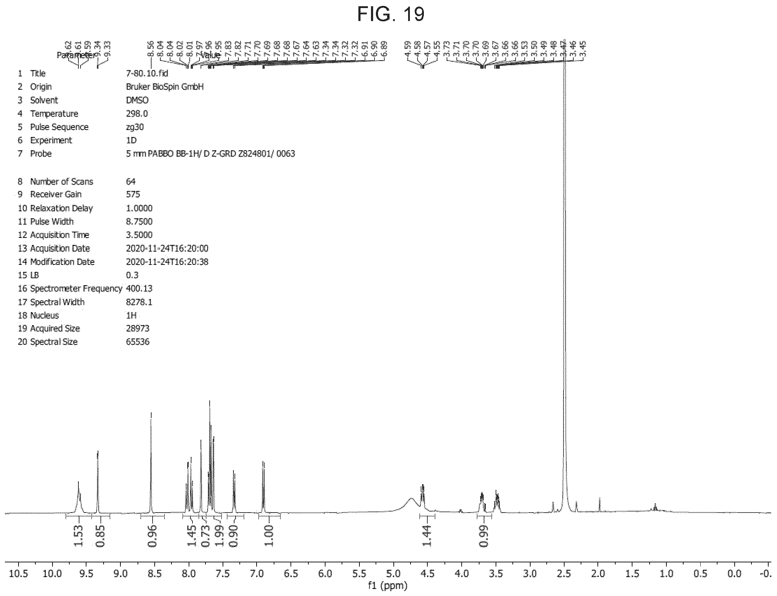

FIG. 19 schematically illustrates the proton NMR spectra of compound 12A, as discussed in detail in Example 5.1, below.

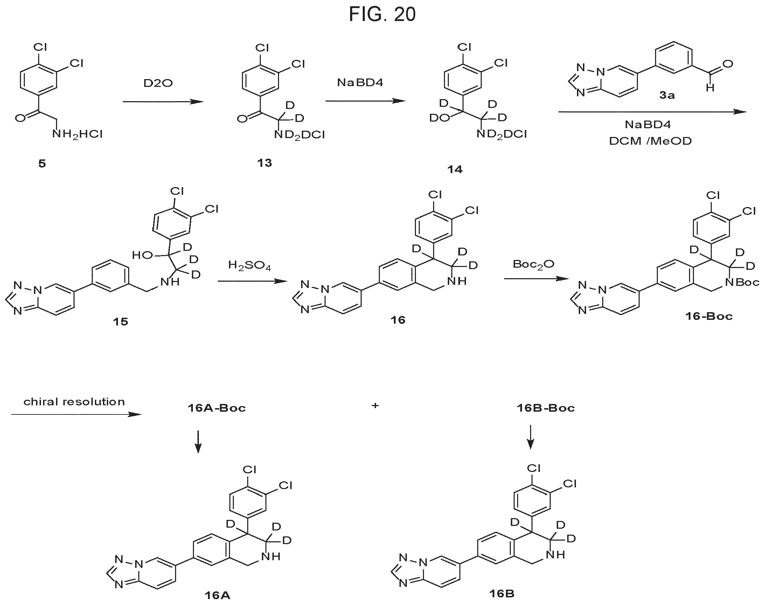

FIG. 20 illustrates an exemplary protocol for synthesizing the exemplary compounds 7-([1,2,4]triazolo[1,5-a]pyridin-6-yl)-4-(3,4-dichlorophenyl)-1,2,3,4-tetrahydroisoquinoline-3,3,4-d3 16A and 16B, as discussed in Example 6.1, below.

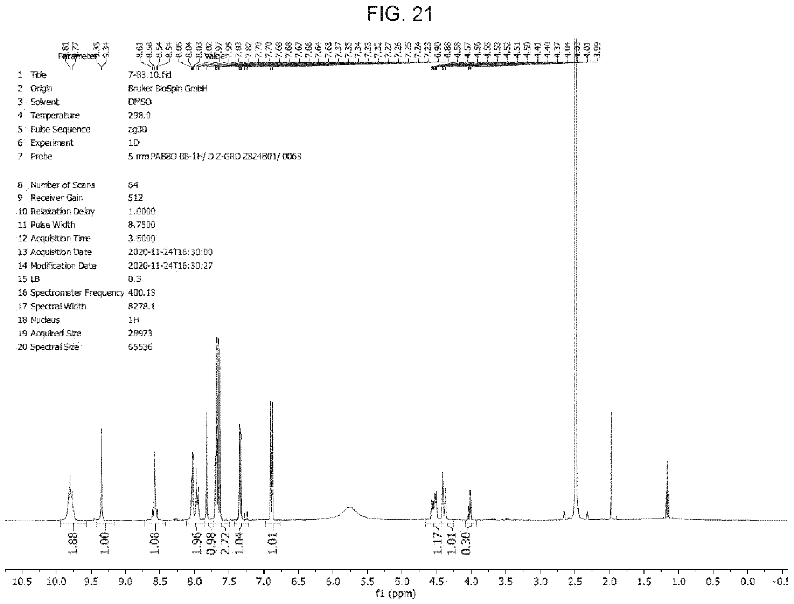

FIG. 21 schematically illustrates the proton NMR spectra of compound 16B, as discussed in detail in Example 6.1, below.

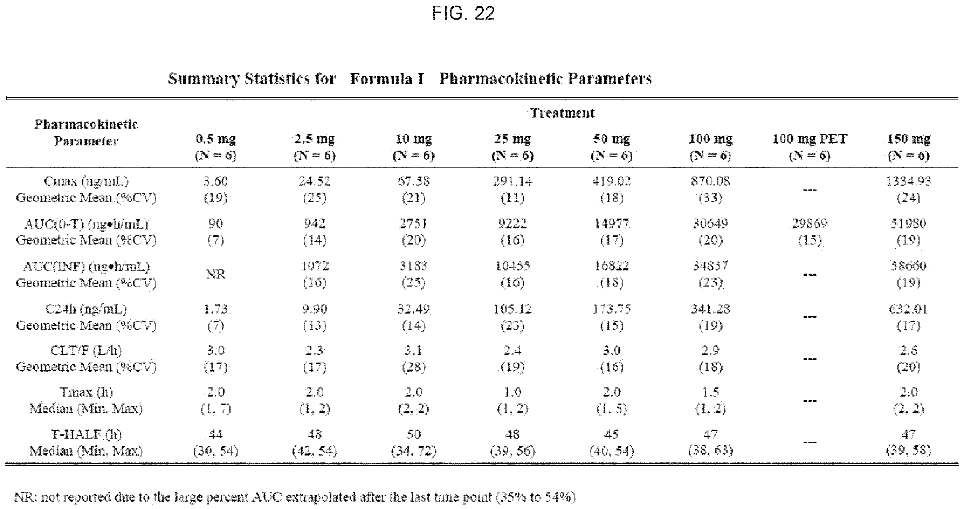

FIG. 22 illustrates the summary statistics for Formula I Pharmacokinetic Parameters from health human single ascending dose PK study.

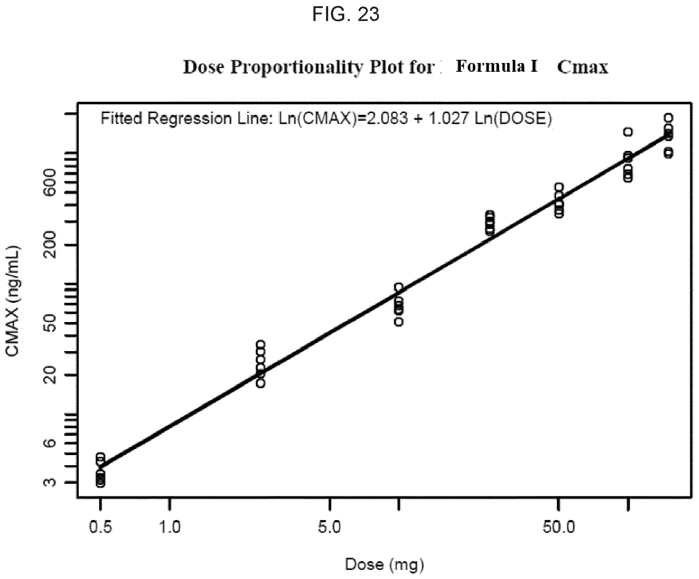

FIG. 23 illustrates the Dose Proportionality Plot for Formula I Cmax. from health human single ascending dose PK study.

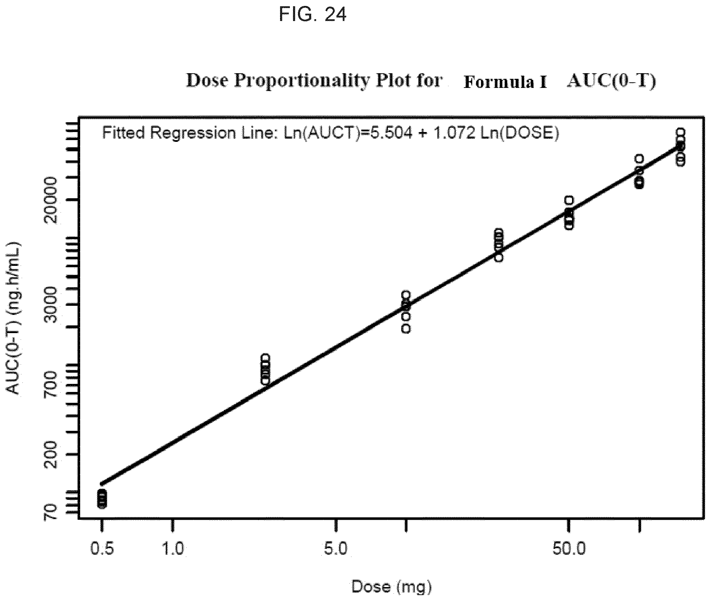

FIG. 24 illustrates the Dose Proportionality Plot for Formula I AUC(0-T) from health human single ascending dose PK study.
FIG. 25 illustrates the PK Profiles in Phase I Single Ascending Dose study of Formula I.
FIG. 26 illustrates the 3- compartment PK model for Formula I.
FIG. 27 illustrates the Goodness-of-fit plots of the Population PK model for Formula I.
FIG. 28 illustrates the Consistency of PK results of the PK-model with those determined by data from human SAD Study of Formula I.
FIG. 29 illustrates the Visual predictive check (VPC) stratified by dose panels of Formula I
FIG. 30 illustrates the Emax model fitting using PK-PD data from Example 12.
FIG. 31 illustrates the PK/PD model parameters for simulation of Formula I.
FIG. 32 illustrates the Ctrough levels of Formula I needed for 30, 45 and 60% DAT receptor occupancy, as discussed in Example 16, below.
FIG. 33 illustrates the simulation of Formula I on the relationship between Receptor Occupancy and time after dose (in hours), as discussed in Example 16, below.
FIG. 34 illustrates the simulation of individualized dosing based on Ctrough on day 7 to estimate dose change on day 14 to achieve 30% DAT receptor occupancy, as discussed in Example 16, below.
FIG. 35 illustrates the simulation of individualized dosing to achieve 60% DAT receptor occupancy for 160 kg patients, as discussed in Example 16, below.

Like reference symbols in the various drawings indicate like elements.

### DETAILED DESCRIPTION

In alternative embodiments, provided are novel deuterated [1,2,4]triazolo[1,5-a]pyridinyl-6-yl-substituted tetrahydroisoquinoline derivative triple monoamine reuptake inhibitors. In alternative embodiments, provided are novel deuterated (1-azinone)-substituted pyridoindole melanin concentrating hormone receptor 1 (MCHR1) antagonists or inhibitors.

In alternative embodiments, provided are therapeutic combinations or formulations of drugs comprising various combinations of triple monoamine reuptake antagonists or inhibitors, melanin concentrating hormone receptor 1 (MCHR1) antagonists or inhibitors and diazoxide or diazoxide Choline Controlled-Release (DCCR) formulations, these in combination with other drugs or active agents. In alternative embodiments, provided are methods for the treatment of various conditions, including genetic confirmed syndromes, and diseases, using therapeutic combinations and formulations of drugs as provided herein.

While the embodiments as provided herein are not limited by any particular mechanism of action, evidence suggests that while TRI, MCHR1 antagonist and diazoxide target different areas and pharmacological targets in the brain, their actions are complementary to each other, thus, described herein for the first time is the finding that administering combinations of TRI, MCHR1 antagonist and diazoxide, or all three, can provide a synergistic benefit to an individual in need thereof, for example, in the treatment of a hyperphagia and/or a related eating disorder.

### Pharmaceutical Compositions and Formulations

In alternative embodiments, drugs or compounds are provided herein, or drugs or compounds used to practice methods are provided herein, are formulated for administration by any or a variety of means including orally, parenterally, by inhalation spray, nasally, topically, intrathecally, intrathecally, intracerebrally, epidurally, intracranially or rectally. Drugs or compounds are provided herein, or drugs or compounds used to practice methods are provided herein, can further comprise pharmaceutically acceptable carriers, adjuvants and vehicles. In alternative embodiments, drugs or compounds as provided herein, or a composition used to practice the methods as provided herein, are formulated for parenteral administration, including administration intrathecally, intracerebrally or epidurally (into a intrathecal, intracerebral, epidural space), subcutaneously, intravenously, intramuscularly and/or intraarterially; for example, by injection routes but also including a variety of infusion techniques. Intraarterial, intrathecal, intracranial, epidural, intravenous and other injections as used in some embodiments can include administration through catheters or pumps, for example, an intrathecal pump, or an implantable medical device (which can be an intrathecal pump or catheter).

In alternative embodiments, drugs or compounds as provided herein, or a composition used to practice the methods as provided herein, can be formulated in accordance with a routine procedure(s) adapted for a desired administration route. In alternative embodiments, drugs or compounds as provided herein, or a composition used to practice the methods as provided herein, are formulated or manufactured as lyophilates, powders, lozenges, liposomes, suspensions, solutions or emulsions in oily or aqueous vehicles, and can contain formulatory agents such as suspending, stabilizing and/or dispersing agents.

In alternative embodiments, drugs or compounds as provided herein, or a composition used to practice the methods as provided herein, can be formulated as a preparation for implantation or injection. Thus, for example, the compounds can be formulated with suitable polymeric or hydrophobic materials (for example, as an emulsion in an acceptable oil) or ion exchange resins, or as sparingly soluble derivatives (for example, as a sparingly soluble salt). Alternatively, the active ingredient can be in powder form for constitution with a suitable vehicle, for example, sterile pyrogen-free water, before use. Suitable alternative and exemplary formulations for each of these methods of administration can be found, for example, in Remington: The Science and Practice of Pharmacy, A. Gennaro, ed., 20th edition, Lippincott, Williams & Wilkins, Philadelphia, Pa.

In alternative embodiments, drugs or compounds as provided herein, or a composition used to practice the methods as provided herein, can be formulations for parenteral administration comprising any common excipient, for example, sterile water or saline, a polyalkylene glycol such as a polyethylene glycol, an oil of synthetic or vegetable origin, a hydrogenated naphthalene and the like. In alternative embodiments, drugs or compounds as provided herein, or a composition used to practice the methods as provided herein, can be a biocompatible, biodegradable lactide polymer, a lactide/glycolide copolymer, or polyoxyethylene-polyoxypropylene copolymers can be useful excipients to control the release of active compounds.

In alternative embodiments, drugs or compounds as provided herein, or a composition used to practice the methods as provided herein, are administered using parenteral delivery systems such as ethylene-vinyl acetate copolymer particles, osmotic pumps, implantable infusion systems, intrathecal catheters, pumps and implants, and/or use of liposomes. Formulations for parenteral administration can also include glycocholate for buccal administration, methoxysalicylate for rectal administration, or citric acid for vaginal administration. Formulations for inhalation administration can contain as excipients, for example, lactose, or can be aqueous solutions containing, for example, polyoxyethylene-9-auryl ether, glycocholate and deoxycholate, or oily solutions for administration in the form of nasal drops, or as a gel to be applied intranasally.

In alternative embodiments, drugs or compounds as provided herein, or a composition used to practice the methods as provided herein, are administered intranasally. When given by this route, examples of appropriate dosage forms are a nasal spray or dry powder, as is known to those skilled in the art. For example, a nasal formulation can comprise a conventional surfactant, generally a non-ionic surfactant. When a surfactant is employed in a nasal formulation, the amount present will vary depending on the particular surfactant chosen, the particular mode of administration (for example drop or spray) and the effect desired.

In alternative embodiments, drugs or compounds as provided herein, or a composition used to practice the methods as provided herein, are in the form of a sterile injectable preparation, such as a sterile injectable aqueous or oleaginous suspension. This suspension may be formulated according to the known art using those suitable dispersing or wetting agents and suspending agents. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In alternative embodiments, sterile fixed oils are conventionally be employed as a solvent or suspending medium. For this purpose any bland fixed oil may be employed including synthetic mono- or diglycerides. In alternative embodiments, fatty acids such as oleic acid may likewise be used in the preparation of injectables. Formulations for intravenous administration can comprise solutions in sterile isotonic aqueous buffer. Where necessary, the formulations can also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule (ampoule) or sachet indicating the quantity of active agent. Where the compound is to be administered by infusion, it can be dispensed in a formulation with an infusion bottle containing sterile pharmaceutical grade water, saline or dextrose/water. Where the compound is administered by injection, an ampule of sterile water for injection or saline can be provided so that the ingredients can be mixed prior to administration.

In alternative embodiments, drugs or compounds as provided herein, or a composition used to practice the methods as provided herein, further comprise aqueous and non-aqueous sterile injection solutions that can contain (comprise) antioxidants, buffers, bacteriostats, bactericidal antibiotics and solutes that render the formulation isotonic with the bodily fluids of the intended recipient; and/or aqueous and non-aqueous sterile suspensions, which can include suspending agents and thickening agents.

In alternative embodiments, drugs or compounds as provided herein, or a composition used to practice the methods as provided herein, are formulated for topical administration, for example, in the form of a liquid, lotion, cream or gel. Topical administration can be accomplished by application directly on the treatment area. For example, such application can be accomplished by rubbing the formulation (such as a lotion or gel) onto the skin of the treatment area, or by a spray application of a liquid formulation onto the application or treatment area.

In alternative embodiments, drugs or compounds as provided herein, or a composition used to practice the methods as provided herein, comprise a bioimplant or a bioimplant material, and also can be coated with a compound of the invention or other compounds so as to improve interaction between cells and the implant.

In alternative embodiments, drugs or compounds as provided herein, or a composition used to practice the methods as provided herein, comprise minor amounts of wetting or emulsifying agents, or pH buffering agents.

In alternative embodiments, drugs or compounds as provided herein, or a composition used to practice the methods as provided herein, are formulated as a suppository, with traditional binders and carriers such as triglycerides.

In alternative embodiments, drugs or compounds as provided herein, or a composition used to practice the methods as provided herein, comprise oral formulations such as tablets, pills, troches, lozenges (see, for example, as described in USPN 5,780,055), aqueous or oil suspensions, dispersible powders or granules, emulsions, hard or soft capsules or geltabs, gels, jellies, syrups and/or elixirs. Compositions intended for oral use may be prepared according to any method known to the art for the manufacture of pharmaceutical compositions and such compositions may contain one or more agents including sweetening agents, taste-masking agents, flavoring agents, coloring agents and preserving agents, in order to provide a palatable preparation. Oral formulations can include standard carriers such as pharmaceutical grades of mannitol, lactose, starch, magnesium stearate, polyvinyl pyrrolidone, sodium saccharine, cellulose, magnesium carbonate, etc. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

In alternative embodiments, formulations for oral use are hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

In alternative embodiments, drugs or compounds as provided herein, or a composition used to practice the methods as provided herein, comprise aqueous suspensions comprising the active material in admixture with excipients suitable for the manufacture of aqueous suspensions. Exemplary excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (for example, lecithin), a condensation product of an alkylene oxide with a fatty acid (for example, polyoxyethylene stearate), a condensation product of ethylene oxide with a long chain aliphatic alcohol (for example, heptadecaethyleneoxycetanol), a condensation product of ethylene oxide with a partial ester derived from a fatty acid and a hexitol anhydride (for example, polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

In alternative embodiments, drugs or compounds as provided herein, or a composition used to practice the methods as provided herein, comprise oil suspensions that can be formulated by suspending the active ingredient (for example, a compound of this invention) in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

In alternative embodiments, drugs or compounds as provided herein, or a composition used to practice the methods as provided herein, include an agent which controls release of the compound, thereby providing a timed or sustained release compound.

In alternative embodiments, drugs or compounds as provided herein, or a composition used to practice the methods as provided herein, are formulated or made as a multiparticulate and/or a solid dispersion formulation, for example, as described in, for example, U.S. Patent App. Pub. No. 20080118560, for example, comprising a hydrophobic matrix former which is a water-insoluble, non-swelling amphiphilic lipid; and a hydrophilic matrix former which is a meltable, water-soluble excipient. In one embodiment, drugs or compounds as provided herein, or a composition used to practice the methods as provided herein, are contained in tablets, pills, capsules, troches, and the like comprising any combination of a binder, for example, as a starch, polyvinyl pyrrolidone, gum tragacanth or gelatin; a filler, such as microcrystalline cellulose or lactose; a disintegrating agent, such as crospovidone, sodium starch glycolate, corn starch, and the like; a lubricant, such as magnesium stearate, stearic acid, glyceryl behenate; a glidant, such as colloidal silicon dioxide and talc; a sweetening agent, such as sucrose or saccharin, aspartame, acesulfame-K; and/or flavoring agent, such as peppermint, methyl salicylate, or orange flavoring. When the dosage unit form is a capsule, it also can comprise a liquid carrier, such as a fatty oil.

In alternative embodiments, drugs or compounds as provided herein, or a composition used to practice the methods as provided herein, comprise (or are contained or packaged in) unit dosage formulations having a coating, for example, a coat comprising a sugar, shellac, sustained and/or other enteric coating agents, or any pharmaceutically pure and/or nontoxic agents.

In alternative embodiments, drugs or compounds as provided herein, or a composition used to practice the methods as provided herein, comprise (or are contained or packaged in) unit dosage formulations, wherein each different compound of the composition or product of manufacture is contained in a different layer of a pill, tablet or capsule, for example, as described in USPN 7,384,653, for example, having an outer base-soluble layer and an inner acid-soluble layer. In alternative embodiments, drugs or compounds as provided herein, or a composition used to practice the methods as provided herein, comprise (or are contained or packaged in) unit dosage formulations, wherein each different compound of the composition or product of manufacture is contained in a liquid or a gel of different viscosity, for example, described in U.S. Patent App. Pub. No. 20050214223. In alternative embodiments, drugs or compounds as provided herein, or a composition used to practice the methods as provided herein, comprise (or are contained or packaged in) unit dosage formulations having reduced abuse potential, for example, as described in U.S. Patent App. Pub. No. 20040228802, for example, comprising a bittering agent, a bright deterrent/indicator dye, or a fine insoluble particulate matter.

### Carriers

In alternative embodiments, drugs or compounds as provided herein, or a composition used to practice the methods as provided herein, comprise or are formulated with or as aqueous or non-aqueous solutions, suspensions, emulsions and solids. Examples of non-aqueous solvents suitable for use as disclosed herein include, but are not limited to, propylene glycol, polyethylene glycol, vegetable oils such as olive oil, and injectable organic esters such as ethyl oleate. In alternative embodiments, aqueous carriers can comprise water, ethanol, alcoholic/aqueous solutions, glycerol, emulsions and/or suspensions, including saline and buffered media. Oral carriers can be elixirs, syrups, capsules, tablets and the like.

In alternative embodiments, liquid carriers are used to manufacture or formulate drugs or compounds as provided herein, or a composition used to practice the methods as provided herein, including carriers for preparing solutions, suspensions, emulsions, syrups, elixirs and pressurized compounds. The active ingredient can be dissolved or suspended in a pharmaceutically acceptable liquid carrier such as water, an organic solvent, a mixture of both or pharmaceutically acceptable oils or fats. The liquid carrier can comprise other suitable pharmaceutical additives such as solubilizers, emulsifiers, buffers, preservatives, sweeteners, flavoring agents, suspending agents, thickening agents, colors, viscosity regulators, stabilizers or osmo-regulators.

In alternative embodiments, liquid carriers used to manufacture or formulate compounds of this invention comprise water (partially containing additives as above, for example cellulose derivatives, preferably sodium carboxymethyl cellulose solution), alcohols (including monohydric alcohols and polyhydric alcohols, for example glycols) and their derivatives, and oils (for example fractionated coconut oil and arachis oil). For parenteral administration, the carrier can also include an oily ester such as ethyl oleate and isopropyl myristate. Sterile liquid carriers are useful in sterile liquid form comprising compounds for parenteral administration. The liquid carrier for pressurized compounds disclosed herein can be halogenated hydrocarbon or other pharmaceutically acceptable propellant.

In alternative embodiments, solid carriers are used to manufacture or formulate drugs or compounds as provided herein, or a composition used to practice the methods as provided herein, including solid carriers comprising substances such as lactose, starch, glucose, methyl-cellulose, magnesium stearate, dicalcium phosphate, mannitol and the like. A solid carrier can further include one or more substances acting as flavoring agents, lubricants, solubilizers, suspending agents, fillers, glidants, compression aids, binders or tablet-disintegrating agents; it can also be an encapsulating material. In powders, the carrier can be a finely divided solid which is in admixture with the finely divided active compound. In tablets, the active compound is mixed with a carrier having the necessary compression properties in suitable proportions and compacted in the shape and size desired. Suitable solid carriers include, for example, calcium phosphate, magnesium stearate, talc, sugars, lactose, dextrin, starch, gelatin, cellulose, polyvinylpyrrolidine, low melting waxes and ion exchange resins. A tablet may be made by compression or molding, optionally with one or more accessory ingredients. Compressed tablets may be prepared by compressing in a suitable machine the active ingredient in a free flowing form such as a powder or granules, optionally mixed with a binder (for example, povidone, gelatin, hydroxypropylmethyl cellulose), lubricant, inert diluent, preservative, disintegrant (for example, sodium starch glycolate, cross-linked povidone, cross-linked sodium carboxymethyl cellulose) surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropyl methylcellulose in varying proportions to provide the desired release profile. Tablets may optionally be provided with an enteric coating, to provide release in parts of the gut other than the stomach.

In alternative embodiments, parenteral carriers are used to manufacture or formulate drugs or compounds as provided herein, or a composition used to practice the methods as provided herein, including parenteral carriers suitable for use as disclosed herein include, but are not limited to, sodium chloride solution, Ringer's dextrose, dextrose and sodium chloride, lactated Ringer's and fixed oils. Intravenous carriers can comprise fluid and nutrient replenishers, electrolyte replenishers such as those based on Ringer's dextrose and the like. Preservatives and other additives can also comprise, for example, antimicrobials, antioxidants, chelating agents, inert gases and the like.

In alternative embodiments, carriers used to manufacture or formulate drugs or compounds as provided herein, or a composition used to practice the methods as provided herein, can be mixed as needed with disintegrants, diluents, granulating agents, lubricants, binders and the like using conventional techniques known in the art. The carriers can also be sterilized using methods that do not deleteriously react with the compounds, as is generally known in the art.

The invention also provides articles of manufacture and kits containing (comprising) drugs or compounds as provided herein, or a composition used to practice the methods as provided herein, including pharmaceutical compositions and formulations. By way of example only a kit or article of manufacture can include a container (such as a bottle) with a desired amount of a compound (or pharmaceutical composition of a compound) described herein. Such a kit or article of manufacture can further include instructions for using the compound (or pharmaceutical composition of a compound) described herein. The instructions can be attached to the container, or can be included in a package (such as a box or a plastic or foil bag) holding the container.

The drugs or compounds as provided herein, or a composition used to practice the methods as provided herein, can be delivered to the body or targeted to a specific tissue or organ (for example, a muscle or a brain) by any method or protocol, for example, including *ex vivo* "loading of cells" with drugs or compounds as provided herein, or a composition used to practice the methods as provided herein, where the "loaded cell" is the administered intramuscularly, or intrathecally, intracerebrally, or epidurally into the central nervous system (CNS), for example, as described in U.S. Pat. App. Pub. No. 20050048002.

In alternative embodiments, drugs or compounds as provided herein, or a composition used to practice the methods as provided herein, are first lyophilized and then suspended in a hydrophobic medium, for example, comprising aliphatic, cyclic or aromatic molecules, for example, as described in U.S. Pat. App. Pub. No. 20080159984.

In alternative embodiments, drugs or compounds as provided herein, or a composition used to practice the methods as provided herein, comprise or are formulated as pharmaceutically acceptable salts. Pharmaceutically acceptable salts can include suitable acid addition or base salts thereof. In alternative embodiments, compounds can be formulated as described in Berge et al, J Pharm Sci, 66, 1-19 (1977).

In alternative embodiments, drugs or compounds as provided herein, or a composition used to practice the methods as provided herein, are formulated as salts that are formed, for example, with strong inorganic acids such as mineral acids, for example hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (for example, by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with amino acids, for example aspartic or glutamic acid; with benzoic acid; or with organic sulfonic acids, such as (C₁-C₄)-alkyl- or arylsulfonic acids which are unsubstituted or substituted (for example, by a halogen) such as methane- or p-toluene sulfonic acid. Compounds of the invention also encompass salts which are not pharmaceutically acceptable, for example, a salt may still be valuable as an intermediate in a synthetic or analytical process or protocol.

In alternative embodiments, compounds, active agents or drugs as provided herein, or as used to practice methods as provided herein, comprise any acceptable salt for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. Pharmaceutical compositions as disclosed herein can be prepared in accordance with methods well known and routinely practiced in the art. See, for example, Remington: The Science and Practice of Pharmacy, Mack Publishing Co., 20th ed., 2000; and Sustained and Controlled Release Drug Delivery Systems, J.R. Robinson, ed., Marcel Dekker, Inc., New York, 1978.

In some embodiments, compounds, active agents or drugs as provided herein, or as used to practice methods as provided herein, are provided in the form of pharmaceutically acceptable salts comprising an amine that is basic in nature and can react with an inorganic or organic acid to form a pharmaceutically acceptable acid addition salt; for example, such salts comprise inorganic acids such as hydrochloric, hydrobromic, hydriodic, sulfuric and phosphoric acid, as well as organic acids such as para-toluenesulfonic, methanesulfonic, oxalic, para-bromophenylsulfonic, carbonic, succinic, citric, benzoic and acetic acid, and related inorganic and organic acids; or optionally such pharmaceutically acceptable salts comprise sulfate, pyrosulfate, bisulfate, sulfite, bisulfite, phosphate, mono-hydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephathalate, sulfonate, xylenesulfonate, phenylacetate, phenylpropionate, phenylbutyrate, citrate, lactate, .beta.-hydroxybutyrate, glycollate, maleate, tartrate, methanesulfonate, propanesulfonates, naphthalene-1-sulfonate, naphthalene-2-sulfonate, mandelate, hippurate, gluconate, lactobionate, methylene-bis-b-hydroxynaphthoates, gentisates, isethionates, di-p-toluoyltartrates, methane-sulphonates, ethanesulphonates, benzenesulphonates, p-toluenesulphonates, cyclohexylsulphamates and quinateslaurylsulphonate salts, and the like salts.

In alternative embodiments, compounds, active agents or drugs as provided herein, or as used to practice methods as provided herein, comprise compositions manufactured under "Good manufacturing practice" or GMP, or "current good manufacturing practices" (cGMP), conditions.

### Derivatized and Deuterated Compounds

In alternative embodiments, compounds, active agents or drugs as provided herein, or as used to practice methods as provided herein, are derivatized analogs, for example, metabolically blocked or otherwise altered derivatives, including deuterated, hydroxylated, fluorinated or methylated analogs or derivatives, or any combination thereof.

With regard to deuterated compounds as provided herein, or as used to practice methods as provided herein, it will be recognized that some variation of natural isotopic abundance occurs in a synthesized compound depending upon the origin of chemical materials used in the synthesis. Thus, a preparation of a compound will inherently contain small amounts of deuterated isotopologues. For compounds as provided herein, or as used to practice methods as provided herein, which include pharmaceutical preparations and formulations, when a particular position is designated as having deuterium ("-D"), it is understood that the abundance of deuterium at that position is greater than, or substantially greater than, the natural abundance of deuterium, which is 0.015%. For example, alternative embodiments of the invention comprise analogs of drugs or compounds as provided herein, or drugs or compounds used to practice methods as provided herein, having greater than 0.02%, or greater than about 0.1% deuterium. In one embodiment, the deuterium substitution, or "enrichment", occurs at a specific position or positions. In one embodiment, the deuterium enrichment is no less than about 1%, 10%, 20%, 50%, 70%, 80%, 90% or 95% or more or between about 1% and 100%.

In one embodiment, the deuterated (or otherwise substituted) compounds as provided herein, or as used to practice methods as provided herein have a slower rate of metabolism, for example, slower rate of hydroxylation, than a corresponding protonated (non-deuterated, non-substituted) compound.

Regarding providing novel sites for deuterating Formula II, a significant metabolic pathway of Formula II was found to comprise its N-acetylation via N-acetyltransferase 2 (NAT-2), which produces 1-(2-acetyl-5-methyl-2,3,4,5-tetrahydro-1H-pyrido[4,3-b]indol-7-yl)-4-((5-fluoropyridin-2-yl)methoxy)pyridin-2(1H)-one, i.e., the structure below:

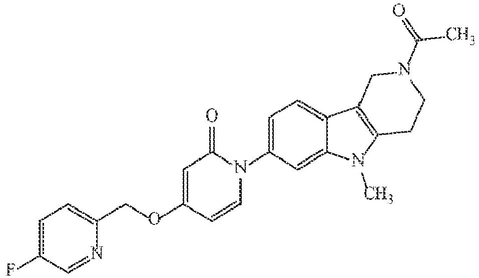

The amount of this metabolite was found to be very significant comparing to parent compound Formula II in healthy volunteers in phase I clinical trial. This metabolite does not contribute to the pharmacology of MCHR1 antagonism. Combining Formula II with a NAT-2 inhibitor such as acetaminophen is expected to reduce this metabolite and increase the effective concentration of Formula II in patients, hence lowering the dose needed. By substituting the hydrogens with deuterium on the carbon(s) adjacent to the nitrogen which is acetylated (see for example, Examples 5 to 7), acetylation process can be slowed down to reduce the formation of this metabolite.

### Stereoisomers

In alternative embodiments, compounds, active agents or drugs as provided herein, or as used to practice methods as provided herein, exist as (comprise) individual respective stereoisomers that are substantially free from another possible stereoisomer. In alternative embodiments, the term "substantially free of other stereoisomers" as used herein means less than about 15%, 20%, 25%, 30%, 35%, 40%, 50% or 55% of other stereoisomers, or less than about 10% of other stereoisomers, or less than about 5% of other stereoisomers, or less than about 2% of other stereoisomers, or less than about 1% or less of other stereoisomers, or less than "X"% of other stereoisomers (wherein X is a number between 0 and 100, inclusive) are present. Methods of obtaining or synthesizing an individual enantiomer for a given compound are known in the art and may be applied as practicable to final compounds or to starting material or intermediates.

### Methods of administration

In alternative embodiments, drugs or compounds are provided herein, or drugs or compounds used to practice methods are provided herein, are administered by any or a variety of means including orally, parenterally, by inhalation spray, nasally, topically, intrathecally, intrathecally, intracerebrally, epidurally, intracranially or rectally. Drugs or compounds are provided herein, or drugs or compounds used to practice methods are provided herein, are administered with pharmaceutically acceptable carriers, adjuvants and vehicles. In alternative embodiments, drugs or compounds as provided herein, or a composition used to practice the methods as provided herein, are administered intrathecally, intracerebrally or epidurally (into a intrathecal, intracerebral, epidural space), subcutaneously, intravenously, intramuscularly and/or intraarterially; for example, by injection routes but also including a variety of infusion techniques. Intraarterial, intrathecal, intracranial, epidural, intravenous and other injections can include administration through catheters or pumps, for example, an intrathecal pump, or an implantable medical device (which can be an intrathecal pump or catheter).

In alternative embodiments, therapeutic combinations of drugs, pharmaceutical compositions, preparations and kits, can be administered by any known method or route, including by intranasal, intramuscular, intravenous, topical or oral, or combinations thereof, routes.

One embodiment comprises a product of manufacture comprising a pharmaceutical composition or a formulation, a blister package, a lidded blister or a blister card or packet, a clamshell, a tray or a shrink wrap, or a kit, comprising: therapeutic combinations of drugs, pharmaceutical compositions or preparations as provided herein for oral administration.

In alternative embodiments, although all ingredients can be in one blister package, a lidded blister or a blister card or packet, a clamshell, a tray or a shrink wrap, or a kit, separate ingredients can be formulated for example, for topical application, for oral or for topical application. Each ingredient can be either separately packaged, or can be formulated as one unit dose, for example, as one tube (for example, with gel, lotion etc.), ampoule, blister packette and the like.

### Dosages

In alternative embodiments, drugs or compounds as provided herein, or a composition used to practice the methods as provided herein, are formulated and administered in a variety of different dosages and treatment regimens, depending on the disease or condition to be ameliorated, the condition of the individual to be treated, the goal of the treatment, and the like, as to be routinely determined by the clinician, see for example, the latest edition of Remington: The Science and Practice of Pharmacy, Mack Publishing Co., supra.

In alternative embodiments, an effective amount of a drug or compound as provided herein, or a composition used to practice the methods as provided herein, including a stereoisomer, salt, hydrate or solvate, is between about 0.1 mg and about 20.0 mg per kg of body weight of the individual or subject (for example, patient). In another variation, the effective amount is between about 0.1 mg and about 10.0 mg per kg of body weight of the individual or subject (for example, patient) or between about 0.1 mg and about 5.0 mg per kg of body weight of the patient. Alternately, the effective amount is between about 0.2 mg and about 2 mg per kg of body weight of the individual or subject (for example, patient).

In alternative embodiments, an effective amount of a drug or compound as provided herein, or a composition used to practice the methods as provided herein (for example, as a solid dosage, such as a pill, tablet or lozenge) is between about 0.1 mg and about 2.0 mg per kg of body weight of said individual, subject or patient; or is between about 0.1 mg and about 1.0 mg per kg of body weight; or is about 0.1 mg, about 0.15 mg, about 0.2 mg, about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, or about 1.0 mg, per kg of body weight; or an effective amount of a drug or compound as provided herein, or a composition used to practice the methods as provided herein, is about 0.1 mg, about 0.15 mg, about 0.2 mg, about 0.25 mg or about 0.3 mg per kg of body weight.

In alternative embodiment, an effective amount (for example, as a solid dosage, such as a pill, tablet or lozenge) of a drug or compound as provided herein, or a composition used to practice the methods as provided herein, is between about 1 mg and about 400 mg; or is a solid dosage form comprising between about 1 mg and about 250 mg; or the solid dosage form comprises between about 5 mg and about 150; or the solid dosage form (for example, as a pill, tablet or lozenge) comprises between about 1 mg and about 75; or the solid dosage form comprises about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, or about 75 mg.

### Methods for safe and predictable titration to an effective dose

In alternative embodiments, provided is an empirical method for safe and predictable titration and to determine the initial therapeutic dose, or to determine the lowest therapeutic dose, or to determine an optimal effective dose for any individual drug as used in methods as provided herein (for example, as when that drug is used alone), or for any combination of drugs as provided herein; the method comprises:
a. Administrating a test dose (for example, a no adverse events observed dose amount or a minimal adverse events observed dose amount or a safe dose amount) daily until a patient achieves a steady state plasma drug concentration,
b. Prior to the next scheduled dose, drawing a blood sample and assessing the test plasma drug concentration,
c. Calculating the initial therapeutic dose/day by:
   1. For drugs with a linear dose to plasma concentration, divide the initial target therapeutic plasma drug concentration by the test plasma drug concentration, and then multiply the dividend by the test dose amount, and
   2. For drugs with a non-linear dose to plasma concentration, divide the initial target therapeutic plasma drug concentration by the test plasma drug concentration, then multiply the dividend by the test dose amount and then multiply the product by a non-linear index factor, optionally, the initial target therapeutic plasma drug concentration is the concentration corresponding to: (i) certain pharmacodynamic efficacy marker levels, such as occupancy of serotonin transporter (SERT or 5-HTT), dopamine transporter (DAT) or norepinephrine transporter (NET) determined by imaging method such as positron emission tomography (PET); or, (ii) the lowest effective therapeutic dose determined by a clinical study.
d. Optionally comprising:
   i. administrating the initial therapeutic dose, daily, until a patient achieves a steady state plasma drug concentration,
   ii. prior to the next scheduled dose, drawing a blood sample and assessing the current therapeutic plasma drug concentration,
   iii. Calculating the next therapeutic target dose/day by:
      1. For drugs with a linear dose to plasma concentration, divide the next target therapeutic plasma drug concentration by the current therapeutic plasma drug concentration and then multiply the dividend by the current therapeutic dose amount, and
      2. For drugs with a non-linear dose to plasma concentration, divide next target therapeutic plasma drug concentration by the current therapeutic plasma drug concentration, then multiply the dividend by the current therapeutic dose amount and then multiply the product by a non-linear index factor, and
   iv. continuing the process until the lowest therapeutic dose or optimal effective dose is achieved.

In alternative embodiments, provided is a model-based method for safe and predictable titration, and to determine the initial therapeutic dose and to determine the lowest therapeutic dose, or to determine an optimal effective dose, and method comprising:
a. Administrating a test dose amount daily for between about 1 to 28 days, 1 to 21 days, 1 to 14 days, 1 to 10 days, 1 to 7 days, 1 to 3 days, or about 1 day,
b. Drawing a first blood sample at between about 1 hour to 3 days, 6 hours to 2 days or about 1 day after administering the first test dose,
c. Drawing a second or subsequent blood samples at between about 1 hour to 28 days, 6 hours to 14 days, 1 day to 7 days, or at about 2 days, after drawing of the first blood samples or subsequent blood samples,
d. calculating the initial therapeutic starting dose/day by:
   i. using the initial target therapeutic plasma drug concentration, the test dose amount, the time at the administration of the test dose(s), the plasma concentration of the blood samples and the time the draws of the blood samples and a pharmacometric model,
   ii. and optionally, using the initial target therapeutic plasma drug concentration, the test dose amount, the time at the administration of the test dose(s), the plasma concentration of the blood samples, the time of the draws of the blood samples, human PK data and a Bayesian pharmacometric model,
   iii. and optionally, using the initial target therapeutic plasma drug concentration, the test dose amount, the time at the administration of the test dose, the plasma concentration of the blood samples, the time of the draws of the blood samples, human PK data, a patient's genetic information, a patient's non-heritable host factors, a patient's other medications, and a Bayesian pharmacometric model,
e. and optionally comprising:
   i. administrating the initial therapeutic starting dose, daily until a patient achieves a steady state plasma drug concentration,
   ii. Prior to the next scheduled dose, drawing a blood sample and assessing the current therapeutic plasma drug concentration,
   iii. calculating the next therapeutic target dose/day:
      1. For drugs with a linear dose to plasma concentration, divide the next target therapeutic plasma drug concentration by the current therapeutic plasma drug concentration and then multiply the dividend by the current therapeutic dose amount, and
      2. For drugs with a non-linear dose to plasma concentration, divide he next target therapeutic plasma drug concentration by the current therapeutic plasma drug concentration and then multiply the dividend by the current therapeutic dose amount and then multiply the product by the a non-linear index factor, and
   iv. Continuing the process until the lowest therapeutic dose or optimal effective dose is achieved.

In alternative embodiments, provided is a method to maintain an effective dose, wherein the method comprises:
(a) recording the maintenance dose amount and the maintenance therapeutic plasma drug concentration associated with the lowest effective therapeutic dose or optimal therapeutic dose,
(b) at the early of between about 1 to 12 months, 3 to 9 month 4 to 8 months or 6 months or change in body weight, medication or health status then:
   1. prior to the next scheduled dose, draw a blood sample and assess the current plasma drug concentration
   2. if the current plasma drug concentration is different from the maintenance therapeutic plasma drug concentration by more than about 20%, optionally more than about 50%, then calculate a new therapeutic dose/day by a method comprising:
      i. For drugs with a linear dose to plasma concentration, divide the maintenance therapeutic plasma drug concentration by the current plasma drug concentration, and then and multiple the dividend by the maintenance dose amount, and
      ii. For drugs with a non-linear dose to plasma concentration, divide the maintenance therapeutic plasma drug concentration by the current plasma drug concentration, then multiply the dividend by the maintenance dose amount, and then multiply the product by a non-linear index, or
      iii. optionally, reaching steady state after two subsequent measured plasma drug concentration results at least one week apart and within about 20% of the maintenance therapeutic plasma concentration, pr
      iv. optionally, continuing the process until a maintenance therapeutic dose at steady state is achieved.

In alternative embodiments, there is need for rounding of the lowest therapeutic dose or optimal effective dose amount to the closest available dosage amount. For example, the calculation might determine the optimal effective dose amount is 23 mg/day, however the closest available dosage amount might be only 20 mg. Therefore the patient would be administered only 20 mg/day.

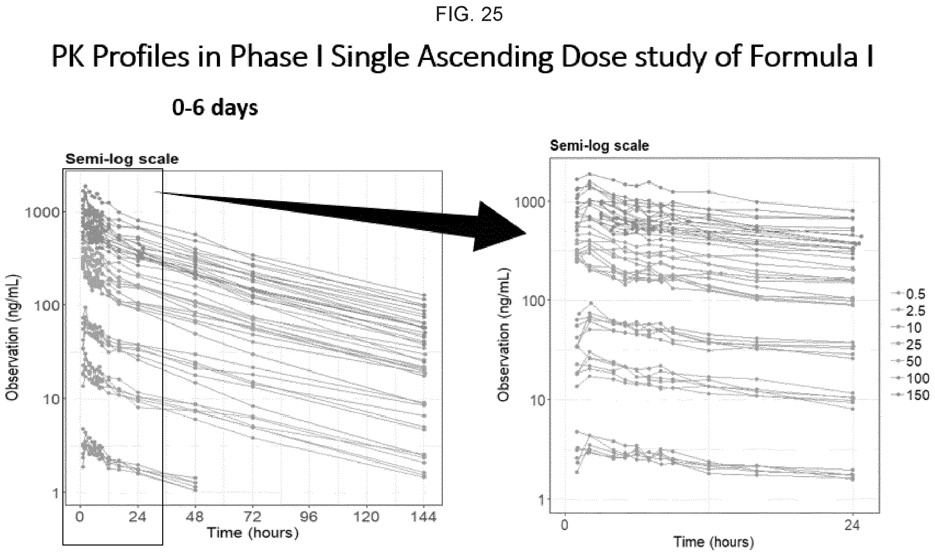

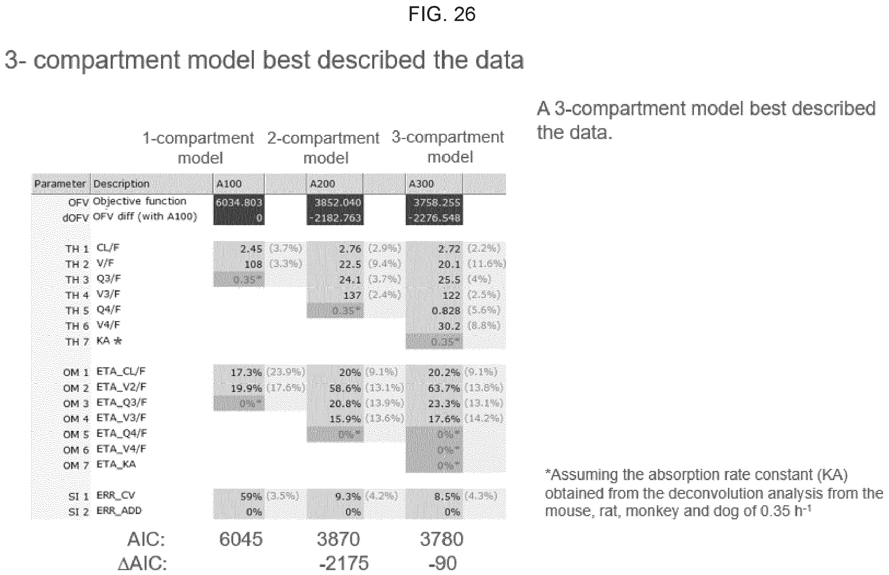

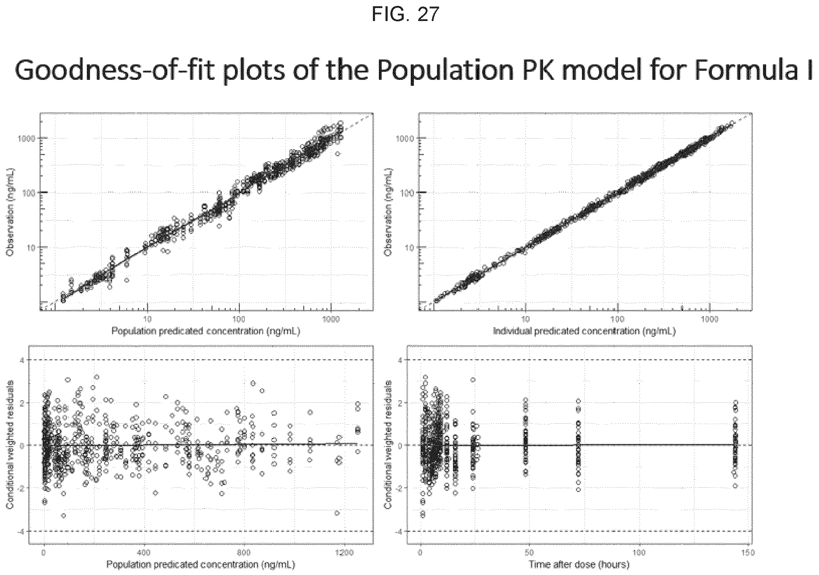

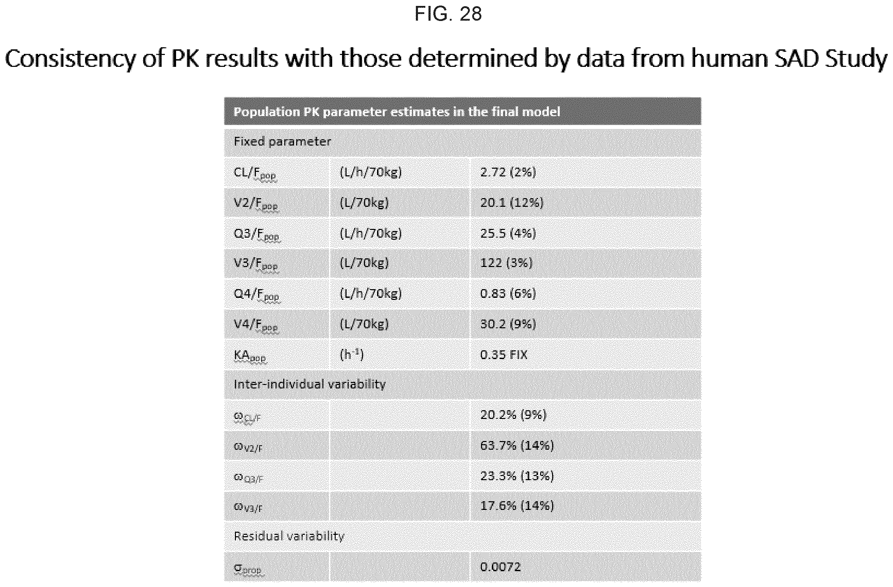

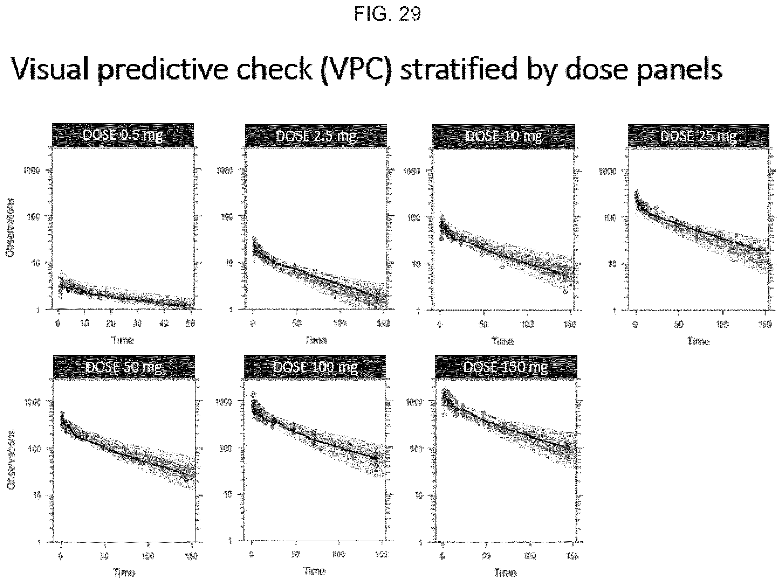

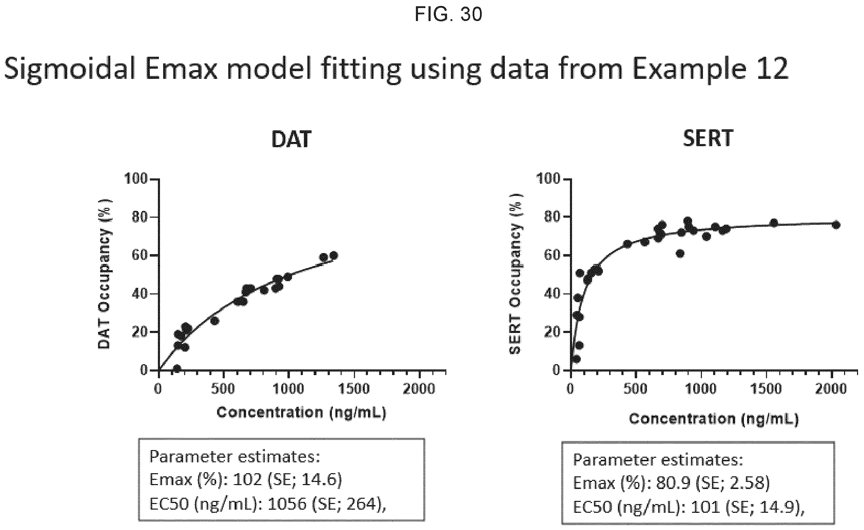

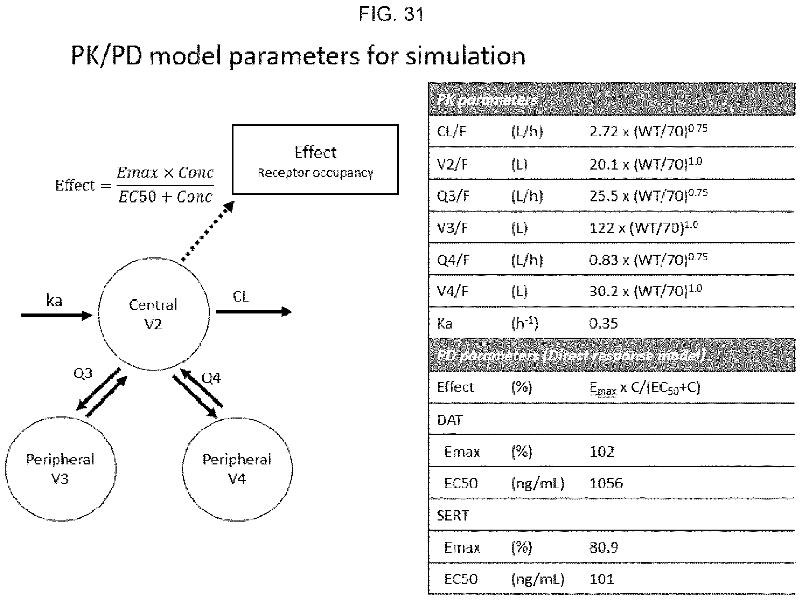

In alternative embodiments, provided are pharmacokinetic modeling and simulation methods for Formula I, comprising using human PK data, as described sequentially in FIG. 25 to FIG. 29. A 3-compartment model best described the human PK data. Between-patient variability in healthy volunteers were 20.2%. Visual predictive check (VPC) plots indicated that the final model was unbiased.In alternative embodiments, is a population PK/PD model for formula I, using human PK data as described in FIG. 30 and FIG. 31.

In alternative embodiments, provided are PK-guided, precision dosing methods using the above mentioned pharmacokinetic modeling and simulation methods, or similar modeling and simulation methods, and a population PK/PD model of formula I to treat hyperphagia, hyperphagia in PWS, Disruptive Mood Dysregulation Disorder (DMDD), Oppositional Defiant Disorder (ODD), obesity in hypothalamic-injury induced obesity, and binge eating disorder (BED) with an oral dosage of Formula I.

### Packaging and Drug Delivery Systems

In alternative embodiments, provided are therapeutic combinations, preparations, formulations and/or kits, comprising combinations of ingredients, as described herein. In one aspect, each member of the combination of ingredients is manufactured in a separate package, kit or container; or, all or a subset of the combinations of ingredients are manufactured in a separate package or container. In alternative aspects, the package, kit or container comprises a blister package, a clamshell, a tray, a shrink wrap and the like.

In one aspect, the package, kit or container comprises a "blister package" (also called a blister pack, or bubble pack). In alternative embodiments, provided are therapeutic combinations, preparations, formulations and/or kits manufactured as "blister packages" or as a plurality of packettes, including as lidded blister packages, lidded blister or blister card or packets or packettes, or a shrink wrap.

In one aspect, the blister package is made up of two separate elements: a transparent or occlusive plastic cavity shaped to the product and its blister foil backing. These two elements are then sealed together into a blister strip of one or more blister with each blister an environmentally (for example moisture, pathogen, light) protected unit dose. One or more blister strips can be further joined with board material which allows the product to be package, handled, hung, displayed or shipped without damaging the blister seal and provided child resistant features. Exemplary types of "blister packages" include: Face seal blister packages, gang run blister packages, mock blister packages, interactive blister packages, slide blister packages.

Blister packs, clamshells or trays are forms of packaging used for goods; thus, provided are blister packs, clamshells or trays comprising a composition (for example, a (the multi-ingredient combination of drugs as provided herein) combination of active ingredients) as provided herein. Blister packs, clamshells or trays can be designed to be non-reclosable, so consumers can tell if a package has already opened. They are used to package for sale goods where product tampering is a consideration, such as the pharmaceuticals as provided herein. In one aspect, a blister pack as provided herein comprises a molded PVC base, with raised areas (the "blisters") to contain the tablets, pills, etc. comprising the combinations as provided herein, covered by a foil laminate. Tablets, pills, etc. are removed from the pack either by peeling the foil back or by pushing the blister to force the tablet to break the foil. In one aspect, a specialized form of a blister pack is a strip pack. In one aspect, in the United Kingdom, blister packs adhere to British Standard 8404.

In alternative embodiments, laminated aluminum foil blister packs are used, for example, for the preparation of drugs designed to dissolve immediately in the mouth of a patient. This exemplary process comprises having the drug combinations, therapeutic combinations and pharmaceutical dosage forms as provided herein prepared as an aqueous solution(s) which are dispensed (for example, by measured dose) into an aluminum (for example, alufoil) laminated tray portion of a blister pack. This tray is then freeze-dried to form tablets which take the shape of the blister pockets. The alufoil laminate of both the tray and lid fully protects any highly hygroscopic and/or sensitive individual doses. In one aspect, the pack incorporates a child-proof peel open security laminate. In one aspect, the system gives tablets an identification mark by embossing a design into the alufoil pocket that is taken up by the tablets when they change from aqueous to solid state. In one aspect, individual 'push-through' blister packs/ packettes are used, for example, using hard temper aluminum (for example, alufoil) lidding material. In one aspect, hermetically-sealed high barrier aluminum (for example, alufoil) laminates are used. In one aspect, any products of manufacture as provided herein, including kits or blister packs, use foil laminations and strip packs, stick packs, sachets and pouches, peelable and non-peelable laminations combining foil, paper, and film for high barrier packaging.

In alternative embodiments, any products of manufacture as provided herein, including kits or blister packs, include memory aids to help remind patients when and how to take the drug. This safeguards the drug's efficacy by protecting each pill until it's taken; gives the product or kit portability, makes it easy to take a dose anytime or anywhere.

In alternative embodiments, drug combinations, therapeutic combinations, pharmaceutical dosage forms, drug delivery devices and products of manufacture as provided herein, use child resistant and elderly friendly packaging, for example, packaging compliant to U.S. Government child resistant packaging regulation that requires minimal finger and grip strength. For example, in alternative embodiments foil-only containment of pills is used.

In alternative embodiments, drug combinations, therapeutic combinations, pharmaceutical dosage forms, drug delivery devices and products of manufacture as provided herein, please tablets, capsules, pills or equivalents on a blister card or equivalent to track usage. By tracking usage, the blister card monitor can remind the patient and/or the primary care-giver to take medication (or that medication has been taken) at the correct time, for example, in AM and/or PM; and can facilitate discussion with health care professionals to identify and overcome barriers to adherence.

In alternative embodiments, patient usage is monitored by use of customized blister cards or equivalents using an Electronic Compliance Monitor (ECM) system (Intelligent Devices SEZC Inc. (IDI), Grand Cayman, Cayman Islands), or equivalents. For example, in alternative embodiments, the blister cards or equivalents comprise an electronic component that detects, records, safeguards and/or transmits medication removal from the blister cards or equivalents. For example, a sensor detects medication removal from the blister cards or equivalents, and this information can be transferred to a remote location for review by for example, the drug provider and/or the primary care institution or individuals. The data transfer can be by hard contact downloading of data to a transmitting and/or storage device, and can be scanned and data downloaded remotely using a radio-frequency identification (RFID) chip, tag or device or equivalent, which can be operatively connected to a computer and/or a mobile phone or other device. Radio-frequency identification uses electromagnetic fields to automatically identify and track tags attached to objects, where the tags contain electronically stored information, which in this embodiment is transmitting whether and/or when medication is removed from each compartment of the blister cards or equivalents, or by Near-Field Communication (NFC) to a NFC-enabled mobile device or mobile phone. The NFC is a set of communication protocols that enable two electronic devices, one of which is usually a portable device such as a smartphone, to establish communication by bringing them within 4 cm (1.6 in) of each other.

In alternative embodiments, multi-drug delivery systems as used in methods as provided herein can comprise use of a box to house or enclose drug delivery devices or packages, blister packages, clamshells or trays, as provided herein, where in this exemplary delivery system a week of pharmaceutical dosage form (for example, one, two or three or more tablets, pills, capsules, geltabs or equivalents) are stored on four rows, two rows for administration (for opening and self-administering by user, for example, patient) are for morning or breakfast, or AM administration, and two rows are for evening, dinnertime or PM administration; morning or breakfast, or AM administration rows are clearly separated from the evening, dinnertime or PM administration rows, and each day, and the spare dose, are arranged in column form. In alternative embodiments the blister packages, clamshells or trays are physical linked to a storage box, wherein the blister packages, clamshells or trays slide into and out of the storage box, and in alternative embodiments if needed the PM set of rows can be folded over the AM set of rows for reinsertion of the blister packages, clamshells or trays into the storage box. In alternative embodiments, the storage box comprises sensors to detect medication removal from each of the compartments (for example, which compartment is opened and when), and this information can be transferred to a remote location, for example, by Near-Field Communication (NFC) to a NFC-enabled mobile device or mobile phone, for review by for example, the drug provider and/or the primary care institution or individuals.

In any embodiment of methods as provided herein, the plasma concentration for the drug, or therapeutic combination, pharmaceutical dosage form is: (a) the trough level or trough concentration (C_{trough}), or the lowest concentration reached by the drug, or therapeutic combination or pharmaceutical dosage form before a second or next dose is administered, or (b) determined from blood samples taken between about 0.5 hours to 24 hours, or 4 to 12 hours, or 1, 2, 3, 4, 5, 6, 7, 8, 9, or 10 or more hours, after the last dose or administration of the drug, or therapeutic combination, pharmaceutical dosage form.

In alternative embodiments, "Individualized dosing" and "PK-guided dosing", "precision dosing" are interchangeable terms, mean administering each patient by a drug's PK properties in the patient.

In alternative embodiments, terms of "plasma concentration", "plasma drug concentration", "serum concentration", "serum drug concentration", "plasma level", "level" in the context of drug concentration, are interchangeable, and mean drug concentration in humans or animals, and measured by and interpreted by skilled in the art see Loftsson T. Essential Pharmacokinetics - 1st Edition. Elsevier. 2015.

In alternative embodiments, "target therapeutic plasma drug concentration", or a similar term, is within the therapeutic plasma range, optionally about the mid-point of a therapeutic level range of plasma concentration. For example, if the therapeutic, ie, the effective or efficacious plasma concentration range for treating PWS is between 1000 ng/ml to 1400 ng/ml, the target therapeutic plasma drug concentration can be about 1000 ng/ml, about 1300 ng/ml, or optionally the mid-point about 1200 ng/ml.

In alternative embodiments, therapeutic in the context of plasma concentration means effective or efficacious in treating a medical condition(s).

Any of the above aspects and embodiments can be combined with any other aspect or embodiment as disclosed here in the Summary, Figures and/or Detailed Description sections.

As used in this specification and the claims, the singular forms "a," "an" and "the" include plural referents unless the context clearly dictates otherwise.

Unless specifically stated or obvious from context, as used herein, the term "or" is understood to be inclusive and covers both "or" and "and".

Unless specifically stated or obvious from context, as used herein, the term "about" is understood as within a range of normal tolerance in the art, for example within 2 standard deviations of the mean. About (use of the term "about") can be understood as within 20%, 19%, 18%, 17%, 16%, 15%, 14%, 13%, 12% 11%, 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

Unless specifically stated or obvious from context, as used herein, the terms "substantially all", "substantially most of", "substantially all of' or "majority of' encompass at least about 75%, 80%, 85%, 90%, 95%, 97%, 98%, 99% or 99.5%, or more of a referenced amount of a composition. For example, in alternative embodiments, the term "substantially pure" refers to chemical purity, for example, a "substantially pure" compounds as provided or used in a therapeutic formulation or combination as provided herein (for example, comprising Formula I) is at least about 75%, 80%, 85%, 90%, 95%, 97%, 98%, 99% or 99.5%, pure or free of contaminants or other forms, for example, free of any form but the S-enantiomer of Formula I.

The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents. Incorporation by reference of these documents, standing alone, should not be construed as an assertion or admission that any portion of the contents of any document is considered to be essential material for satisfying any national or regional statutory disclosure requirement for patent applications. Notwithstanding, the right is reserved for relying upon any of such documents, where appropriate, for providing material deemed essential to the claimed subject matter by an examining authority or court.

Modifications may be made to the foregoing without departing from the basic aspects of the invention. Although the invention has been described in substantial detail with reference to one or more specific embodiments, those of ordinary skill in the art will recognize that changes may be made to the embodiments specifically disclosed in this application, and yet these modifications and improvements are within the scope and spirit of the invention. The invention illustratively described herein suitably may be practiced in the absence of any element(s) not specifically disclosed herein. Thus, for example, in each instance herein any of the terms "comprising", "consisting essentially of', and "consisting of' may be replaced with either of the other two terms. Thus, the terms and expressions which have been employed are used as terms of description and not of limitation, equivalents of the features shown and described, or portions thereof, are not excluded, and it is recognized that various modifications are possible within the scope of the invention. Embodiments of the invention are set forth in the following claims.

The invention will be further described with reference to the examples described herein; however, it is to be understood that the invention is not limited to such examples.

### EXAMPLES

### Example 1: Exemplary methods for the treatment of Prader-Willi syndrome by administering Formula I or a deuterated analogue thereof

This example describes exemplary methods and compositions for the treatment of Prader-Willi syndrome by administering a pharmaceutical composition comprising Formula I, or 7-([1,2,4]triazolo[1,5-a]pyridin-6-yl)-4-(3,4-dichlorophenyl)-1,2,3,4-tetrahydroisoquinoline.

Male and female patients (age 4 and above) with a documented medical record history of PWS confirmed by genetic testing are chosen for treatment.

A patient is given a low (optionally subtherapeutic) dose of the pharmaceutical composition of between 1 mg to 20 mg per day for one day, one week, two weeks, three weeks or four weeks. Blood sample is taken periodically to determine drug concentration at steady state and to determine the nearest target dose needed to achieve dopamine transporter (DAT) occupancy of between 15% to 75% with or without the aid of a pharmacometrics modeling method that also utilizes the pharmacokinetic/pharmacodynamic (PK/PD) data; optionally the nearest target dose is determined by achieving a plasma drug concentration value between 100 ng/ml to 4000 ng/ml at steady state; optionally the nearest target dose is determined by achieving a plasma drug concentration value between 200 ng/ml to 2000 ng/ml at steady state; optionally the nearest target dose is determined by achieving a plasma drug concentration value between 400 ng/ml to 2000 ng/ml at steady state. Every 3 months or 6 months, or when a patient has significant changes in medications, body weight and other physical changes can impact the drug absorption and metabolism, blood drug level will be retested to adjust the dose if necessary; optionally, patients will start a low dose of between 5 mg to 20 mg per day and gradually increase dose by 5 mg or 10 mg or 20 mg interval until safely reach efficacy for hyperphagia and/or neuropsychiatric and behavioral symptoms. A clinician can accelerate titration, slow down the titrate if necessary, and re-accelerate titration later based on the tolerability of the patient.

### Example 2: Exemplary methods for the treatment of hypothalamic obesity or hypothalamic injury-induced obesity by administering Formula I or any deuterated analogue thereof

This example describes exemplary methods and compositions for the treatment of Hypothalamic obesity, or Hypothalamic injury-induced obesity by administering a pharmaceutical composition comprising Formula I, or 7-([1,2,4]triazolo[1,5-a]pyridin-6-yi)-4-(3,4-dichlorophenyl)-1,2,3,4-tetrahydroisoquinoline.

Male and female patients (age 4 and above) with diagnosed hypothalamic injury-induced obesity are chosen for treatment.

A patient is given a low (optionally, a subtherapeutic) dose of the pharmaceutical composition between 1 mg to 20 mg per day for one day, one week, two weeks, three weeks or four weeks. Blood sample is taken periodically to determine drug concentration at steady state and to determine the nearest target dose needed to achieve DAT occupancy of between 15% to 75% with or without the aid of a pharmacometrics modelling method that also utilizes the PK/PD data; optionally the nearest target dose is determined by achieving a plasma drug concentration value between 100 ng/ml to 4000 ng/ml at steady state; optionally the nearest target dose is determined by achieving a plasma drug concentration value between 200 ng/ml to 2000 ng/ml at steady state; optionally the nearest target dose is determined by achieving a plasma drug concentration value between 400 ng/ml to 2000 ng/ml at steady state, optionally the nearest target dose is determined by achieving a plasma drug concentration value between about 400 ng/ml to 2000 ng/ml at steady state. Every 3 months or 6 months, or when a patient has significant changes in medications, body weight and other physical changes can impact the drug absorption and metabolism, blood drug level will be retested to adjust the dose if necessary; optionally, patients will start a low dose of between 5 mg to 20 mg per day and gradually increase dose by 5 mg or 10 mg or 20 mg interval until safely reach efficacy for hyperphagia and/or neuropsychiatric and behavioral symptoms. A clinician can accelerate titration, slow down the titrate if necessary, and re-accelerate titration later based on the tolerability of the patient.

### Example 3: Exemplary methods for the treatment of Binge Eating Disorder (BED) by administering Formula I or any deuterated analogue thereof

### This example describes exemplary methods and compositions for the treatment of Binge Eating Disorder (BED)

Male and female patients with BED diagnosed with DCM-IV (DSM-IV: Diagnostic and Statistical Manual of Mental Disorders, fourth edition), optionally, male and female patients with BED diagnosed with DCM-IV and Major Depressive Disorder (MDD) diagnosed with DCM-IV; optionally, the BED is refractory BED, optionally the MDD is refractory MDD, optionally the diagnose of MDD involves video- recorded interviews, are chosen for treatment.

A patient is given a low (optionally, a subtherapeutic) dose of the pharmaceutical composition between 1 mg to 20 mg per day for one day, one week, two weeks, three weeks or four weeks. Blood sample is taken periodically to determine drug concentration at steady state and to determine the nearest target dose needed to achieve DAT occupancy of between 15% to 75% with or without the aid of a pharmacometrics modelling method that also utilizes the PK/PD data; optionally the nearest target dose is determined by achieving a plasma drug concentration value between 100 ng/ml to 4000 ng/ml at steady state; optionally the nearest target dose is determined by achieving a plasma drug concentration value between 200 ng/ml to 2000 ng/ml at steady state; optionally the nearest target dose is determined by achieving a plasma drug concentration value between 400 ng/ml to 2000 ng/ml at steady state. Every 3 months or 6 months, or when a patient has significant changes in medications, body weight and other physical changes can impact the drug absorption and metabolism, blood drug level will be retested to adjust the dose if necessary; optionally, patients will start a low dose of between 5 mg to 20 mg per day and gradually increase dose by 5 mg or 10 mg or 20 mg interval until safely reach efficacy for BED. A clinician can accelerate titration, slow down the titrate if necessary, and re-accelerate titration later based on the tolerability of the patient.

### Example 4:

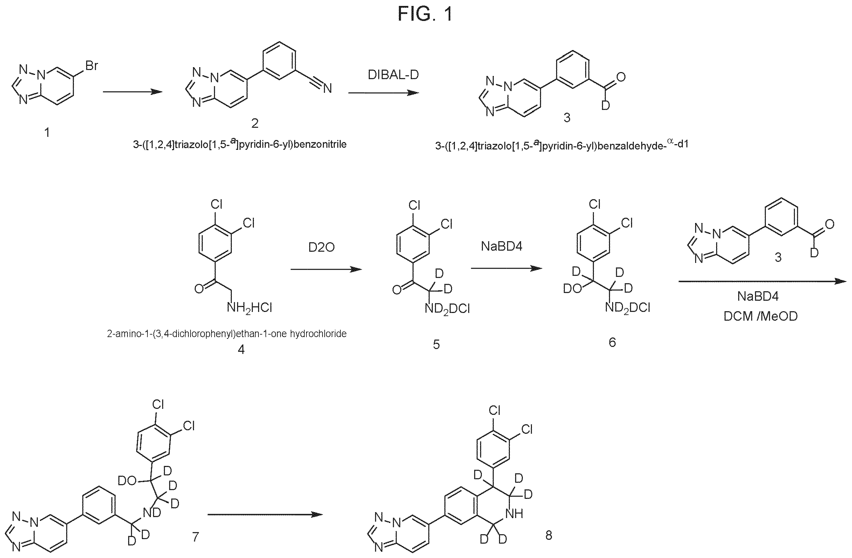

This example describes an exemplary protocol for synthesizing the exemplary compound 7-([1,2,4]triazolo[1,5-a]pyridin-6-yl)-4-(3,4-dichlorophenyl)-1,2,3,4-tetrahydroisoquinoline-1,1,3,3,4-d5, as schematically illustrated in FIG. 1.

Ketone 4, 2-amino-1-(3,4-dichlorophenyl)ethan-1-one hydrochloride, is converted to intermediate 5 under active proton deuterated conditions. Intermediate 5 is reduced by sodium borodeuteride to form alcohol 6. Reductive amination of deuterated aldehyde 3, 3-([1,2,4]triazolo[1,5-a]pyridine-6-yl)benzaldehyde-α-d1, which is prepared via reduction of intermediate 2, 3-([1,2,4]triazolo[1,5-a]pyridine-6-yl)benzonitrile with DIBAL-D (Diisobutylaluminum deuteride), with deuterated amine 6 via sodium borodeuteride afford intermediate 7. Cyclization of intermediate 7 under acidic condition provide target molecule 8, - ([1,2,4]triazolo[1,5-a]pyridin-6-yl)-4-(3,4-dichlorophenyl)-1,2,3,4-tetrahydroisoquinoline-1,1,3,3,4-d5.Example 4.1: Preparation of 7-([1,2,4]triazolo[1,5-a]pyridin-6-yl)-4-(3,4-dichlorophenyl)-1,2,3,4-tetrahydroisoquinoline-1,1,3,3,4-d5 8A and 8B

### Preparation of 2-(amino-d₂)-1-(3,4-dichlorophenyl)ethan-1-one-2,2-d2 DCl salt 5:

A mixture of 2-amino-1-(3,4-dichlorophenyl)ethan-1-one hydrochloride **4** (3.0 g, 12.5 mmol), D₂O (20 mL), THF (20 mL) and DCl (20%, 8 mL) in a sealed tube was stirred at 95 °C for 24 h. The isotopic purity was determined by mass spectroscopy (91% D). Volatiles were removed in vacuo. The procedure was repeated. The product was obtained by concentration in vacuo as an off-white solid (>99% D). ESI-MS (M+1) m/z: 206

### Preparation of 2-(amino-d2)-1-(3,4-dichlorophenyl)ethan-1,2,2-d3-1-ol-d 6:

To a solution of 2-(amino-d₂)-1-(3,4-dichlorophenyl)ethan-1-one-2,2-d₂ DCl salt **5** (367 mg, 1.50 mmol) in CH₃OD (2.8 mL) at 0 °C was added NaBD₄ (61 mg, 1.46 mmol). The mixture was stirred at rt overnight. A few drops of D₂O were added to quench the reaction. The mixture was concentrated in vacuo to dryness to provide crude title compound 6.

### Preparation of 2-(((3-([1,2,4]triazolo[1,5-a]pyridin-6-yl)phenyl)methyl-d2)amino-d)-1-(3,4-dichlorophenyl)ethan-1,2,2-d3-1-ol-d 7:

To a solution of the above residue 6 (1.50 mmol) in CH₃OD (2.8 mL) was added (3-([1,2,4]triazolo[1,5-a]pyridin-6-yl)benzaldehyde-□-d1 **3** (336 mg, 1.50 mmol) and DCM (1.5 mL). The mixture was stirred at rt for 2h before NaBD₄ (61 mg, 1.46 mmol) was added. The resulting mixture was stirred at rt overnight. The mixture was concentrated in vacuo and the residue was partitioned between DCM and 4N NH₄OH. The aqueous phase was extracted with DCM (2X). The combined extracts were dried over Na₂SO₄, filtered and concentrated in vacuo to give crude product (610 mg).

### Preparation of tert-butyl 7-([1,2,4]triazolo[1,5-a]pyridin-6-yl)-4-(3,4-dichlorophenyl)-3,4-dihydroisoquinoline-2(1H)-carboxylate-1,1,3,3,4-d5 8-Boc:

To a cold sulfuric acid (2.15 mL) at 0 °C was added dropwise a solution of 2-(((3-([1,2,4]triazolo[1,5-a]pyridin-6-yl)phenyl)methyl-d2)amino-d)-1-(3,4-dichlorophenyl)ethan-1,2,2-d3-1-ol-d 7 (610 mg) in DCM (1.9 mL). The mixture was stirred and allowed to warm to rt overnight. Ice-water was added to the reaction and the pH was brought to 9-10 by adding 20% NaOH. The mixture was extracted with DCM (3X) and the combined extracts were dried over Na₂SO₄, filtered and concentrated in vacuo to give crude residue which contained 7-([1,2,4]triazolo[1,5-a]pyridin-6-yl)-4-(3,4-dichlorophenyl)-1,2,3,4-tetrahydroisoquinoline-1,1,3,3,4-d5 8. The crude residue containing **8** was dissolved in DCM (20 mL). To the solution was added di-t-butyl dicarbonate (360 mg, 1.65 mmol). The resulting mixture was stirred at rt overnight and then purified by column chromatography (H: EtOAc from 2:1 to 1:2) to give tert-butyl 7-([1,2,4]triazolo[1,5-a]pyridin-6-yl)-4-(3,4-dichlorophenyl)-3,4-dihydroisoquinoline-2(1H)-carboxylate-1,1,3,3,4-d5 **8-Boc** (180 mg, 24% three steps). ESI-MS (M+1) m/z: 500; ¹HNMR (400 MHZ, CDCl3) δ 8.80 (s, 1H), 8.39 (s, 1H), 7.93-7.69 (m, 2H), 7.47-7.30 (m, 3H), 7.20 (s, 1H), 7.12-6.77 (m, 2H?), 1.64-1.14 (m, 9H).

### Chiral SFC separation:

The racemic tert-butyl 7-([1,2,4]triazolo[1,5-a]pyridin-6-yl)-4-(3,4-dichlorophenyl)-3,4-dihydroisoquinoline-2(1H)-carboxylate-1,1,3,3,4-d5 **8-Boc** (165 mg) was dissolved in 5 mL of MeOH and 5 mL of dichloromethane and then resolved by chiral SFC (Regis Whelk-O1 (S, S) 21 × 250 mm; Mobile Phase: 45% 2-Propanol + 0.25 DEA in CO2; 254 nM) to yield enantiomer A **8A-Boc** (88 mg, RT = 2.35 min, 100% ee) and enantiomer B **8B-Boc** (69 mg, RT = 2.77 min, 100%ee).

### Preparation of enantiomer 7-([1,2,4]triazolo[1,5-a]pyridin-6-yl)-4-(3,4-dichlorophenyl)-1,2,3,4-tetrahydroisoquinoline-1,1,3,3,4-d5 HCl salt 8A:

The mixture of the above **8A-Boc** and 4N HCl in dioxane (1 mL) was stirred at rt for 1 h. After removal of the volatiles, the residue was triturated with EtOAc (2 mL). The product enantiomer 7-([1,2,4]triazolo[1,5-a]pyridin-6-yl)-4-(3,4-dichlorophenyl)-1,2,3,4-tetrahydroisoquinoline-1,1,3,3,4-d5 HCl salt **8B** (60 mg) was obtained; ESI-MS (M+1) m/z: 400; ¹HNMR (400 MHz, DMSO) δ 9.8 (bs, 2H), 9.34 (s, 1H), 8.56 (s, 1H), 7.68 (m, 4H), 7.37-7.27 (m, 1H), 6.90 (d, J = 7.80 Hz, 1H).

### Preparation of enantiomer 7-([1,2,4]triazolo[1,5-a]pyridin-6-yl)-4-(3,4-dichlorophenyl)-1,2,3,4-tetrahydroisoquinoline-1,1,3,3,4-d5 HCl salt 8B:

The mixture of the above **8B-Boc** and 4N HCl in dioxane (1 mL) was stirred at rt for 1h. After removal of the volatiles, the residue was triturated with EtOAc (2 mL). The product enantiomer 7-([1,2,4]triazolo[1,5-a]pyridin-6-yl)-4-(3,4-dichlorophenyl)-1,2,3,4-tetrahydroisoquinoline-1,1,3,3,4-d5 HCl salt **8B** (50 mg) was obtained; ESI-MS (M+1) m/z: 400; ¹HNMR: identical to **8A**

### Example 5:

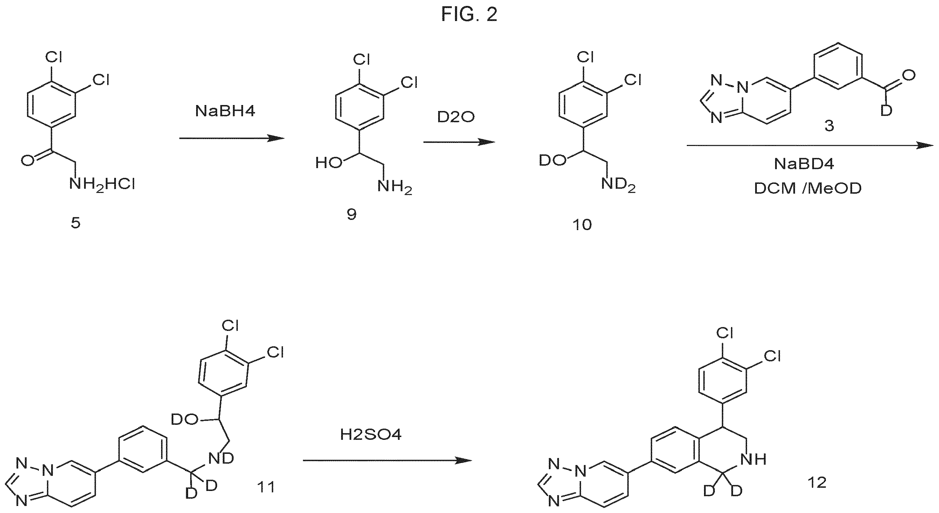

This example describes an exemplary protocol for synthesizing the exemplary compound 7-([1,2,4]triazolo[1,5-a]pyridin-6-yl)-4-(3,4-dichlorophenyl)-1,2,3,4-tetrahydroisoquinoline-1,1-d2, as schematically illustrated in FIG. 2. Compound 5 is reduced to compound alcohol **9 via** sodium borohydride, which is then converted to deuterated amine **10.** Reductive amination of deuterated aldehyde **3,** 3-([1,2,4]triazolo[1,5-a]pyridin-6-yl)benzaldehyde-□-d1, which is prepared via reduction of intermediate **2,** 3-([1,2,4]triazolo[1,5-a]pyridin-6-yl)benzonitrile with DIBAL-D (Diisobutylaluminum deuteride), with deuterated amine **10** via sodium borodeuteride affords intermediate **11.** Cyclization of intermediate **11** under acidic condition provides target molecule **12,** 7-([1,2,4]triazolo[1,5-a]pyridin-6-yl)-4-(3,4-dichlorophenyl)-1,2,3,4-tetrahydroisoquinoline-1,1-d2.

### Example 5.1: Preparation of 7-([1,2,4]triazolo[1,5-a]pyridin-6-yl)-4-(3,4-dichlophenyl)-1,2,3,4-tetrahydroisoquinoline-1,1-d2 12A and 12B

### Preparation of 2-(amino-d2)-1-(3,4-dichlorophenyl)ethan-1-ol-d 10:

To a solution of 2-amino-1-(3,4-dichlorophenyl)ethan-1-one hydrochloride **4** (288 mg, 1.2 mmol) in 2 mL of methanol at 0 °C was added NaBH₄ (33 mg, 0.79 mmol). The mixture was stirred at 0 °C for 30 min and then concentrated *in vacuo.* The residue was treated with D₂O/MeOD (0.5 mL/ 1.5 mL). After stirred at rt for 20 min, the mixture was concentrated *in vacuo.* The residue was used directly in next reactions.

### Preparation of (3-([1,2,4]triazolo[1,5-a]pyridin-6-yl)phenyl)methan-d-ol 3b:

To a solution of 3-([1,2,4]triazolo[1,5-a]pyridin-6-yl)benzaldehyde **3a** (2.51 g, 11.2 mmol) in CH₃OD (22 mL) at 0 °C was added NaBD₄ (265 mg, 6.33 mmol). The mixture was stirred at 0 °C for 30 min and then water (40 mL) was added. Filtration afforded (3-([1,2,4]triazolo[1,5-a]pyridin-6-yl)phenyl)methan-d-ol **3b** as a white solid (1.65 g, 65% yield).

### Preparation of (3-([1,2,4]triazolo[1,5-a]pyridin-6-yl)benzaldehyde-a-d1 3:

To a solution of (3-([1,2,4]triazolo[1,5-a]pyridin-6-yl)phenyl)methan-d-ol **3b** (1.65 g, 7.37 mmol) in CH₂Cl₂ (30 mL) at rt was added Dess-Martin Periodinane (3.75 g, 8.84 mmol). The mixture was stirred at rt overnight. Column chromatography purification gave (3-([1,2,4]tfiazolo[1,5-a]pyridin-6-yl)benzaldehyde-α-d1 3 (1.64 g, 99%, 74% D) as an off-white solid. ESI-MS (M+1) m/z: 225.

### Preparation of (3-([1,2,4]triazolo[1,5-a]pyridin-6-yl)phenyl)methan-d2-ol 3c:

To a solution of 3(3-([1,2,4]triazolo[1,5-a]pyridin-6-yl)benzaldehyde-a-d1 **3** (1.64 g, 7.37 mmol) in CH₃OD (20 mL) at 0 °C was added NaBD₄ (310 mg, 7.37 mmol). The mixture was stirred at 0°C for 30 min and then water (30 mL) was added. Filtration afforded (3-([1,2,4]triazolo[1,5-a]pyridin-6-yl)phenyl)methan-d2-ol **3c** as a white solid (1.40 g, 84% yield, 95% D).

### Preparation of (3-([1,2,4]triazolo[1,5-a]pyridin-6-yl)benzaldehyde-a-d1 3:

To a solution of (3-([1,2,4]tfiazolo[1,5-a]pyridin-6-yl)phenyl)methan-d2-ol **3c** (1.40 g, 6.25 mmol) in CH₂Cl₂ (30 mL) at rt was added Dess-Martin Periodinane (3.18 g, 7.50 mmol). The mixture was stirred at rt overnight. Column chromatography purification gave (3-([1,2,4]triazolo[1,5-a]pyridin-6-yl)benzaldehyde-□-d1 **3** (1.39 g, 99%, 92% D) as an off-white solid. ESI-MS (M+1) m/z: 225

### Preparation of2-(((3-([1,2,4]triazolo[1,5-a]pyridin-6-yl)phenyl)methyl-d2)amino)-1-(3,4-dichlorophenyl)ethan-1-ol 11:

A mixture of 2-(amino-*d*2)-1-(3,4-dichlorophenyl)ethan-1-ol-*d* **10** (210 mg, 0.99 mmol), (3-([1,2,4]triazolo[1,5-a]pyridin-6-yl)benzaldehyde-□-d1 **3** (220 mg, 0.99 mmol), DCM (1 mL) and CH₃OD (1.4 mL) was stirred at rt for 4 h. NaBD₄ (41 mg, 0.90 mmol) was added and the resulting mixture was stirred at rt for 2h. The mixture was concentrated in vacuo and the residue was partitioned between DCM and 4N NH₄OH. The aqueous phase was extracted with DCM (2X). The combined extracts were dried over Na₂SO₄, filtered and concentrated in vacuo to give crude product (440 mg or so, >99% D).

### Preparation of 7-([1,2,4]triazolo[1,5-a]pyridin-6-yl)-4-(3,4-dichlorophenyl)-1,2,3,4-tetrahydroisoquinoline-1,1-d2 12:

To a cold sulfuric acid (1.72 mL) at 0 °C was added dropwise a solution of 2-(((3-([1,2,4]triazolo[1,5-a]pyridin-6-yl)phenyl)methyl-d2)amino)-1-(3,4-dichlorophenyl)ethan-1-ol **11** in DCM (1.5 mL). The mixture was stirred and allowed to warm to rt overnight. Ice-water was added to the reaction and the pH was brought to 9-10 by adding 20% NaOH. The mixture was extracted with DCM (3X) and the combined extracts were dried over Na₂SO₄, filtered and concentrated in vacuo. The residue was purified by column chromatography to give title compound **12** (177 mg, 45% two steps). ESI-MS (M+1) m/z: 397; ¹HNMR (400 MHz, CDC13) δ 8.79 (s, 1H), 8.39 (s, 1H), 7.88-7.73 (m, 2H), 7.47-7.27 (m, 3H), 7.25-7.22 (s, 1H), 7.05-6.97 (m, 2H), 4.32-4.25 (m, 1H), 3.58-3.50 (m, 1H), 3.18-3.10 (m, 1H).

### Preparation of tert-butyl 7-([1,2,4]triazolo[1,5-a]pyridin-6-yl)-4-(3,4-dichlorophenyl)-3,4-dihydroisoquinoline-2(1H)-carboxylate-1,1-d2 12-Boc:

A solution of 7-([1,2,4]triazolo[1,5-a]pyridin-6-yl)-4-(3,4-dichlorophenyl)-1,2,3,4-tetrahydroisoquinoline-1,1-d2 12 (177 mg, 0.446 mmol) in DCM (4 mL) at rt was added Boc₂O (107 mg, 0.490 mmol). The mixture was stirred at rt overnight. The mixture was purified by column chromatography to give title compound **12-Boc** (160 mg).

### Chiral SFC (supercritical fluid chromatography) separation:

The racemic tert-butyl 7-([1,2,4]triazolo[1,5-a]pyridin-6-yl)-4-(3,4-dichlorophenyl)-3,4-dihydroisoquinoline-2(1H)-carboxylate-1,1-d2 **12-Boc** (139 mg) was dissolved in 5 mL of MeOH and 5 mL of dichloromethane and then resolved by chiral SFC (Regis Whelk-O1 (S, S) 21 × 250 mm; Mobile Phase: 45% 2-Propanol + 0.25 DEA in CO2; 254 nM) to yield enantiomer A **12A-Boc** (56 mg, RT = 2.35 min, 100% ee) and enantiomer B **12B-Boc** (56 mg, RT = 2.77 min, 100%ee).

### Preparation of enantiomer (7-([1,2,4]triazolo[1,5-a]pyridin-6-yl)-4-(3,4-dichlorophenyl)-1,2,3,4-tetrahydroisoquinoline-1,1-d2 HCl salt 12A:

The mixture of the above **12A-Boc** and 4N HCl in dioxane (1 mL) was stirred at room temperature (rt) for 1h. After removal of the volatiles, the residue was triturated with EtOAc (2 mL). The product enantiomer (7-([1,2,4]triazolo[1,5-a]pyridin-6-yl)-4-(3,4-dichlorophenyl)-1,2,3,4-tetrahydroisoquinoline-1,1-d2 HCl salt **12A** (50 mg) was obtained; ESI-MS (M+1) m/z: 397; ¹HNMR (400 MHz, DMSO) δ 9.8 (bs, 2H), 9.34 (s, 1H), 8.56 (s, 1H), 7.68 (m, 4H), 7.37-7.27 (m, 1H), 6.90 (d, J = 7.80 Hz, 1H), 4.61-4.39 (m, 1H), 3.76-3.62 (m, 1H), 3.50-3.45 (m, 1H).

### Preparation of enantiomer (7-([1,2,4]triazolo[1,5-a]pyridin-6-yl)-4-(3,4-dichlorophenyl)-1,2,3,4-tetrahydroisoquinoline-1,1-d2 HCl salt 12B:

The mixture of the above **12B-Boc** and 4N HCl in dioxane (1 mL) was stirred at rt for 1h. After removal of the volatiles, the residue was triturated with EtOAc (2 mL). The product enantiomer (7-([1,2,4]triazolo[1,5-a]pyridin-6-yl)-4-(3,4-dichlorophenyl)-1,2,3,4-tetrahydroisoquinoline-1,1-d2 HCl salt **12B** (50 mg) was obtained; ESI-MS (M+1) m/z: 397; ¹HNMR: identical to **12A.**

### Example 6:

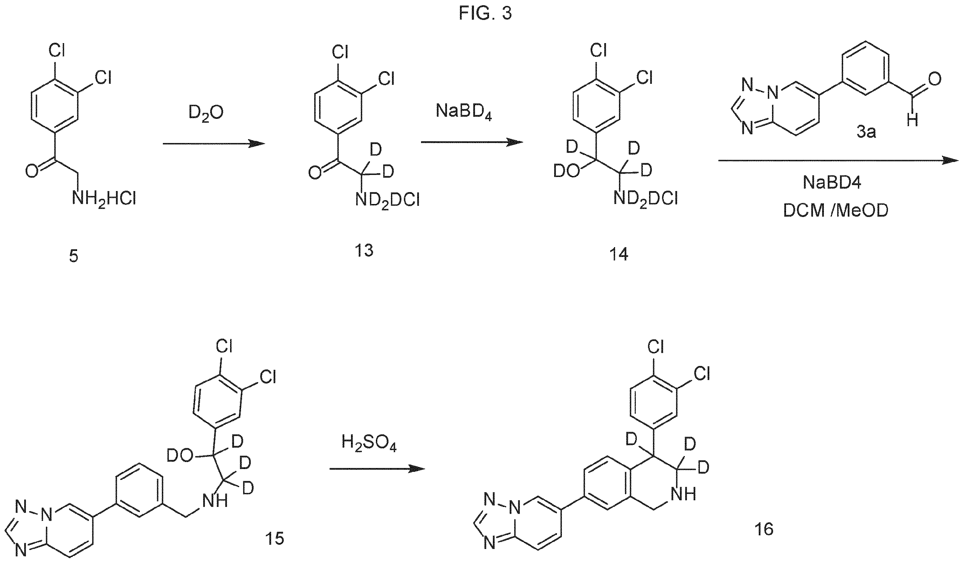

This example describes an exemplary protocol for synthesizing the exemplary compound 7-([1,2,4]triazolo[1,5-a]pyridin-6-yl)-4-(3,4-dichlorophenyl)-1,2,3,4-tetrahydroisoquinoline-3,3,4-d3, as schematically illustrated in FIG. 3.

Ketone **5,** 2-amino-1-(3,4-dichlorophenyl)ethan-1-one hydrochloride, is converted to intermediate **13** under active proton deuterated conditions. Intermediate **13** is reduced by sodium borodeuteride to form alcohol **14.** Reductive amination of aldehyde **3a,** 3-([1,2,4]tdazolo[1,5-a]pyridin-6-yl)benzaldehyde, which was prepared via reduction of intermediate **2,** 3-([1,2,4]triazolo[1,5-a]pyridin-6-yl)benzonitrile with DIBAL (Diisobutylaluminum hydride), with deuterated intermediate **14** via sodium borodeuteride afforded intermediate **15.** Cyclization of intermediate **15** under acidic condition provided target molecule **16,** 7-([1,2,4]triazolo[1,5-a]pyridin-6-yl)-4-(3,4-dichlorophenyl)-1,2,3,4-tetrahydroisoquinoline-3,3,4-d3.

### Example 6.1: Preparation of 7-([1.2.4]triazolo[1,5-a]pyridin-6-yl)-4-(3.4-dichlorophenyl)-1,2,3,4-tetrahydroisoquinoline-3.3.4-d3 16A and 16B:

### Preparation of 2-((3-([1,2,4]triazolo[1 ,5-a]pyridin-6-yl)benzyl)amino)-1-(3,4-dichlorophenyl)ethan-1,2,2-d3-1-ol 15:

To a solution of the above residue **14** (1.50 mmol) in CH₃OH (2.2 mL) was added aldehyde 3a (336 mg, 1.50 mmol) and DCM (1.5 mL). The mixture was stirred at rt for 2h before NaBH₄ (57 mg, 1.50 mmol) was added. The resulting mixture was stirred at rt overnight. The mixture was partitioned between DCM and 4N NH₄OH. The aqueous phase was extracted with DCM (2X). The combined extracts were dried over Na₂SO₄, filtered and concentrated in vacuo to give crude product (580 mg).

### Preparation of tert-butyl 7-([1,2,4]triazolo[1,5-a]pyridin-6-yl)-4-(3,4-dichlorophenyl)-3,4-dihydroi soquinoline-2(1H)-carboxylate-3,3,4-d3 16-Boc:

To a cold sulfuric acid (2.15 mL) at 0 °C was added dropwise a solution of 2-((3-([1,2,4]triazolo[1,5-a]pyridin-6-yl)benzyl)amino)-1-(3,4-dichlorophenyl)ethan-1,2,2-d3-1-ol **15** (580 mg) in DCM (1.9 mL). The mixture was stirred and allowed to warm to rt overnight. Ice-water was added to the reaction and the pH was brought to 9-10 by adding 20% NaOH. The mixture was extracted with DCM (3X) and the combined extracts were dried over Na₂SO₄, filtered and concentrated in vacuo to give the residue which contained 7-([1,2,4]triazolo[1,5-a]pyridin-6-yl)-4-(3,4-dichlorophenyl)-1,2,3,4-tetrahydroisoquinoline-3,3,4-d3 **16.** The residue containing **16** was dissolved in DCM (20 mL). To the solution was added di-t-butyl dicarbonate (360 mg, 1.65 mmol). The resulting mixture was stirred at rt overnight and then purified by column chromatography (H: EtOAc from 2:1 to 1:2) to give 7-([1,2,4]triazolo[1,5-a]pyridin-6-yl)-4-(3,4-dichlorophenyl)-3,4-dihydroisoquinoline-2(1H)-carboxylate-3,3,4-d3 **16-Boc** (168 mg, 22% three steps). ESI-MS (M+1) m/z: 498;

### Chiral SFC separation:

The racemic tert-butyl 7-([1,2,4]triazolo[1,5-a]pyridin-6-yl)-4-(3,4-dichlorophenyl)-3,4-dihydroisoquinoline-2(1H)-carboxylate-3,3,4-d3 **16-Boc** (101 mg) was dissolved in 5 mL of MeOH and 5 mL of dichloromethane and then resolved by chiral SFC (Regis Whelk-O1 (S, S) 21 × 250 mm; Mobile Phase: 45% 2-Propanol + 0.25 DEA in CO2; 254 nM) to yield enantiomer A **16A-Boc** (30 mg, RT = 2.35 min, 100% ee) and enantiomer B **16B-Boc** (33 mg, RT = 2.77 min, 100%ee).

### Preparation of enantiomer 7-([1,2,4]triazolo[1,5-a]pyridin-6-yl)-4-(3,4-dichlorophenyl)-1,2,3,4-tetrahydroisoquinoline-3,3,4-d3 HCl salt 16A:

The mixture of the above **16A-Boc** and 4N HCl in dioxane (1 mL) was stirred at rt for 1h. After removal of the volatiles, the residue was triturated with EtOAc (2 mL). The product enantiomer 7-([1,2,4]triazolo[1,5-a]pyridin-6-yl)-4-(3,4-dichlorophenyl)-1,2,3,4-tetrahydroisoquinoline-3,3,4-d3 HCl salt **16A** (26 mg):was obtained; ESI-MS (M+1) m/z: 398; ¹HNMR: identical to **16B**

### Preparation of enantiomer 7-([1,2,4]triazolo[1,5-a]pyridin-6-yl)-4-(3,4-dichlorophenyl)-1,2,3,4-tetrahydroisoquinoline-3,3,4-d3 HCl salt 16B:

The mixture of the above **16B-Boc** and 4N HCl in dioxane (1 mL) was stirred at rt for 1h. After removal of the volatiles, the residue was triturated with EtOAc (2 mL). The product enantiomer 7-([1,2,4]triazolo[1,5-a]pyridin-6-yl)-4-(3,4-dichlorophenyl)-1,2,3,4-tetrahydroisoquinoline-3,3,4-d3 HCl salt **16B** (27 mg):was obtained; ESI-MS (M+1) m/z: 398; ¹HNMR (400 MHz, DMSO) δ 9.8 (bs, 2H), 9.34 (s, 1H), 8.56 (s, 1H), 7.68 (m, 4H), 7.37-7.27 (m, 1H), 6.90 (d, J = 7.80 Hz, 1H), 4.58-4.50 (m, 1H), 4.41-3.99 (m, 1H).

### Example 7:

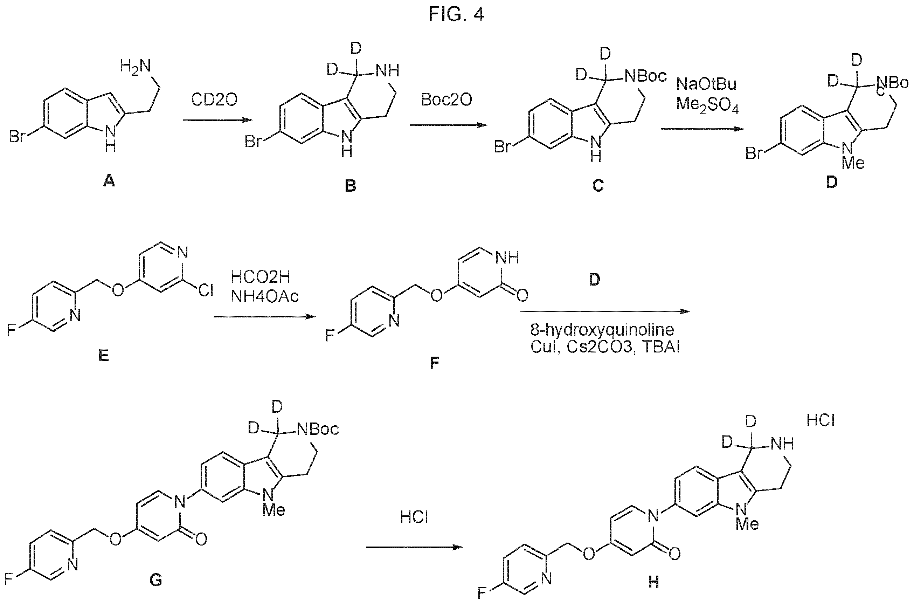

This example describes an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1-d2)pyridin-2(1H)-one, as schematically illustrated in FIG. 4.

Indole amine A is cyclized with deuterated formaldehyde to form intermediate B, which is Boc protected to give intermediate C. Compound D is obtained via methylation of indole C. Ullman coupling of bromide D and pyridine F affords compound G and the following deprotection under acidic conditions yields target compound H as the HCl salt.

### Example 8:

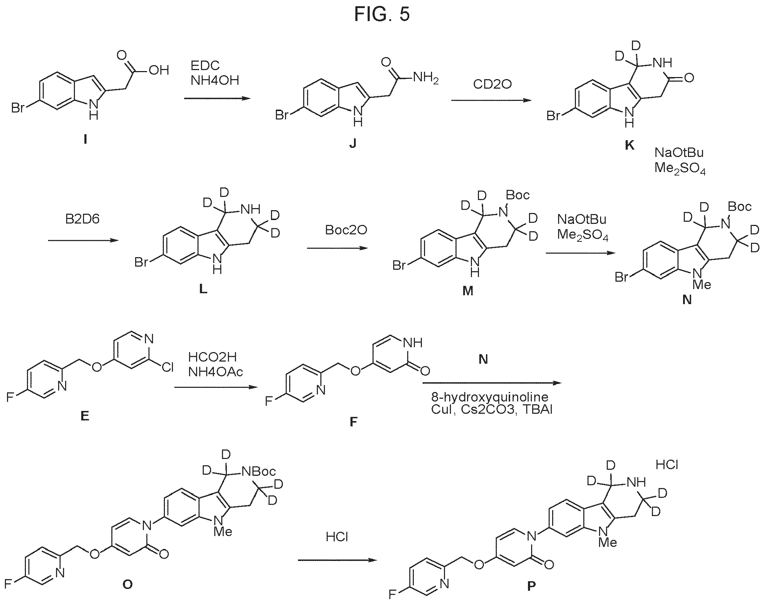

This example describes an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1,3,3-d4)pyridin-2(1H)-one, as schematically illustrated in FIG. 5.

Indole acid I is converted to indole amide J. Pictet-Spingler reaction of indole amide J with CD2O affords lactam K. The reduction of lactam K with deuterated borane gives amine L, which is Boc protected to afford indole M. Compound N is obtained via methylation of indole M. Pyridone F is obtained from compound E by treating with formic acid ammonium acetate. Ullman coupling of bromide N and F affords compound O. The deprotection of O under acidic conditions yields target compound P as the HCl salt.

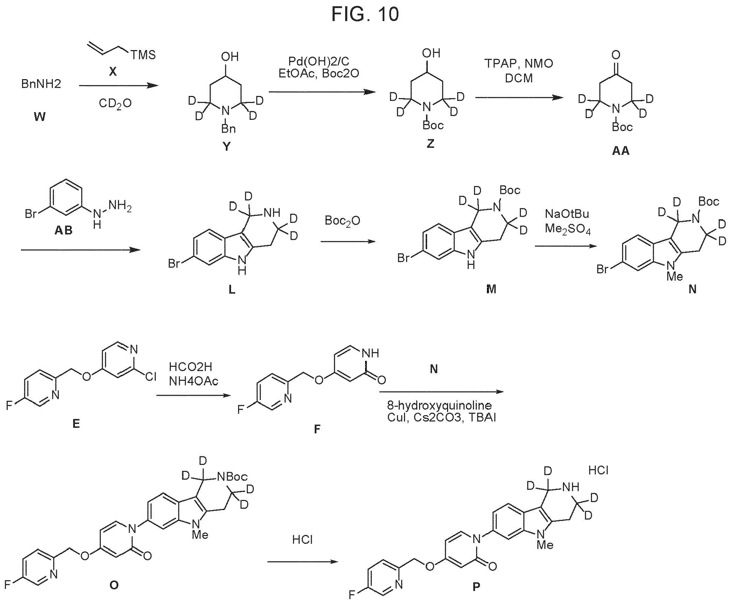

An alternative protocol for synthesizing the exemplary compound, 4-((5-fluoropyridin-2-yl)methoxy)-1-(5-methyl-2,3,4,5-tetrahydro-1H-pyrido[4,3-b]indol-7-yl-1,1,3,3-d4)pyridin-2(1H)-one, as schematically illustrated in FIG.10.

Benzyl amine W reacts with allyltrimethylsilane X in the presence of deuterated formaldehyde to form 1-benzylpiperidin-2,2,6,6-d4-4-ol Y. Debenzylation of Y followed by Boc protection affords alcohol Z. Following oxidation of alcohol Z with N-methyl morpholine N-oxide (NMO) and tetrapropylammonium perruthenate (TPAP), ketone AA is then reacted (3-bromophenyl)hydrazine AB provided deuterated indole L, which is Boc protected to afford indole M. Compound N is obtained via methylation of indole M. Compound E is treated with formic acid and ammonium acetate to afford compound F. Ullman coupling of bromide N and pyridine F afforded compound O and the following deprotection under acidic conditions yield target compound P.

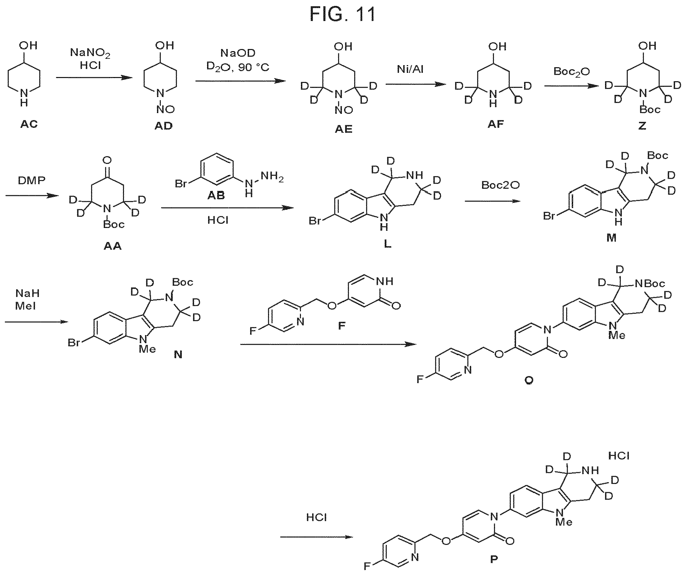

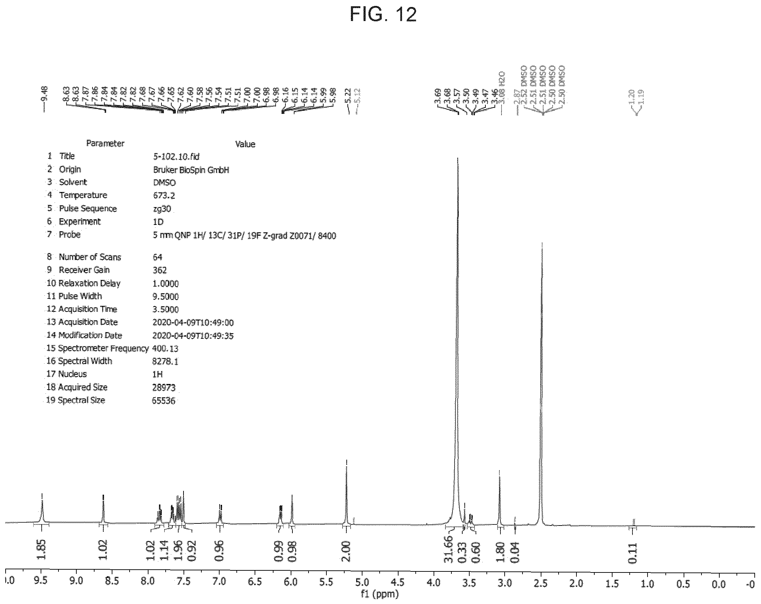

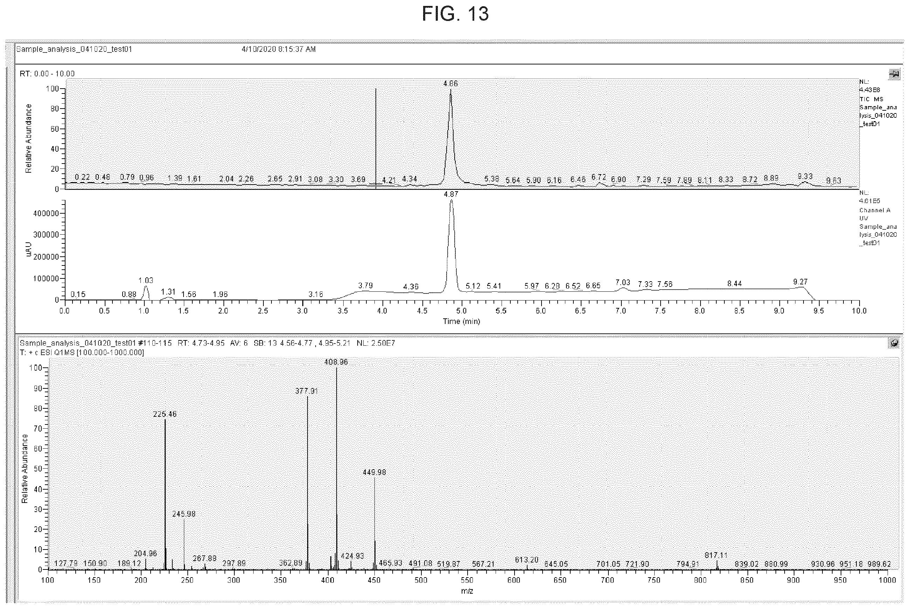

Compound P was synthesized following the route described in FIG. 11. Nitrosamine AD, which was obtained from hydroxylpiperidine AC, was treated with sodium deuterium oxide in deuterium oxide to form 2,2,6,6-d4 N-nitrosopiperidine AE. N-nitroso group was removed via Ni/Al reduction to yield piperidine AF, which was Boc protected to generate compound Z. Hydroxyl piperidine Z was oxidized form ketone AA. Fisher indole synthesis from ketone AA and 3-bromophenyl hydrazine gave indole L, which was Boc protected to form compound M. Compound N was obtained via methylation of indole M. Ullman coupling of bromide N and pyridone F afforded compound O and the following deprotection under acidic conditions yielded target compound P as the HCl salt of more than 50 mg. The proton NMR and LCMS spectra are included as FIG. 12 and FIG. 13 respectively.

### Example 9:

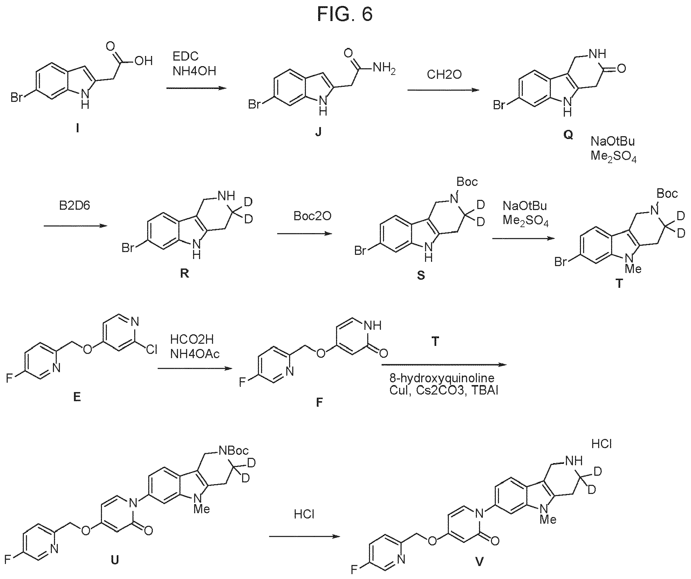

This example describes an exemplary protocol for synthesizing the exemplary compound, 4-((5-fluoropyridin-2-yl)methoxy)-1-(5-methyl-2,3,4,5-tetrahydro-1H-pyrido[4,3-b]indol-7-yl-3,3-d2)pyridin-2(1H)-one, as schematically illustrated in FIG. 6.

Indole acid I is converted to indole amide J. Pictet-Spingler reaction of indole amide J with formaldehyde affords lactam Q. The reduction of lactam Q with deuterated borane gives amine R, which is Boc protected to afford indole S. Compound T is obtained via methylation of indole S. Pyridone F is obtained from compound E by treating with formic acid ammonium acetate. Ullman coupling of bromide T and F affords compound U. The deprotection of U under acidic conditions yields target compound V as the HCl salt.

### Example 10:

This example describes the new algorithm as provided herein to calculate an initial dose and then safely titrate and maintain an effective dose. Central transporter occupancy is a powerful pharmacodynamic marker to assess the target engagement of a transporter inhibitor and guide the dosing in efficacy clinical trials. Occupancies measured by positron emission tomography (PET) has been reported for several reuptake inhibitors. SERT occupancy of 65-85% are reported at the efficacious doses of SSRIs and SSNRIs (Meyer JH et al, Am J Psychiatry. 2004 May;161(5):826-35;, Herold N et al, J Neural Transm (Vienna). 2006 May; 113(5):659-70; Klein N et al, Psychopharmacology (Berl). 2007 Apr;191(2):333-9.). Interestingly, SERT occupancy by clinical doses of sibutramine is of modest magnitude of 25-46%, implying that serotonin reuptake inhibition may be necessary but is not sufficient for sibutramine's efficacy in humans, supporting preclinical data that the hypophagic effect requires the co-inhibition of both SERT and NET (Talbot PS et al, Neuropsychopharmacology. 2010 Feb;35(3):741-51). DAT occupancy are reported for bupropion (14-26%), modafinil (51-57%) and methylphenidate (40-74%) at various approved doses (

VolkowND et al, Am J Psychiatry. 1998 Oct;155(10):1325-31; Kim W et al, Int J Neuropsychopharmacol. 2014 May;17(5):697-703, Kim W et al, Int J Neuropsychopharmacol. 2014 May;17(5):697-703.). DAT occupancy of 50%-70% for tesofensine was reported and suggested as the optimal weight loss doses (Appel L et al, Eur Neuropsychopharmacol. 2014 Feb;24(2):251-61). The radioligand for NET was just recently developed which allowed for the assessment of NET occupancy. At efficacious doses, the NET occupancies were reported ranging from approximately 30-50% for duloxetine (Moriguchi S et al, Int J Neuropsychopharmacol. 2017 Dec 1;20(12):957-962) and 8-61% for venlafaxine (Arakawa 2014). The reported transporter occupancy range for approved monoamine reuptake inhibitors referenced above are summarized in Table 9 for the purpose of easier viewing.

**Table 1: Reported Transporter Occupancy Range for Approved Monoamine Reuptake Inhibitors at Efficacious Doses**

| **Range** | **SERT** | **DAT** | **NET** |
|---|---|---|---|
| Lowest Occupancy | 25% (Sibutramine) | 14% (Bupropion) | 8% (Venlafaxine) |
| | 65% (SSRIs) | | |
| Highest Occupancy | 85% (Sertraline) | 74% (Methylphenidate) | 61% (Venlafaxine) |

The PET study was a part of a strategy in previous clinical development of the compound of formula I to evaluate in healthy male volunteers with respect to safety, tolerability and pharmacokinetics as well as DAT and SERT occupancy.

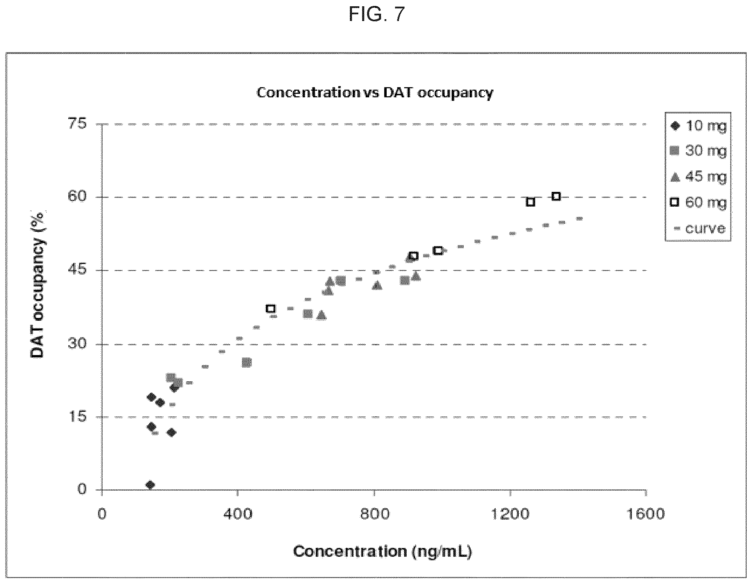

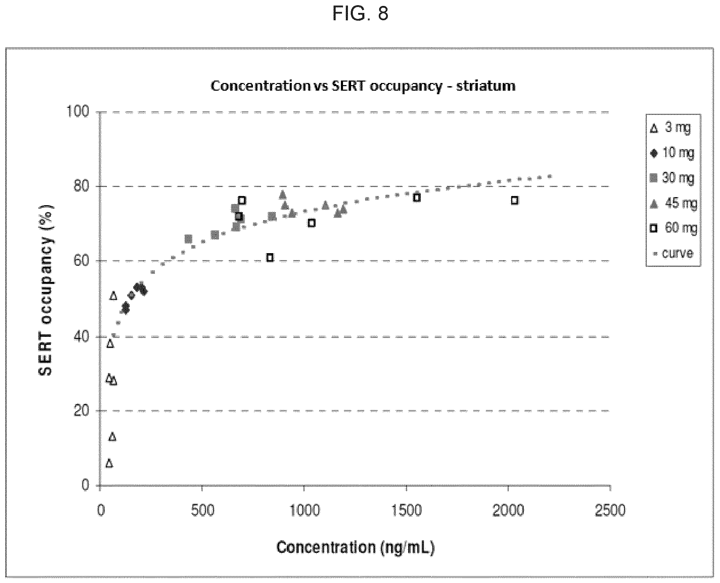

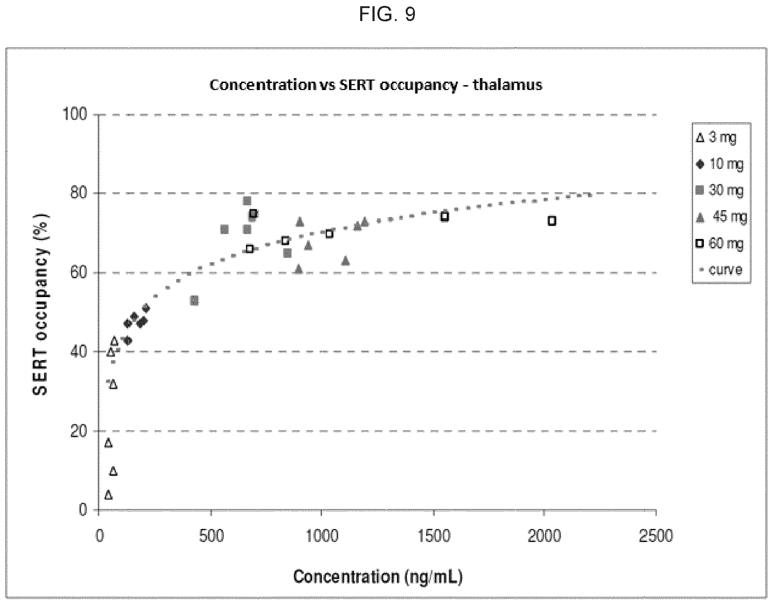

In the study, SERT occupancy of 65-75% were reached for multiple doses of 30-60 mg the compound of formula I at steady state. Pronounced and dose-dependent DAT occupancies were achieved with up to about 60% following multiple doses of 60 mg the compound of formula I. A near linear relationship between plasma the compound of formula I concentration and DAT occupancies, using of the individual values, is presented in FIG. 7. The relationship between plasma the compound of formula I concentration and SERT occupancies, using the individual values, is presented in FIG. 8 and FIG. 9. The numeric values for the individual SERT and DST occupancy and the corresponding plasma concentrations are tabulated in Example 12.

Although notable variabilities of plasma concentrations of a compound of Formula I versus doses were observed in a few cohorts as seen in FIG. 5 and FIG. 6, generally, the magnitude of the plasma concentrations corresponded well with the magnitude of the estimated occupancies. This PK/PD relationship established will be used to guide the dosing in the future studies.

When the PET study was initiated there were no adequate methods available to estimate NET occupancy in the human brain. Based on ex vivo occupancy data in the brain of mice, however, it is expected that the NET occupancies are almost in line with the DAT occupancies after administration of a compound of Formula I.

Taken together the results of this PET study and the preclinical results, it is concluded that Formula I is a potent, competitive and selective triple reuptake inhibitor of DAT, SERT and NET in human. Further, Formula I has a unique profile of triple reuptake inhibition that will allow for dosing within the therapeutic ranges of approved single and dual SERT, DAT and NET inhibitors in efficacious doses. Particularly, Formula I can achieve near efficacious level of SERT occupancy seen in SSRIs across a safe dosing range while allowing for dialing in DAT occupancy up to approximately 60% in the PET study conducted in healthy males. This unique TRI profile of Formula I allows for the flexibility in gradual titration for better tolerability and efficacy tailored to individual patient.

Given the near linear relationship between plasma concentrations and DAT occupancies, the anticipated large variation in dose and plasma drug concentration, using a concentration-controlled dosing approach, utilizing the PK/PD relationship established in the PET study of Formula I, can provide precision dosing to individual patients with optimal outcome.

Example 1, above, describes exemplary methods and compositions for the treatment of Prader-Willi syndrome and related disorders, and Example 2, above, describes methods and compositions for the treatment of hypothalamic injury-induced obesity (HO) and related disorders, with Formula I as described herein and a deuterated analogue thereof, for example, as described herein.

A patient is given a subtherapeutic dose between 1 mg to 20 mg per day for one day, one week, two weeks, three weeks or four weeks. Blood sample is taken periodically to determine drug concentration at steady state and to determine the nearest target dose needed to achieve DAT occupancy of between 15% to 75% with or without the aid of a pharmocometrics modelling method that also utilizes the PK/PD data; optionally the near target dose is determined by achieving a plasma drug concentration value between 100 ng/ml to 4000 ng/ml at steady state; optionally the near target dose is determined by achieving a plasma drug concentration value between 200 ng/ml to 2000 ng/ml at steady state; optionally the near target dose is determined by achieving a plasma drug concentration value between 400 ng/ml to 2000 ng/ml at steady state. Every 3 months or 6 months, or when a patient has significant changes in medications, body weight and other physical changes can impact the drug absorption and metabolism, blood drug level will be retested to adjust the dosing if necessary; optionally, patients will start a low dose of between 5 mg to 20 mg per day and gradually increase dose by 5 mg or 10 mg or 20 mg interval until safely reach efficacy for hyperphagia and/or neuropsychiatric and behavioral symptoms. A clinician can accelerate titration, slow down the titrate if necessary, and re-accelerate titration later based on the tolerability of the patient.

### Example 11:

In alternative embodiments, provided are methods comprising administering a drug in an amount sufficient to achieve a therapeutic level range of plasma concentration (optionally human plasma concentration) at a steady state, and this example describes an exemplary analytical method to determine the concentration of Formula I in human plasma using an LC-MS-MS method known to the people skilled in the art, and an exemplary bioanalysis method to determine the concentration of formula I in human EDTA plasma.

A LC-MS/MS method was developed and validated for the quantitation of Formula I in 0.250 mL of human EDTA plasma in a total of 7 runs, out of which 1 run was rejected. The method utilized stable labeled Formula I as an internal standard. After the addition of the internal standard (equivalent to 285.4 ng/mL in human EDTA plasma) and 0.250 mL of each quality control (QC) sample and calibration standard, 0.525 mL of methyl t-butyl ether (MTBE) was added to the 96-well sample tubes. The organic layer was removed and evaporated to dryness using a liquid-liquid extraction (LLE). The residue was reconstituted and injected into the LC-MS/MS system. Chromatographic separation was achieved through gradient elution on an Acquity UPLC, BEH Shield RP18^{™}, 50 × 2.1 mm, 1.7 µm particle size column. The mobile phase contained water, acetonitrile, ammonium formate, and formic acid. Detection was accomplished using a Sciex API 4000 tandem mass spectrometer in positive ion electrospray SRM mode. The standard curves, which ranged from 1.00 to 1000 ng/mL for Formula I, were fitted to a 1/x2 weighted linear regression model. The intra-assay precisions, based on four levels of analytical QCs (low, mid, GM, and high), were within 4.2% CV and inter-assay precisions were within 7.2% CV for the analyte. The assay accuracy, expressed as %DEV, was within 6.4% of the nominal concentration values for the analyte. At the lower limit of quantitation (LLOQ) at 1.00 ng/mL for Formula I, the deviations of the predicted concentrations from the nominal values were within 14.1% for all LLOQ samples from six different matrix lots. The LLOQ response ratio, when compared to the QC0 response ratio, was ≥ 5 in all matrix lots. The analyte was stable in human EDTA plasma for at least 24 h at room temperature, for at least 14 days at -20°C, and following at least 3 freeze- thaw cycles. The processed samples were stable for 72 hours (h) at room temperature.

Based on the results of this validation, the acceptance criteria for sample analysis are as follows:
1) the predicted concentrations of at least three-fourths of all calibration standards shall be within ±15.0% of their nominal concentrations with the exception of the LLOQ, which will be within ±20.0%. Standards not meeting these criteria will be excluded from the regression. At least three-fourths of all of the standards will meet the acceptance criteria and be included in the final regression;
2) at least one replicate of the lowest concentration in the standard curve will meet the above criteria and be included within the final regression for that level to qualify as the LLOQ. If this criterion is not met, the next standard level will be subjected to the same test and the LLOQ raised accordingly;
3) the predicted concentrations of at least two thirds of all analytical quality control samples will be within ±15.0% of their nominal concentrations, with at least 50% of the QC samples meeting acceptance criteria at each level;
4) If a single dilution of dilution QCs is used, the predicted concentrations of at least 50% of the dilution QC samples at a particular dilution will be within ±15.0% of their nominal concentrations for diluted sample results at the same or lesser dilution to be accepted.

If different dilutions of the dilution QCs are used, the predicted concentrations of at least 50% of the dilution QC samples at each dilution will be within ±15.0% of their nominal concentrations for diluted sample results at the same dilution to be accepted. The results of the dilution QC samples will not be used to determine run acceptability.

The HPLC parameters used are shown in the following table:

| Gradient using mobile phases A and D | Isocratic, 80%A/20%D, for 1.0 min |
|---|---|
| | Then linear gradient to increase %D from 20% to 55% in 0.50 min |
| | Then linear gradient to increase %D from 55% to 95% in 0.1 min |
| | Hold %D at 95% for 0.3 min |
| | Decrease %D from 95% to 20% in 0.1 min |
| | Stop run at 2.0 min |
| Flow rate | 0.6 mL/min |
| Column | Acquity UPLC, BEH Shield RP18 analytical column, 50 × 2.1 mm, 1.7 µm particle size |
| Column temperature | 60°C |
| Autosampler wash solution 1 | 1% EDTA in 50/50 water and methanol |
| Autosampler wash solution 2 | Water, acetone, 2-propanol, and methanol (1/1/1/1; v/v) |
| Injection volume | 10 □L using a 5 µL fixed loop |
| Autosampler temperature | Room temperature |
| Formula I retention time | 0.9 min |
| IS- Formula I retention time | 0.9 min |
| Total run time | 2.0 min |

Mobile phase A - 10 mM ammonium formate and 0.1% formic acid in water and acetonitrile (90/10). Ammonium formate, 0.63 g, was dissolved in 900 mL of water and 100 mL of acetonitrile with 1.0 mL of formic acid added.

Mobile phase D - 10 mM ammonium formate and 0.1% formic acid in water and acetonitrile (10/90). Ammonium formate, 0.63 gram (g), was dissolved in 100 mL of water and 900 mL of acetonitrile, with 1.0 mL of formic acid added.

The mass spectrometric parameters used are shown in the following table:

| **Parameter for Sciex API 4000** | **Value** | |
|---|---|---|
| Ion polarity | Positive | |
| Source type | Electrospray | |
| Curtain gas (CUR) | nitrogen, UHP | 10 |
| Collision gas (CAD) | nitrogen, UHP | 5.0 |
| Declustering Potential (DP) | 85 V, Formula I | |
| | 76 V, IS- Formula I | |
| Collision Energy (CE) | 39 V, Formula I | |
| | 34 V, IS- Formula I | |
| Collision Cell Exit Potential (CXP) | 19.5 V, Formula I | |
| | 27.3 V, IS- Formula I | |
| TurboIonSpray Voltage (IS) | 4500 V | |
| Turbo probe temperature setting (TEM) | 500 degrees C | |
| Entrance Potential (EP) | 10.6 V, 10.3 V, IS- Formula I | |
| Ion Source Gas 1 (GS1) | 50 | |
| Ion Source Gas 2 (GS2) | 50 | |
| Dwell time | 200 msec | |

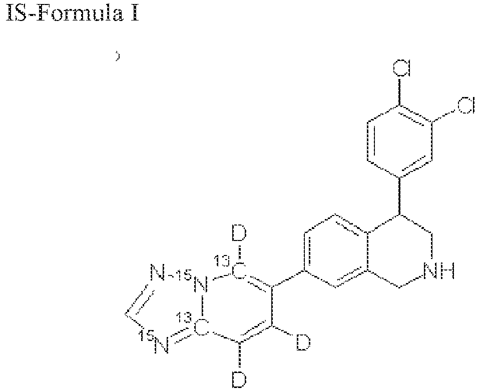

### Example 12:

Striatal receptor occupancy at dopamine transporters (DAT) and serotonin transporters (SERT) with plasma concentrations of Formula I at the midpoint time of the post dosing PET (positron emission tomography) scans (T=4 and 24) after once-a-day multiple doses of 3-60 mg and 10-60 mg for SERT and DAT healthy male cohorts, respectively (Tables below, RO stands for receptor occupancy). Post dosing PET scans were conducted at day 10 (4 h post last dose) and day 14 (24 h post last dose) assuming steady state conditions, using the specific DAT and SERT tracers ¹¹C-PE2I and ¹¹C-MADAM, respectively. For 45 mg and 60 mg cohorts, there was titration by administration of 30 mg for 3 days and then from day 4 the specified dose. Representative reports on using these two tracers are Learned-Coughlin SM et al, Biol Psychiatry.2003, 54(8):800-5; Jucaite A et al, Eur J Nucl Med Mol Imaging. 2006, 33(6):657-68; and Lundberg J et al, Int J Neuropsychopharmacol. 2007, 10(6):777-85.

| Subject ID | Dose (mg) | Day | Time (h) | Formula I plasma concentration (ng/ml) | SERT RO% |
|---|---|---|---|---|---|
| 1 | 3 | 10 | 4 | 68 | 51 |
| 2 | 3 | 10 | 4 | 64 | 28 |
| 5 | 3 | 10 | 4 | 63 | 13 |
| 1 | 3 | 14 | 24 | 52 | 38 |
| 2 | 3 | 14 | 24 | 45 | 29 |
| 5 | 3 | 14 | 24 | 42 | 6 |
| 33 | 10 | 10 | 4 | 184 | 53 |
| 35 | 10 | 10 | 4 | 201 | 53 |
| 37 | 10 | 10 | 4 | 212 | 52 |
| 33 | 10 | 14 | 24 | 127 | 47 |
| 35 | 10 | 14 | 24 | 129 | 48 |
| 37 | 10 | 14 | 24 | 156 | 51 |
| 47 | 30 | 10 | 4 | 691 | 71 |
| 48 | 30 | 10 | 4 | 847 | 72 |
| 57 | 30 | 10 | 4 | 667 | 74 |
| 47 | 30 | 14 | 24 | 668 | 69 |
| 48 | 30 | 14 | 24 | 434 | 66 |
| 57 | 30 | 14 | 24 | 567 | 67 |
| 63 | 45 | 10 | 4 | 1163 | 73 |
| 65 | 45 | 10 | 4 | 1191 | 74 |
| 69 | 45 | 10 | 4 | 1105 | 75 |
| 63 | 45 | 14 | 24 | 940 | 73 |
| 65 | 45 | 14 | 24 | 904 | 75 |
| 69 | 45 | 14 | 24 | 895 | 78 |
| 75 | 60 | 10 | 4 | 1039 | 70 |
| 84 | 60 | 10 | 4 | 837 | 61 |
| 86 | 60 | 10 | 4 | 2035 | 76 |
| 75 | 60 | 14 | 24 | 698 | 76 |
| 84 | 60 | 14 | 24 | 679 | 72 |
| 86 | 60 | 14 | 24 | 1557 | 77 |
| 36 | 10 | 10 | 4 | 204 | 12 |
| 38 | 10 | 10 | 4 | 214 | 21 |
| 40 | 10 | 10 | 4 | 173 | 18 |
| 36 | 10 | 14 | 24 | 141 | 1 |
| 38 | 10 | 14 | 24 | 149 | 19 |
| 40 | 10 | 14 | 24 | 147 | 13 |
| 45 | 30 | 10 | 4 | 607 | 36 |
| 49 | 30 | 10 | 4 | 225 | 22 |
| 53 | 30 | 10 | 4 | 896 | 43 |
| 45 | 30 | 14 | 24 | 430 | 26 |
| 49 | 30 | 14 | 24 | 207 | 23 |
| 53 | 30 | 14 | 24 | 705 | 43 |
| 62 | 45 | 10 | 4 | 809 | 42 |
| 64 | 45 | 10 | 4 | 924 | 44 |
| 66 | 45 | 10 | 4 | 905 | 48 |
| 62 | 45 | 14 | 24 | 647 | 36 |
| 64 | 45 | 14 | 24 | 668 | 41 |
| 66 | 45 | 14 | 24 | 673 | 43 |
| 77 | 60 | 11 | 4 | 1340 | 60 |
| 80 | 60 | 10 | 4 | 1263 | 59 |
| 77 | 60 | 14 | 24 | 920 | 48 |
| 80 | 60 | 14 | 24 | 991 | 49 |

### Example 13

This example describes methods for measuring the inhibition potency against dopamine transporter (DAT), norepinephrine transporter (NET) and serotonin transporter (SERT or 5-HTT) and the inhibition potency of compounds Formula I, 8A, 8B, 12A, 12B, 16A, and 16B using these methods (Eurofin Discovery Taiwan Category #s 220320, 204410, 274030); and for measuring inhibition potency against Transporter, Dopamine (DAT) (Giros et al, Trends Pharmacol Sci. 14(2): 43-49, 1993; Gu et al, J Biol Chem. 269(10):7124-7130, 1994):
Protocol summary:
Source: Human recombinant CHO-S cells;
Vehicle: 1.0% DMSO;
Incubation Time/Temp: 3 hours @ 4°C;
Incubation Buffer: 50 mM Tris-HCl, pH 7.4, 100 mM NaCl, 1 µM Leupeptin, 10 µM PMSF;
Kd: 0.58 nM (Historical Values);
Ligand: 0.15 nM [¹²⁵I] RTI-55;
Non-Specific Ligand: 10.0 µM Nomifensine;
Specific Binding: 90% (Historical Values);
Quantitation Method: Radioligand Binding;
Significance Criteria: ≥50% of max stimulation or inhibition;
Bmax: 0.047 pmole/mg Protein

### Measuring inhibition potency against Transporter, Norepinephrine (NET) (Galli et al, J Exp Biol. 198(Pt 10):2197-2212, 1995)

Protocol summary:
Source: Human recombinant MDCK cells;
Vehicle: 1.0% DMSO;
Incubation Time/Temp: 3 hours @ 4°C;
Incubation Buffer: 50 mM Tris-HCl, pH 7.4, 100 mM NaCl, 1 µM Leupeptin, 10 µM PMSF;
Kd: 0.024 µM (Historical Values);
Ligand: 0.2 nM [¹²⁵I] RTI-55;
Non-Specific Ligand: 10.0 µM Desipramine;
Specific Binding: 75% (Historical Values);
Quantitation Method: Radioligand Binding;
Significance Criteria: ≥50% of max stimulation or inhibition;
Bmax: 2.50 pmole/mg Protein

### Measuring inhibition potency against Transporter, Serotonin (5-Hydroxytryptamine) (SERT) (Shearman et al, Am J Physiol. 275(6 Pt 1): C1621-1629, 1998; Wolf et al, J Biol Chem. 267(29): 20820-20825, 1992)

Protocol summary:
Source: Human recombinant HEK-293 cells;
Vehicle: 1.0% DMSO;
Incubation Time/Temp: 60 minutes @ 25°C;
Incubation Buffer: 50 mM Tris-HCl, pH 7.4, 120 mM NaCl, 5 mM KCl;
Kd: 0.078 nM (Historical Values);
Ligand: 0.4 nM [3H] Paroxetine;
Non-Specific Ligand: 10.0 µM Imipramine;
Specific Binding: 95% (Historical Values);
Quantitation Method: Radioligand Binding;
Significance Criteria: greater than or equal to (≥) 50% of max stimulation or inhibition;
Bmax: 4.40 pmole/mg Protein (Historical Values)

The potency data are expressed as %inhibition at 10 nM and 100 nM of the compound concentration in the table below.

| Compound # | SERT, %inhibition | | DAT, %inhibition | | NET, %inhibition | |
|---|---|---|---|---|---|---|
| | 100 nM | 10 nM | 100 nM | 10 nM | 100 nM | 10 nM |
| formula I | 91 | 59 | 92 | 47 | 84 | 35 |
| 8A | 91 | 51 | 93 | 44 | 86 | 29 |
| 8B | 51 | 34 | 75 | 4 | 71 | 37 |
| 12A | 94 | 54 | 92 | 53 | 79 | 34 |
| 12B | 54 | 21 | 74 | 0 | 74 | 44 |
| 16A | 96 | 59 | 91 | 47 | 83 | 38 |
| 16B | 46 | 2 | 76 | -2 | 72 | 49 |

### Example 14

This example describe methods for measuring the inhibition potency against MCHR1 and the inhibition potency of compounds Formula II using this method (Eurofin Discovery Taiwan Category # 251010):
Protocol summary:
Source: Human recombinant CHO-K1 cells;
Vehicle: 1.00% DMSO;
Incubation Time/Temp: 2 hours @ 25°C;
Incubation Buffer: 25 mM HEPES, pH 7.4, 10 mM MgCl₂, 1 mM EDTA, 0.2% BSA;
Kd: 0.10 nM (Historical Values);
Ligand: 0.050 nM [¹²⁵I] Tyr-S36057;
Non-Specific Ligand: 1.0 µM MCH (human, mouse, rat);
Specific Binding: 85% (Historical Values);
Quantitation Method: Radioligand Binding;
Significance Criteria: ≥50% of max stimulation or inhibition;
Bmax: 26.0 pmole/mg Protein (Historical Values)

The potency data are expressed as IC50 and Ki in table below

| Compound # | IC50 | Ki |
|---|---|---|
| Formula II | 10.6 nM | 7.04 nM |
| P | 10.5 nM | 6.99 nM |

### Example 16:

Individually dosing compound of Formula I to patients of PWS, or Hypothalamic injury-induced obesity, using PK-guided dosing

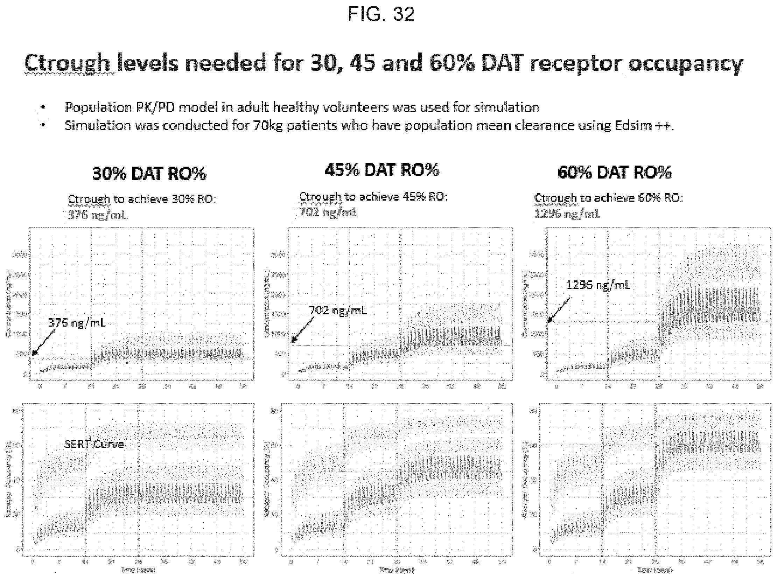

Patients are given an initial low dose of 10 mg/day of compound of Formula I for 2 weeks followed by an optionally additional titration step, which will increase the dose individually with the goal to achieve the target trough plasma concentration of approximately 375 ng/mL (or corresponding to approximately 30% DAT occupancy in healthy human) for 2 weeks. Then, patients will be titrated up to a dose amount required to achieve the target trough plasma concentration of approximately 700 ng/mL for the Low dose (corresponding to approximately 45% DAT occupancy in healthy human), or approximately 1295 ng/mL (corresponding to approximately 60% DAT occupancy in healthy human). Figure 32 illustrate PK/PD model-informed simulations of the plasma levels required to achieve the target receptor occupancy. The target plasma concentrations are calculated using PK-PD model described for formula I in the embodiments.

The PK-guided dosing is based on drug plasma concentration, not an actual dose in mg given to an individual person. In other words, depending on their age, size, ability to metabolize the drug and other factors, two patients may receive quite different doses, even though they may be randomized into the same "dose" group as described below.

The drug is formulated as tablets or capsules are available from 5 to 240 mg with appropriate increment, which should allow all participants to be as close as possible to their target plasma levels. All patients will receive one or two tablet/capsules daily, to be taken in the morning.

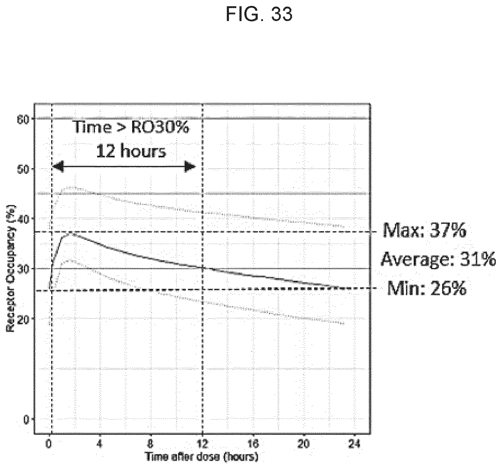

The required Ctrough for each target DAT occupancy level has been selected so that they are at or slightly above the target DAT receptor occupancy for 12 hours during the daytime, as shown on the simulation below, corresponding with the fluctuation of the plasma levels throughout each day, following morning drug administration. The Pk-guided dosing provides the best opportunity for each patient to achieve the desired efficacy without unnecessary exposure of the drug. Figure 25 shows the plasma levels over 24 hours with the dose optimized to provide the desired 30% DAT receptor occupancy coverage during the daytime 12-hour period. See Figure 33 for illustration.

### Example 17:

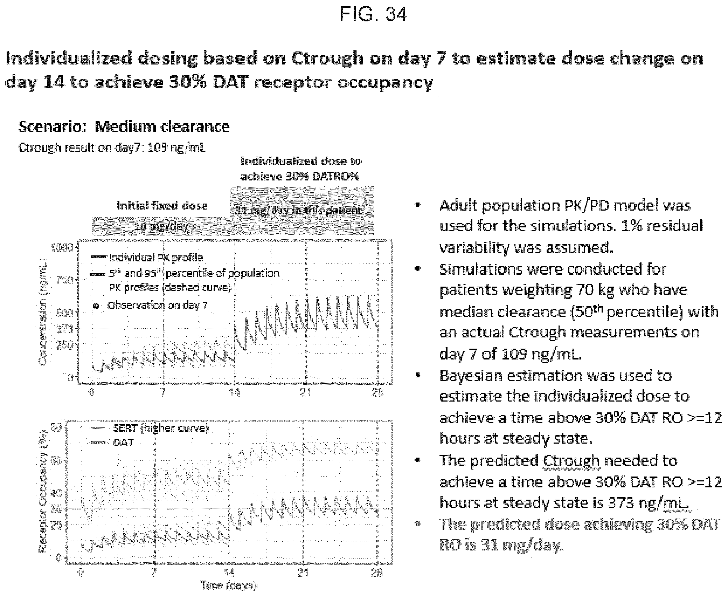

This example describes simulation-individualized dosing based on Ctrough on day 7 to estimate dose change on day 14 to achieve 30% DAT receptor occupancy. See illustration in Figure 34.

### Example 18:

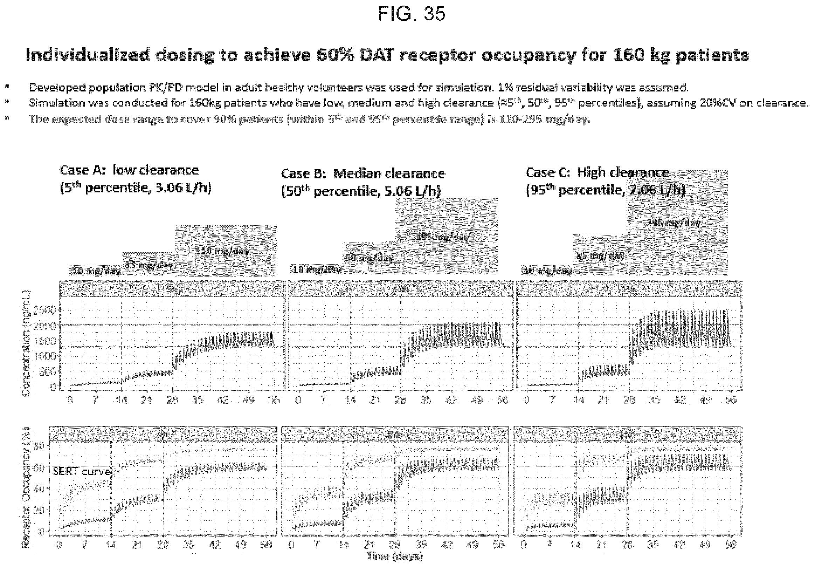

This example describes simulation of individualized dosing to achieve 60% DAT receptor occupancy for 160 kg patients. See illustration in Figure 35.

### Example 19:

This example describes individually dosing compound of Formula I to patients of PWS, or Hypothalamic injury-induced obesity, using an initial PK-assessment followed by PK-guided dosing.

Patients will undergo an initial PK-assessment to determine the subject's individual PK parameters to tailor the initial starting dose for Formula I. At the initial PK-assessment, subjects will receive a single low dose such as 10 mg dose of compound of Formula I. PK draws will occur Pre-dose and post dose hour, 2 hour, 4 hour, 24 hour, 3 days and 7 days.

Then, patients will be individually dosed such they can achieve the target trough plasma concentration of ∼375 ng/mL (or corresponding to -30% DAT occupancy in healthy human), -700 ng/mL (corresponding to -45% DAT occupancy in healthy human), or approximately 1300 ng/mL (corresponding to approximately 60% DAT occupancy in healthy human) using PK simulations methods described in the embodiments. An optional repeat PK at steady state will be performed after 3 to 12 months of treatment if needed.

The PK-guided dosing is based on drug plasma concentration, not an actual dose in mg given to an individual person. In other words, depending on their age, size, ability to metabolize the drug and other factors, two patients may receive quite different doses, even though they may be randomized into the same "dose" group as described below.

The drug is formulated as tablets or capsules are available from 5 to 240 mg with appropriate increment, which should allow all participants to be as close as possible to their target plasma levels. All patients will receive one or two tablet/capsules daily, to be taken in the morning.

The required Ctrough for each target DAT occupancy level has been selected so that they are at or slightly above the target DAT receptor occupancy for 12 hours during the daytime, as shown on the simulation below, corresponding with the fluctuation of the plasma levels throughout each day, following morning drug administration. The PK-guided dosing provides the best opportunity for each patient to achieve the desired efficacy without unnecessary exposure of the drug.

### Example 20

Tabulated, day-14 PK data (Cmax, Ctrough and AUC) for healthy humans in a 14-day, once-a-day oral dosing, multiple ascending dose study of compound of Formula I.

| **Subject ID** | **Sex** | **Dally Dose (mg)** | **Time (h) of blood draw** | **Formula I plasma concentration (ng/ml)** | **Cmax** | **AUC** |
|---|---|---|---|---|---|---|
| 1 | male | 3 | 0 | 51 | | |
| 1 | male | 3 | 2.017 | 77 | Yes | 73 |
| 1 | male | 3 | 23.05 | 48 | | 525 |
| 2 | male | 3 | 0 | 44 | | |
| 2 | male | 3 | 3 | 74 | Yes | 73 |
| 2 | male | 3 | 22.967 | 50 | | 439 |
| 5 | male | 3 | 0 | 42 | | |
| 5 | male | 3 | 2 | 66 | Yes | 63 |
| 5 | male | 3 | 22.933 | 42 | | 374 |
| 7 | male | 3 | 0 | 36 | | |
| 7 | male | 3 | 3 | 64 | Yes | 60 |
| 7 | male | 3 | 24 | 38 | | 369 |
| 9 | male | 3 | 0 | 40 | | |
| 9 | male | 3 | 3 | 67 | Yes | 66 |
| 9 | male | 3 | 23.967 | 42 | | 414 |
| 14 | male | 3 | 0 | 31 | | |
| 14 | male | 3 | 1 | 63 | Yes | 29 |
| 14 | male | 3 | 23.95 | 34 | | 338 |
| 33 | male | 10 | 0 | 125 | | |
| 33 | male | 10 | 1.117 | 218 | Yes | 120 |
| 33 | male | 10 | 23 | 130 | | 1263 |
| 35 | male | 10 | 0 | 127 | | |
| 35 | male | 10 | 2 | 249 | Yes | 231 |
| 35 | male | 10 | 23 | 140 | | 1447 |
| 36 | male | 10 | 0 | 134 | | |
| 36 | male | 10 | 3 | 243 | Yes | 240 |
| 36 | male | 10 | 22.967 | 138 | | 1302 |
| 37 | male | 10 | 0 | 183 | | |
| 37 | male | 10 | 2 | 285 | Yes | 280 |
| 37 | male | 10 | 23.017 | 178 | | 1925 |
| 38 | male | 10 | 0 | 155 | | |
| 38 | male | 10 | 3 | 259 | Yes | 258 |
| 38 | male | 10 | 24 | 160 | | 1884 |
| 40 | male | 10 | 0 | 147 | | |
| 40 | male | 10 | 1 | 242 | Yes | 109 |
| 40 | male | 10 | 24.033 | 162 | | 1985 |
| 45 | male | 30 | 0 | 461 | | |
| 45 | male | 30 | 3 | 805 | Yes | 764 |
| 45 | male | 30 | 23 | 473 | | 4727 |
| 47 | male | 30 | 0 | 595 | | |
| 47 | male | 30 | 2 | 979 | Yes | 938 |
| 47 | male | 30 | 23 | 617 | | 5862 |
| 48 | male | 30 | 0 | 460 | | |
| 48 | male | 30 | 2 | 768 | Yes | 723 |
| 48 | male | 30 | 23.017 | 472 | | 4181 |
| 49 | male | 30 | 0 | 256 | | |
| 49 | male | 30 | 1 | 353 | Yes | 166 |
| 49 | male | 30 | 23.017 | 208 | | 2231 |
| 53 | male | 30 | 0 | 809 | | |
| 53 | male | 30 | 1 | 1549 | Yes | 655 |
| 53 | male | 30 | 24 | 791 | | 9425 |
| 57 | male | 30 | 0 | 556 | | |
| 57 | male | 30 | 2 | 853 | Yes | 849 |
| 57 | male | 30 | 23.033 | 602 | | 6523 |
| 62 | male | 45 | 0 | 635 | | |
| 62 | male | 45 | 1 | 1152 | Yes | 523 |
| 62 | male | 45 | 23 | 672 | | 6276 |
| 63 | male | 45 | 0 | 866 | | |
| 63 | male | 45 | 1 | 1570 | Yes | 682 |
| 63 | male | 45 | 23 | 890 | | 8418 |
| 64 | male | 45 | 0 | 695 | | |
| 64 | male | 45 | 1 | 1394 | Yes | 505 |
| 64 | male | 45 | 23 | 693 | | 6879 |
| 65 | male | 45 | 0 | 862 | | |
| 65 | male | 45 | 2 | 1579 | Yes | 1472 |
| 65 | male | 45 | 23 | 941 | | 8712 |
| 66 | male | 45 | 0 | 791 | | |
| 66 | male | 45 | 1 | 1348 | Yes | 571 |
| 66 | male | 45 | 23 | 779 | | 8687 |
| 69 | male | 45 | 0 | 957 | | |
| 69 | male | 45 | 1 | 1808 | Yes | 801 |
| 69 | male | 45 | 23 | 924 | | 9833 |
| 87 | female | 45 | 0 | 997 | | |
| 87 | female | 45 | 3 | 1679 | Yes | 1673 |
| 87 | female | 45 | 23.983 | 901 | | 10113 |
| 89 | female | 45 | 0 | 832 | | |
| 89 | female | 45 | 1 | 1465 | Yes | 699 |
| 89 | female | 45 | 24 | 929 | | 9936 |
| 90 | female | 45 | 0 | 732 | | |
| 90 | female | 45 | 1 | 1198 | Yes | 513 |
| 90 | female | 45 | 24 | 649 | | 6959 |
| 91 | female | 45 | 0 | 714 | | |
| 91 | female | 45 | 1 | 1313 | Yes | 541 |
| 91 | female | 45 | 24.05 | 749 | | 8066 |
| 95 | female | 45 | 0 | 973 | | |
| 95 | female | 45 | 2 | 1713 | Yes | 1625 |
| 95 | female | 45 | 24.017 | 1033 | | 11276 |
| 96 | female | 45 | 0 | 1274 | | |
| 96 | female | 45 | 1 | 2112 | Yes | 906 |
| 96 | female | 45 | 24.017 | 1249 | | 12977 |
| 75 | male | 60 | 0 | 761 | | |
| 75 | male | 60 | 1 | 1596 | Yes | 736 |
| 75 | male | 60 | 23 | 695 | | 7304 |
| 77 | male | 60 | 0 | 960 | | |
| 77 | male | 60 | 1 | 1764 | Yes | 849 |
| 77 | male | 60 | 23 | 995 | | 11087 |
| 80 | male | 60 | 0 | 1070 | | |
| 80 | male | 60 | 1 | 1839 | Yes | 807 |
| 80 | male | 60 | 23 | 990 | | 10073 |
| 84 | male | 60 | 0 | 639 | | |
| 84 | male | 60 | 1 | 1211 | Yes | 509 |
| 84 | male | 60 | 23 | 619 | | 7876 |
| 86 | male | 60 | 0 | 1578 | | |
| 86 | male | 60 | 3 | 2710 | Yes | 2593 |
| 86 | male | 60 | 23 | 1667 | | 18777 |

A number of embodiments of the invention have been described. Nevertheless, it can be understood that various modifications may be made without departing from the spirit and scope of the invention. Accordingly, other embodiments are within the scope of the following claims.

Further embodiments are set out in the following numbered items:
1. A therapeutic combination, a pharmaceutical dosage form, a drug delivery device or a product of manufacture, comprising one or more of any of the active agents or drugs comprising:
   (a)
      (i) a triple monoamine reuptake inhibitor (TRI) and a melanin concentrating hormone receptor 1 (MCHR1) antagonist,
      (ii) a triple monoamine reuptake inhibitor (TRI) and a diazoxide (or PROGLYCEM^{™}) or a diazoxide Choline Controlled-Release (DCCR formulation),
      (iii) a melanin concentrating hormone receptor 1 (MCHR1) antagonist and a diazoxide (or PROGLYCEM^{™}) or a diazoxide Choline Controlled-Release (DCCR formulation), or
      (iv) a triple monoamine reuptake inhibitor (TRI) a melanin concentrating hormone receptor 1 (MCHR1) antagonist, and, a diazoxide (or PROGLYCEM^{™}) or a diazoxide Choline Controlled-Release (DCCR formulation); or
   (b)
      (i) a triple monoamine reuptake inhibitor (TRI),
      (ii) a melanin concentrating hormone receptor 1 (MCHR1) antagonist,
      (iii) a diazoxide Choline Controlled-Release (DCCR formulation), or
   (c)
      (a) or (b) formulated with any one or several of the following drugs or small molecules:
      (1) an unacylated ghrelin (UAG) analog and/or livolitide (also known as AZP-531),
      (2) carbetocin, or DURATOCIN^{™}, PABAL^{™} or LONACTENE^{™},
      (3) oxytocin and/or the precursor oxytocin-neurophysin,
      (4) liraglutide, or VICTOZA^{™} or SAXENDA^{™},
      (5) exenatide, or BYETTA^{™}, BYDUREON^{™}, BYDUREON^{™} or BCISE^{™},
      (6) setmelanotide (also known as IMCIVREE^{™}, RM-493, BIM-22493, IRC-022493, N2-Acetyl-L-arginyl-L-cysteinyl-D-alanyl-L-histidyl-D-phenylalanyl-L-arginyl-L-tryptophyl-L-cysteinamide),
      (7) rimonabant (also known as SR141716) and/or ACOMPLIA^{™}, ZIMULTI^{™},
      (8) a beta adrenergic blocker, wherein optionally the beta adrenergic blocker is or comprises: metoprolol (or LOPRESSOR^{™}, METOLAR XR^{™}, TOPROL X^{™}), atenolol (or TENORMIN^{™}), propranolol (or INDERAL^{™}) and/or nadolol (or CORGARD^{™}), or any combination thereof,
      (9) a melatonin receptor agonist, wherein optionally the melatonin receptor agonist is or comprises: melatonin or N-acetyl-5-methoxy tryptamine, ramelteon (or ROZEREM^{™}) and/or tesimelteon (or HETLIOZ^{™}), or any combination thereof,
      (10) a histamine receptor 1 antagonist, wherein the histamine receptor 1 antagonist is or comprises: doxepin (or SINEQUAN^{™}, QUITAXON^{™} or APONAL^{™}), or doxepin low dose (wherein optionally the low dose is 3 mg or 6 mg per dose, trazadone (or DESYREL^{™}, DESYREL DIVIDOSE^{™} or OLEPTRO^{™}), amitriptyline (or ELAVIL^{™}, PROTANOL^{™}, QUALITRIPTINE^{™}, REDOMEX^{™} or SAROTEN^{™}) or amitriptyline with chlordiazepoxide (MORELIN^{™}, RISTRYL^{™} or SEDANS^{™}), and/or mirtazapine (or REMERON^{™}), or any combination thereof,
      (11) a histamine H3 receptor antagonist or inverse agonist, wherein optionally the H3 receptor antagonist or inverse agonist is or comprises: pitolisant (or tiprolisant, ciproxidine or WAKIX^{™}), thioperamide, clobenpropit, ciproxifan, conessine (or nerine, roquessine, wrightine) and/or betahistine (or SERC^{™}), SUVN-G3031 (Suven Life Sciences Ltd), or any combination thereof,
      (12) modafinil (or PROVIGIL^{™}, ALERTEC^{™} or MODAVIGIL^{™}), and/or amodafonil (or NUVIGIL^{™}),
      (13) human growth hormone (hGH) (or somatotropin) or recombinant forms thereof (or OMNITROPE^{™}, JINTROPIN^{™}, NUTROPIN^{™} or NUTROPIN DEPOT^{™}, HUMATROPE^{™}, GENOTROPIN^{™}, NORDITROPIN^{™} or SAIZEN^{™}), or any combination thereof,
      (14) testosterone and/or 17β-Hydroxyandrost-4-en-3-one,
      (15) progesterone and/or pregn-4-ene-3,20-dione,
      (16) estrogen, estrone, estradiol or estriol, or any combination thereof,
      (17) a thyroid hormone or derivative thereof, wherein optionally the thyroid hormone or derivative thereof is or comprises: triiodothyronine (T3), thyroxine (T4), levothyroxine or L-thyroxine, or any combination thereof,
      (18) chorionic gonadotropin (hCG) hormone or recombinant forms thereof, or NOVAREL^{™} or PREGNYL^{™}, or any combination thereof,
      (19) metformin (or GLUCOPHAGE^{™}) and/or repaglinide (or PRANDIN)^{™},
      (20) an insulin or human insulin or recombinant or analog forms thereof (or ACTRAPID^{™}, HUMALOG^{™}, NOVORAPID^{™}, APIDRA^{™}, LANTUS^{™} or LEVEMIR^{™}) or any combination thereof,
      (21) a glucocorticoid receptor antagonist or an anticorticosteroid, or mifepristone (or RU-486, or MIFEGYNE^{™} or MIFEPREX^{™}), metyrapone (or METOPIRONE^{™}), ketoconazole (or NIZORAL^{™}) or aminoglutethimide (or ELIPTEN^{™}, CYTADREN^{™} or ORIMETEN^{™}),
      (22) a histamine receptor 2 antagonist, wherein optionally the histamine receptor 2 antagonist is or comprises: cimetidine (or TAGAMET^{™}), ranitidine (or ZANTAC^{™}) and/or famotidine (or PEPCID^{™}), or any combination thereof,
      (23) a mood stabilizer, wherein optionally the mood stabilizer is or comprises: gabapentin (or NEURONTIN^{™}); clonazepam (or KLONOPIN^{™} or RIVOTRILF^{™}); valproate, valproic acid, sodium valproate or valproate semisodium (or CONVULEX^{™}, DEPAKOTE^{™}, EPILIM^{™} or STAVZOR^{™}); oxcarbazepine (or TRILEPTAL^{™} or OXTELLAR XR^{™}); lithium or lithium carbonate (or LITHOBID^{™} or LITHOMAX^{™}); topiramate (or TOPAMAX^{™}, TROKENDI XR^{™} or QUDEXY XR^{™}) and/or lamotrigine (or LAMICTAL^{™}), or any combination thereof,
      (24) a neuroleptic, wherein optionally the neuroleptic comprises: risperidone (or Risperdal), aripiprazole (or ABILIFY^{™}), quetiapine (or SEROQUEL^{™}), olanzapine (or ZYPREXA^{™}), ziprasidone (or GEODON^{™}) and/or haloperidol (or HALDOL^{™} or SERENACE^{™}), or any combination thereof,
      (25) quetiapine, or SEROQUEL^{™} or TEMPROLIDE^{™},
      (26) naltrexone (or FEBIA^{™} or VIVITROL^{™}),
      (27) γ-aminobutyric acid (GABA) B (GABA_{B}) receptors modulator such as sodium oxybate (Xyrem^{™},), or controlled-release sodium oxybate (or FT218), or a low sodium oxybate, optionally JZP-258, or baclofen,
      (28) solriamfetol or SUNOS^{™},
      (29) hypocretin/orexin 2 receptor-selective agonists, optionally TAK-925, TAK-988 or TAK-994,
      (30) a selective norepinephrine reuptake inhibitor (NRI), or a selective serotonin reuptake inhibitor (SSRI), or a selective serotonin norepinephrine inhibitor (SNRI), wherein optionally the SSRI or SNRI inhibitor is or comprises reboxetine (or AXS-12, or Edronax^{™}), atomoxetine (or Strattera^{™}), venlafaxine (Effexor^{™} or Effexor XR^{™}), fluoxetine (Prozac^{™}), citalopram (Celexa^{™}), escitalopram (Lexapro), paroxetine (Paxil^{™}), sertraline (Zoloft^{™}), or duloxetine (Cymbalta^{™}),
      (31) an amphetamine, wherein optionally the amphetamine or comprises amphetamine, dextroamphetamine (or Adderall^{™}), dextroamphetamine-amphetamine (Mydayis^{™}) or lisdexamfetamine (or Vyvanse^{™}).
      (32) methylphenidate or Ritalin, Ritalin LA, Concerta, Metadate CD, Methylin, Methylin ER, Daytrana, Quillivant XR, Quillichew ER, Aptensio XR, Cotempla XR-ODT, Jornay PM, or Adhansia XR,
      (33) a tricyclic antidepressant (TCA) wherein optionally the TCA is or comprises imipramine (Tofranil^{™}), or amitriptyline (Elavil^{™}), clomipramine (Anafranil^{™}) or another TCA,
      (34) a monoamine oxidase inhibitor (MAOI) wherein optionally the MAOI is or comprises selegiline (Emsam^{™}), isocarboxazid (Marplan^{™}), phenelzine (Nardil^{™}), and tranylcypromine (Parnate^{™}) or another MAOI,
      (35) THN102, or a combination of modafinil and flecainide,
      (36) an inhibitor of astroglial connexins inhibitor, wherein optionally the inhibitor of astroglial connexins inhibitor is or comprises flecainide,
      (37) a prostaglandin DP1 receptor antagonist, wherein optionally the prostaglandin DP1 receptor antagonist is or comprises ONO-4127Na,
      (38) an opioid, wherein optionally the opioid comprises morphine,
      (39) an anti-obesity medication, wherein optionally the anti-obesity medication comprises lorcaserin (Belviq^{™}), orlistat (Alli^{™}), phentermine and topiramate (Qsymia^{™}), ornaltrexone HCl or bupropion HCl (Contrave^{™}),
      (40) a famesoid X receptor (FXR) agonist, wherein optionally the FXR agonist comprises Obeticholic Acid (OCA), EYP001 (ENYO Pharma), TQA3526, Px-102, Px-104, or tropifexor,
      (41) a PPAR agonist, optionally a PPAR α/δ agonist, or a PPAR-α/γ agonist, wherein optionally the PPAR-α/γ agonist comprises pioglitazone, elafibranor or lanifibranor (or IVA337, Inventiva),
      (42) a CC chemokine receptor CCR2/CCR5 inhibitor, wherein optionally the CC chemokine receptor comprises tropifexor, a combination of tropifexor and cenicriviroc, or cenicriviroc,
      (43) a mitochondrial pyruvate carrier (MPC) inhibitor, wherein optionally the MPC inhibitor comprises MSDC-0602K (Cirius Therapeutics),
      (44) a Fibroblast Growth Factor 19 (FGF19) analogue, wherein optionally the FGF19 analogue comprises aldafermin (or NGM282, NGM Biopharmaceuticals)
      (45) a Fibroblast Growth Factor 21 (FGF21) analogue, wherein optionally the FGF21 analogue comprises a PEGylated Fibroblast Growth Factor 21 analogue, optionally pegbelfermin,
      (46) a thyroid hormone receptor beta (THR-β) agonist, wherein optionally the THR-β agonist comprises resmetirom (MGL-3196, Magrigal Pharmaceuticals) or VK-2890,
      (47) a Stearoyl-CoA desaturase-1 (SCD1) inhibitor, wherein optionally the SCD1 inhibitor comprises aramchol (Galmed Pharmaceuticals),
      (48) an apoptosis signal-regulating kinase 1 (ASK1) inhibitor, wherein optionally the ASK1 inhibitor comprises selonsertib (Gilead Sciences),
      (49) an acetyl-CoA carboxylase (ACC) inhibitor, wherein optionally the ACC inhibitor comprises firsocostat (GS-0976) or MK-4074, or
      (50) any combination of (1) to (49).
2. The therapeutic combination, pharmaceutical dosage form, drug delivery device or product of manufacture of item 1, wherein the triple monoamine reuptake inhibitor is or comprises:
   (a) tesofensine, tesomet or tesofen (Saniona, Ballerup, Denmark),
   (b) a [1,2,4]triazolo[1,5-a]pyridinyl-6-yl-substituted tetrahydroisoquinoline derivative as described in USPN 8,802,696, or 7-([1,2,4]triazolo[1,5-a]pyridin-6-yl)-4-(3,4-dichlorophenyl)-1,2,3,4-tetrahydroisoquinoline, or a compound having the formula (Formula I): 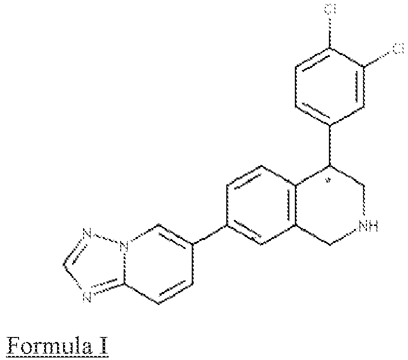 or a pharmaceutically acceptable salt thereof, or a (+)-stereoisomer or a (-)-stereoisomer thereof, or a compound in the S or R configuration, or a (S)(+)-stereoisomer or a (R)(-)-stereoisomer thereof,
   (c) a deuterated form of a compound or drug of (a) or (b).
3. The therapeutic combination, pharmaceutical dosage form, drug delivery device or product of manufacture of item 1, wherein the melanin concentrating hormone receptor 1 (MCHR1) antagonist is or comprises:
   (a) GW8564649 (GSK), AZD-1979 (AstraZeneca), AMG-076 (Amgen), BMS-830216 (BMS), ATC-0065 or ATC-0175 (see Chaki et al (2005) CNS Drug Reviews vol 11(4):341-52), GW-803430 or GW-3430 (see Gehlert et al (2009) J Pharm and Experimental Therapeutics, vol 329(2):429-38), NGD-4715 (Ligand Pharmaceuticals), SNAP-7941 (see Klemenhagen et al (2007) J Pharm and Experimental Therapeutics, vol 321 (1): 237-48), T-226 or T-296 (see Takekawa et al (2002) Eur J Pharm vol 438(3):129-35), or any combination thereof,
   (b) a compound (designated Formula II) having the formula: 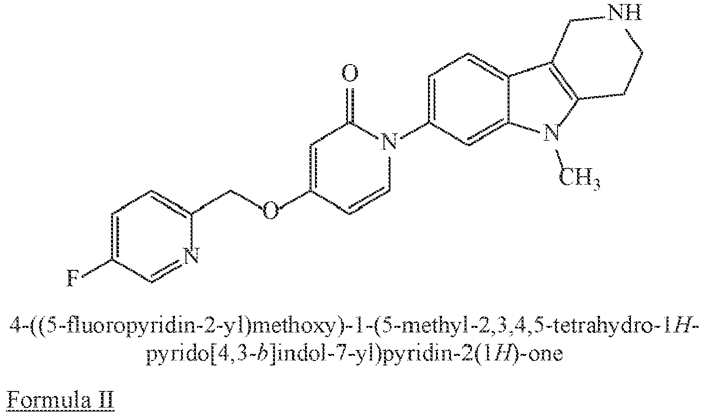 , or
      a (1-azinone)-substituted pyridoindole as described in USPN 8,716, 308, or a compound having the formula: 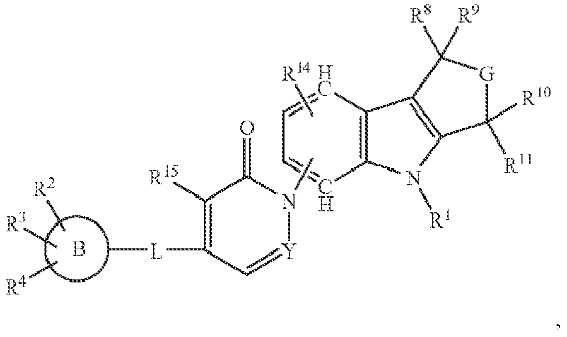
      wherein R₁ is H or optionally substituted alkyl;
      R₂, R₃, R₄ are each independently selected from H, --O-alkyl, --S-alkyl, alkyl, halo, -CF₃, and -CN;
      G is -CR¹²R¹³-NR⁵- or -NR⁵-CR¹²R¹³; R⁵ is H, optionally substituted alkyl, optionally substituted heterocycle, -C(=O)-R⁶,-C(=O)-O-R⁷, or -C(=O)-NR¹⁹R²⁰; R⁶ and R⁷ are each optionally substituted alkyl or optionally substituted heterocycle;
      R₈, R₉, R₁₀, R₁₁, R₁₂, R₁₃, R₁₉ and R₂₀ are each independently selected from H or optionally substituted alkyl; R¹⁴ and R¹⁵ are each independently H or halogen; Y is CH; L is -CH₂-O-, -CH₂CH₂-, -CH=CH- or a bond; and B is aryl or heteroaryl or cycloalkyl; with the proviso that, when L is a direct bond, B cannot be unsubstituted heteroaryl or heteroaryl monosubstituted with fluorine, or
   (c) a deuterated form of a compound or drug of (a) or (b),
      and optionally the therapeutic combination, pharmaceutical dosage form, drug delivery device or product of manufacture further comprises a N-acetyltransferase 2 (NAT2) or arylamine N-acetyltransferase inhibitor, optionally acetaminophen, N-acetyl-para-aminophenol (APAP), or paracetamol (or TYLENOL^{™} or PANADOL^{™}), in combination with any compound of (b) or a deuterated form of a compound or drug of (b), wherein optionally in this combination (b) is Formula II.
4. The therapeutic combination, pharmaceutical dosage form, drug delivery device or product of manufacture of any of items 1 to 3, wherein two or three or more of the drugs or active agents are formulated as separate compositions, or two or three or more of the drugs or active agents are formulated into one composition or drug formulation (two or more drugs or active agents are formulated together).
5. The therapeutic combination, pharmaceutical dosage form, drug delivery device or product of manufacture of any of items 1 to 4, or any of the preceding items , wherein one or two or more of the drugs or active agents are packaged individually, or are packaged together, or packaged in any combination, in a single package, a plurality of packages or packettes, or a blister packet, lidded blister or blister card or packets, or a shrink wrap.
6. The therapeutic combination, pharmaceutical dosage form, drug delivery device or product of manufacture of any of items 1 to 5, or any of the preceding items , wherein one or two or more or all of the drugs or active agents are formulated or manufactured as a parenteral formulation, an aqueous solution, a liposome, an injectable solution, a tablet, a pill, a lozenge, a capsule, a caplet, a spray, a sachet, an inhalant, a powder, a freeze-dried powder, an inhalant, a patch, a gel, a geltab, a nanosuspension, a nanoparticle, a nanoliposome, a microgel, a pellet, a suppository or any combination thereof,
   and optionally the drug delivery device or product of manufacture is or comprises an implant.
7. The therapeutic combination, pharmaceutical dosage form, drug delivery device or product of manufacture of item 6, wherein the one or two or more or all of the drugs or active agents are formulated or manufactured together in one parenteral formulation, one aqueous solution, one liposome, one injectable solution, one freeze-dried powder, one feed, one food, one food supplement, one pellet, one lozenge, one liquid, one elixir, one aerosol, one inhalant, one adhesive, one spray, one powder, one freeze-dried powder, one patch, one tablet, one pill, one capsule, one gel, one geltab, one lozenge, one caplet, one nanosuspension, one nanoparticle, one nanoliposome, one microgel or one suppository.
8. The therapeutic combination, pharmaceutical dosage form, drug delivery device or product of manufacture of any of items 1 to 7, or any of the preceding items , wherein the one or two or more or all of the drugs or active agents are packaged in dosages that match a chrono-dosing regimen to match an optimal dose for the time of day.
9. The therapeutic combination, pharmaceutical dosage form, drug delivery device or product of manufacture of any of items 1 to 8, or any of the preceding items , wherein the drugs or active agents comprise:
   (a) a triple monoamine reuptake inhibitor or a [1,2,4]triazolo[1,5-a]pyridinyl-6-yl-substituted tetrahydroisoquinoline derivative, optionally a compound of Formula I, and a histamine receptor 1 antagonist, optionally doxepin;
   (b) a triple monoamine reuptake inhibitor or a [1,2,4]triazolo[1,5-a]pyridinyl-6-yl-substituted tetrahydroisoquinoline derivative, optionally a compound of Formula I , and naltrexone;
   (c) a triple monoamine reuptake inhibitor or a [1,2,4]triazolo[1,5-a]pyridinyl-6-yl-substituted tetrahydroisoquinoline derivative, optionally a compound of Formula I , and a histamine receptor 1 antagonist, optionally doxepin, and naltrexone;
   (d) a triple monoamine reuptake inhibitor or a [1,2,4]triazolo[1,5-a]pyridinyl-6-yl-substituted tetrahydroisoquinoline derivative, optionally a compound of Formula I , and a diazoxide (or PROGLYCEM^{™}) or a diazoxide Choline Controlled-Release (DCCR formulation);
   (e) a triple monoamine reuptake inhibitor or a [1,2,4]triazolo[1,5-a]pyridinyl-6-yl-substituted tetrahydroisoquinoline derivative, optionally a compound of Formula I , and a diazoxide (or PROGLYCEM^{™}) or a diazoxide Choline Controlled-Release (DCCR formulation), and a melatonin receptor agonist, optionally melatonin or N-acetyl-5-methoxy tryptamine, ramelteon (or ROZEREM^{™}) and/or tesimelteon (or HETLIOZ^{™}), or any combination thereof;
   (f) a triple monoamine reuptake inhibitor or a [1,2,4]triazolo[1,5-a]pyridinyl-6-yl-substituted tetrahydroisoquinoline derivative, optionally a compound of Formula I, and a melanin concentrating hormone receptor 1 (MCHR1) antagonist, optionally a compound of Formula II, or any combination thereof;
   (g) a triple monoamine reuptake inhibitor or a [1,2,4]triazolo[1,5-a]pyridinyl-6-yl-substituted tetrahydroisoquinoline derivative, optionally a compound of Formula I, and a beta blocker, optionally, atenolol (TENORMIN^{™}), bisoprolol (CARDICOR^{™}, EMCOR^{™}), or metoprolol (BETALOC^{™}, LOPRESOR^{™}, TOPROL XL^{™}), or any combination thereof; and/or
   (h) any combination of (a) to (g).
10. A pharmaceutical composition, drug or formulation comprising a compound having the formula: 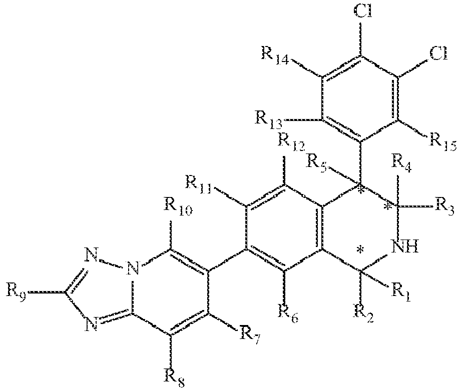
   wherein at least one of R₁ through R₁₅ is -D (deuterium), or all of R₁ through R₁₅ is -D,
   and optionally the carbon atom designated ^{∗} is in an R or an S configuration if it is a stereocenter;
   or a pharmaceutically acceptable salt thereof.
11. The pharmaceutical composition, drug or formulation of item 10, wherein the compound is:
   (a) a 7-([1,2,4]triazolo[1,5-a]pyridin-6-yl)-4-(3,4-dichlorophenyl)-1,2,3,4-tetrahydroisoquinoline-1,1,3,3,4,8-d6 compound having the formula: 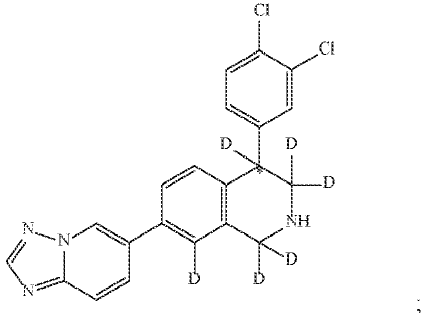
   (b) a 7-([1,2,4]triazolo[1,5-a]pyridin-6-yl)-4-(3,4-dichlorophenyl)-1,2,3,4-tetrahydroisoquinoline-1,1,3,3,4-d5 compound having the formula: 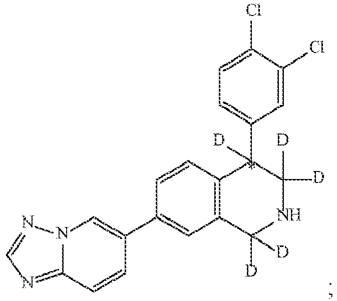
   (c) a7-([1,2,4]triazolo[1,5-a]pyridin-6-yl)-4-(3,4-dichlorophenyl)-1,2,3,4-tetrahydroisoquinoline-1,1-d2 compound having the formula: 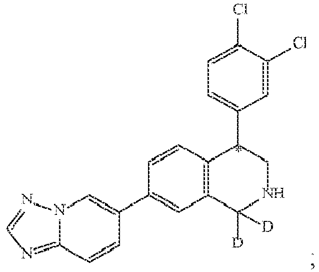
   (d) a 7-([1,2,4]triazolo[1,5-a]pyridin-6-yl)-4-(3,4-dichlorophenyl)-1,2,3,4-tetrahydroisoquinoline-3,3-d2 compound having the formula: 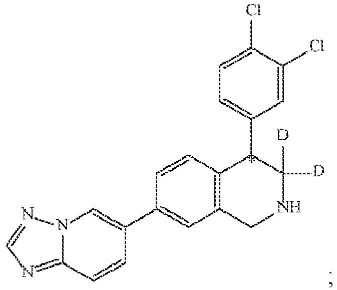
   (e) a 7-([1,2,4]triazolo[1,5-a]pyridin-6-yl)-4-(3,4-dichlorophenyl)-1,2,3,4-tetrahydroisoquinoline-4-d compound having the formula: 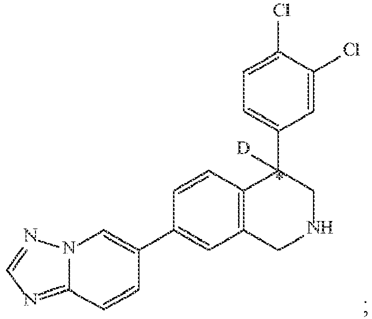
   (f) a 7-([1,2,4]triazolo[1,5-a]pyridin-6-yl)-4-(3,4-dichlorophenyl)-1,2,3,4-tetrahydroisoquinoline-8-d compound having the formula: 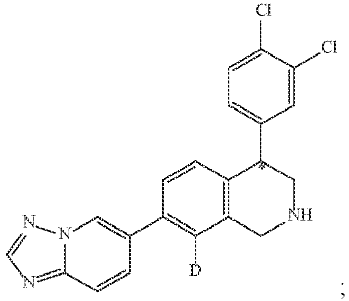 or,
   (g) a 7-([1,2,4]triazolo[1,5-a]pyridin-6-yl)-4-(3,4-dichlorophenyl)-1,2,3,4-tetrahydroisoquinoline-3,3,4-d3 compound having the formula: 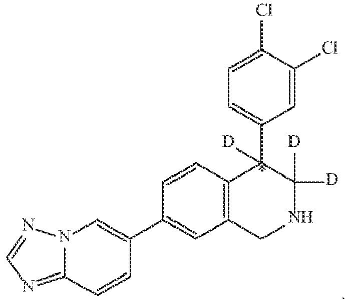
12. A pharmaceutical composition, drug or formulation comprising a compound having the formula: 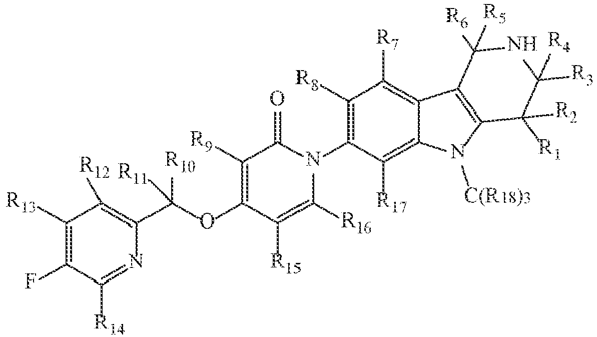
   wherein at least one of R₁ through R₁₈ is -D (deuterium), or all of R₁ through R₁₈ is -D;
   or a pharmaceutically acceptable salt thereof.
13. The pharmaceutical composition, drug or formulation of item 12, wherein the compound is: 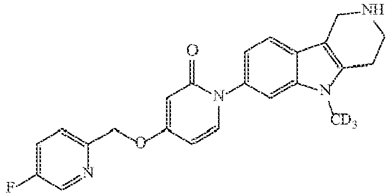
   4-((5-fluoropyridin-2-yl)methoxy)-1-(5-(methyl-*d*₃)-2,3,4,5-tetrahydro-1*H-*pyrido[4,3-*b*]indol-7-yl)pyridin-2(1*H*)-one , 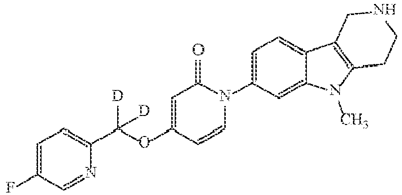
   4-((5-fluoropyridin-2-yl)methoxy-*d*₂)-1-(5-methyl-2,3,4,5-tetrahydro-1*H-*pyrido[4,3-*b*]indol-7-yl)pyridin-2(1*H*)-one 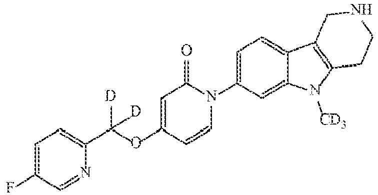
   4-((5-fluoropyridin-2-yl)methoxy-*d*₂)-1-(5-(methyl-*d*₃)-2,3,4,5-tetrahydo)-1*H-*pyrido[4,3-*b*]indol-7-yl)pyridin-2(1*H*)-one 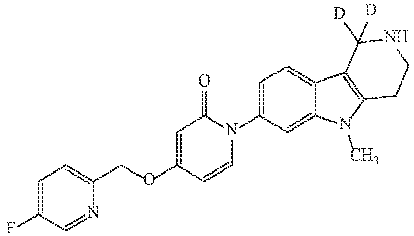
   4-((5-fluoropyridin-2-yl)methoxy)-1-(5-methyl-2,3,4,5-tetrahydro-1*H-*pyrido[4,3-*b*]indol-7-yl-1,1-*d*₂)pyridin-2(1*H*)-one 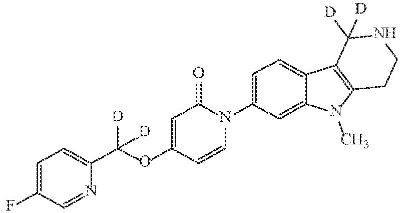
   4-((5-fluoropyridin-2-yl)methoxy-*d*₂)-1-(5-methyl-2,3,4,5-tetrahydro-1*H-*pyrido[4,3-*b*]indol-7-yl-1,1-*d*₂)pyridin-2(1*H*)-one 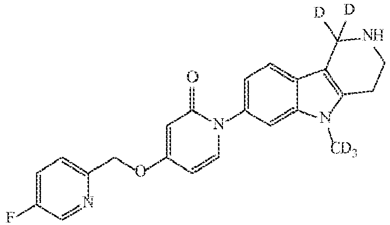
   4-((5-fluoropyridin-2-yl)methoxy)-1-(5-(methyl-*d*₃)-2,3,4,5-tetrahydro-1*H-*pyrido[4,3-*b*]indol-7-yl-1,1-*d*₂)pyridin-2(1*H*)-one 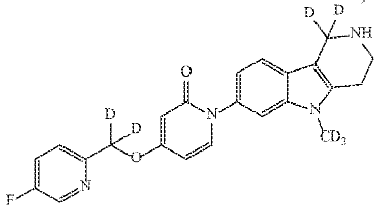
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-1,1-*d*₂)pyridin-2(1*H*)-one , 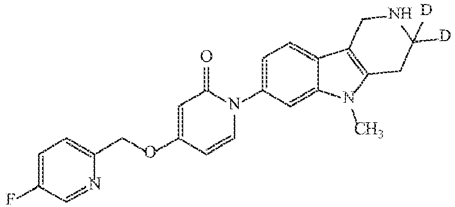
   4-((5-fluoropyridin-2-yl)methoxy)-1-(5-methyl-2,3,4,5-tetrahydro-1*H-*pyrido[4,3-*b*]indol-7-yl-3,3-*d*₂)pyridin-2(1*H*)-one, 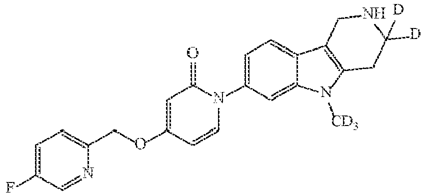
   4-((5-fluoropyridin-2-yl)methoxy)-1-(5-(methyl-*d*₃)-2,3,4,5-tetraydro-1*H-*pyrido[4,3-*b*]indol-7-yl-3,3-*d*₂)pyridin-2(1*H*)-one , 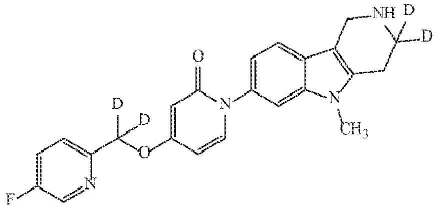
   4-((5-fluoropyridin-2-yl)methoxy-*d*₂)-1-(5-methyl-2,3,4,5-tetrahydro-1*H-*pyrido[4,3-*b*]indol-7-yl-3.3-*d*₂)pyridin-2(1*H*)-one , 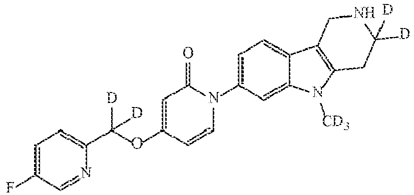
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-3,3-*d*₂)pyridin-2(1*H*)one , 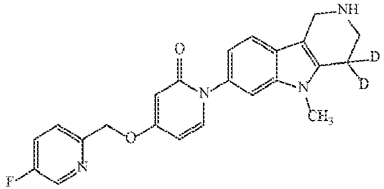
   4-((5-fluoropyridin-2-yl)methoxy)-1-(5-methyl-2,3.4,5-tetrahydro-1*H*-pyrido[4,3-*b*]indol-7,yl-4,4-*d*₂)pyridin-2(1*H*)-one , 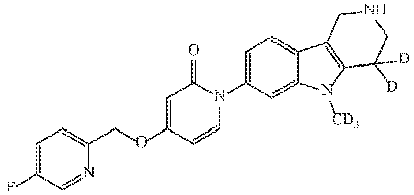
   4-((5-fluoropyridin-2-yl)methoxy)-1-(5-(methyl-*d*₃)-2,3,4,5-tetrahydro)-1*H-*pyrido[4,3-b]indol-7-yl-4,4-*d*₂)pyridin-2(1*H*)-one 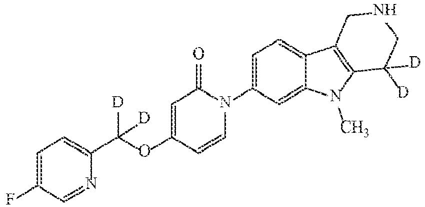
   4-((5-fluoropyridin-2-yl)methoxy-*d*₂)-1-(5-methyl-2,3,4,5-tetrahydro-1*H-*pyrido[4,3-*b*]indol-7-yl-4,4-*d*₂)pyridin-2(1*H*)-one , 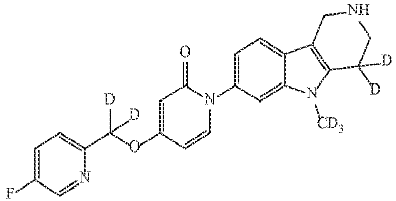
   4-((5-fluoropyridin-2-yl)methoxy-*d*₂)-1-(5-(methyl-*d*₃)-2,3,4,5-tetrahydro)-1*H-*pyrido[4,3-*b*]indol-7-yl-4,4-*d*₂)pyridin-2(1H)-one , 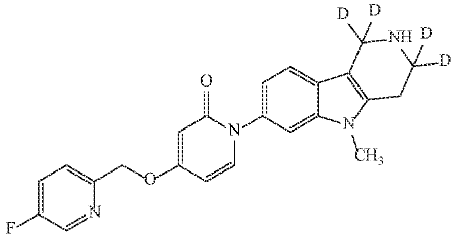
   4-((5-fluoropyridin-2-yl)methxy)-1-(5-methyl-2,3,4,5-tetrahydro-1*H-*pyrido[4,3-*b*]indol-7-yl-1,1,3,3-*d*₄)pyridin-2(1*H*)-one , 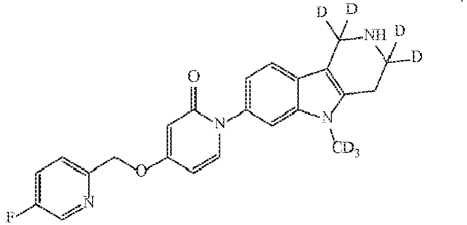
   4-((5-fluoropyridin-2-yl)methoxy)-1-(5-(methyl-*d*₃)-2,3,4,5-tetrahydro-1*H-*pyrido[4.3-*b*]indol-7-yl-1,1,3,3-*d*₄)pyridin-2(1*H*)-one , 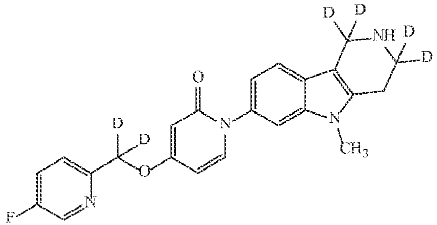
   4-((5-fluoropyridin-2-yl)methoxy-*d*₂)-1-(5-methyl-2,3,4,5-tetrahydro-1*H-*pyrido[4,3-*b*]indol-7-yl-1,1,3,3-*d*₄)pyridin-2(1*H*)-one , 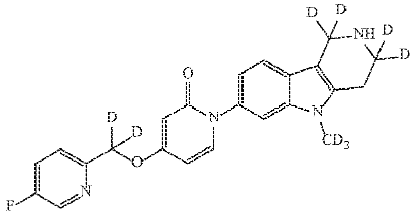
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-1,1,3,3-*d*₄)pyridin-2(1*H*)-one , 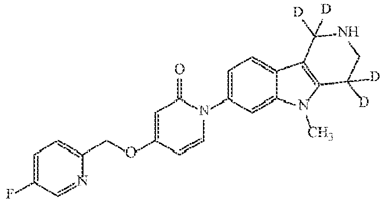
   4-((5-fluoropyridin-2-yl)methoxy)-1-(5-methyl-2,3,4,5-tetrahydio-1*H*-pyrido[4,3-*b*]indol-7-yl-1,1,4,4-*d*₄)pyridin-2(1*H*)-one , 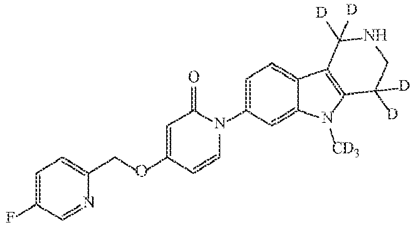
   4-((5-fluoropyridin-2-yl)methoxy)-1-(5-(methyl-*d*₃))-2,3,4,5-tetrahydro-1*H-*pyrido[4,3-*b*]indol-7-yl-1,1,4,4-*d*₄)pyridin-2(1*H*)-one , 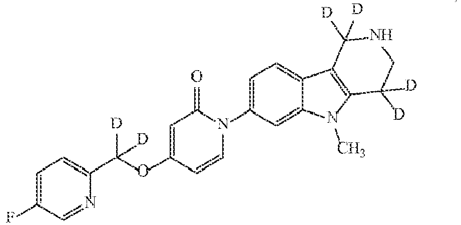
   4-((5-fluoropyridin-2-yl)methoxy-*d*₂)-1-(5-methyl-2,3,4,5-tetrahydro-1*H-*pyrido[4,3-*b*]indol-7-yl-1,1,4,4-*d*₄)pyridin-2(1*H*)-one , 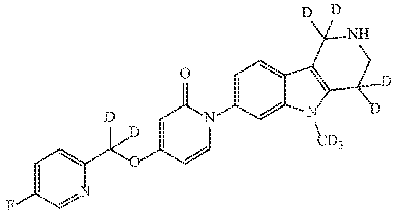
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-1,1,4,4-*d*₄)pyridin-2(1*H*)-one , 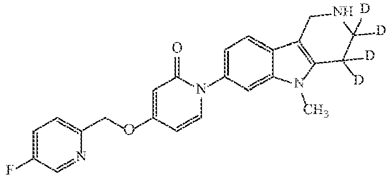
   4-((5-fluoropyridin-2-yl)methoxy)-1-(5-methyl-2,3,4,5-tetrahydro-1*H*-pyrido[4,3-*b*]indol-7-yl-3,3,4,4-*d*₄)pyridin-2(1*H*-one , 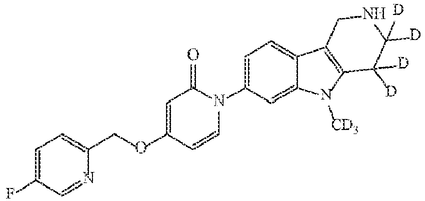
   4-((5-fluoropyridin-2-yl)methoxy)-1-(5-(methyl-*d*₃)-2,3,4,5-tetrahiydro-1*H-*pyrido[4,3-*b*]indol-7-yl-3,3,4,4-*d*₄)pyridin-2(1*H*)-one , 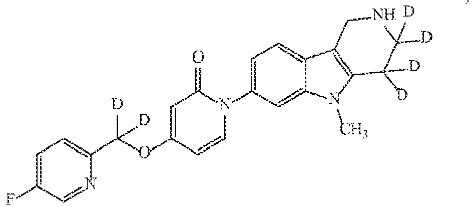
   4-((5-fluoropyridin-2-yl)methoxy-*d*₂)-1-(5-methyl-2,3,4,5-tetrahydro-1*H-*pyrido[4,3-*b*]indol-7-yl-3,3,4,4-*d*₄)pyridin-2(1*H*)-one , 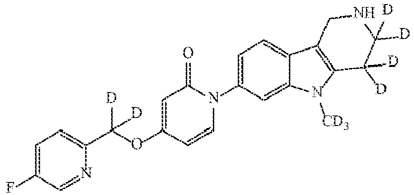
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-3,3,4,4-*d*₄)pyridin-2(1*H*)-one , 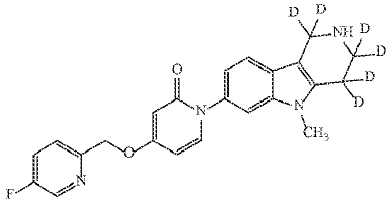
   4-((3-fluoropyridin-2-yl)methoxy)-1-(3-methyl-2,3,4,5-tetrahydro-1*H*-pyrido[4,3-*b*]indol-7-yl-1,1,3,3,4,4-*d*₆)pyridin-2(1*H*)-one , 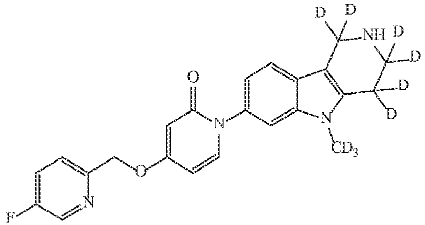
   4-((5-fluoropyridin-2-yl)methoxy)-1-(5-methyl-*d*₃)-2,3,4,5-tetrahydro-1*H-*pyrido[4,3-*b*]indol-7-yl-1,1,3,3,4,4-*d*₆)pyridin-2(1*H*)-one , 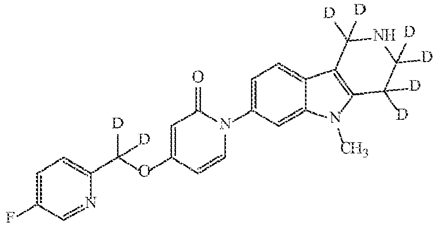
   4-((5-fluoropyridin-2-yl)methoxy-*d*₂)-1-(5-methyl-2,3,4,5-tetrahydro-1*H-*pyrido[4,3-*b*]indol-7-yl-1,1,3,3,4,4-*d*₆)pyridin-2(1*H*)-one , 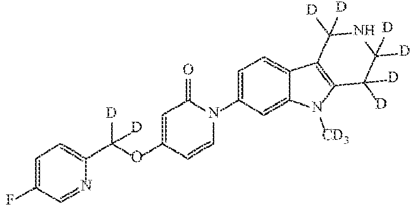
   4-((5-fluoropyridin-2-yl)methoxy-*d*₂)-1-(5-(methyl-*d*₃)-2,3,4,5-tetrahydro-1*H-*pyrido[4,3-*b*]indol-7-yl-1,1,3,3,4,4-*d*₆)pyridin-2(1*H*)-one , or 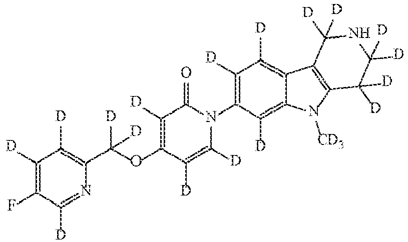
   4-((5-fluoropyridin-2-yl-3,4,6-*d*₃)methoxy-*d*₂)-1-(5-(methyl-*d*₃)-2,3,4,5-tetrahydro-1*H*-pyrido[4,3-*b*]indol-7-yl-1,1,3,3,4,4,6,8,9-*d*₉)pyridin-2(1*H*)-one-3,5,6-*d*₃
14. A method for treating, ameliorating, slowing the progress of, reducing the symptoms of, reducing adverse events associated with, or preventing, a disease or condition comprising or associated with:
   hyperphagia (optionally as assessed by HQ-CT),
   moderate to severe binge eating disorder (BED),
   bulimia nervosa,
   management of obesity, optionally further comprising management of weight loss and maintenance of weight loss, or optionally further comprising as an adjunct to a reduced-calorie diet or for increased physical activity for chronic weight management,
   early onset morbid obesity,
   non-Alcoholic Steatohepatitis (NASH),
   non-alcoholic fatty liver disease (NAFLD),
   Primary sclerosing cholangitis (PSC),
   Primary biliary cholangitis liver (PBC),
   inflammatory bowel disease (IBD), or irritable bowel syndrome (IBS),
   type II diabetes,
   hypothalamic injury-induced obesity,
   obsessive-compulsive disorder (OCD),
   disruptive mood dysregulation disorder (DMDD),
   oppositional defiant disorder (ODD),
   excoriation,
   trichotillomania,
   intermittent explosive disorder (IED),
   excessive daytime sleepiness (EDS),
   excessive daytime sleepiness associated with narcolepsy and sleep apnea,
   excessive daytime sleepiness associated with narcolepsy,
   excessive daytime sleepiness associated with central or obstructive sleep apnea,
   narcolepsy,
   narcolepsy type 1 (NT1) according to the International Classification of Sleep Disorders-Third Edition (ICSD-3),
   narcolepsy with cataplexy,
   narcolepsy type 2 (NT2) according to ICSD-3,
   narcolepsy without cataplexy,
   cataplexy,
   idiopathic hypersomnia,
   rapid eye movement (REM) sleep behavior disorder,
   seizures,
   epilepsy, an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
   major depressive disorder (MDD),
   treatment resistant depression (TRD),
   negative symptoms of schizophrenia,
   MDD Associated with Parkinson's Disease,
   Parkinson's Disease,
   general anxiety,
   Social Anxiety Disorder (Social Phobia),
   Fibromyalgia (FM),
   diabetic neuropathy.
   lower back pain,
   chronic fatigue syndrome,
   attention deficit hyperactivity disorder (ADHD),
   autism,
   Asperger spectrum, a genetically confirmed disease or syndrome, wherein optionally the genetically confirmed disease or syndrome is or comprises Prader-Willi Syndrome, Bardet Biedl Syndrome, Smith-Magenis Syndrome, 1p36 deletion syndrome, 16p11.2 deletion Syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,
   wherein optionally reducing adverse events comprises reducing psychosis, serotonin syndrome, tachycardia or postural orthostatic tachycardia syndrome,
   the method comprising:
   (a)
      (i) providing or having provided a therapeutic combination, pharmaceutical dosage form, drug delivery device or product of manufacture of any of the preceding items , or a pharmaceutical composition of any of the preceding items , and
      (ii) administering to or implanting into an individual in need thereof the therapeutic combination, pharmaceutical dosage form, drug delivery device or product of manufacture, or
   (b) administering to or implanting into an individual in need thereof a therapeutically effective dose of the therapeutic combination, pharmaceutical dosage form, drug delivery device or product of manufacture of any of the preceding items .
15. The method of item 14, wherein the drug, or therapeutic combination, pharmaceutical dosage form, is administered orally, parenterally, by inhalation spray, nasally, topically, intrathecally, intrathecally, intracerebrally, epidurally, intracranially or rectally,
   and optionally parenteral administration comprises administration intrathecally, intracerebrally or epidurally (into a intrathecal, intracerebral, epidural space), subcutaneously, intravenously, intramuscularly and/or intraarterially.
16. The method of item 14 or item 15, wherein the drug, or therapeutic combination, pharmaceutical dosage form, is administered to achieve a therapeutic level range of plasma concentration at a steady state, and wherein:
   the therapeutic level range of plasma concentration for the [1,2,4]triazolo[1,5-a]pyridinyl-6-yl-substituted tetrahydroisoquinoline derivative, or a compound of Formula I , is between about: 50 to 4000 ng/ml; 100 to 3000 ng/ml; 250 to 1600 ng/ml; 500 to 1400 ng/ml, 600 to 1300 ng/ml, 800 to 1250 ng/ml, 1000 ng/ml to 1250 ng/ml, 1250 to 1500 ng/ml, 1500 to 2000 ng/ml, 2000 to 2500 ng/ml; 2500 to 3000 ng/ml; 3000 to 3500 ng/ml; or, 3500 to 4000 ng/ml;
   the therapeutic level range of plasma concentration for the (1-azinone)-substituted pyridoindole, or a compound of Formula II, is between about: 5 to 4000 ng/ml, 10 to 1000 ng/ml, 10 to 500 ng/ml, 25 to 500 ng/ml, 25 to 250 ng/ml, 50 to 500 ng/ml, 50 to 1000 ng/ml, 50 to 500 ng/ml, 100 to 1000 ng/ml, 100 to 500 ng/ml, 200 to 1000 ng/ml, 500 to 1000 ng/ml, 1000 to 2000 ng/ml, 2000 to 3000 ng/ml, or 3000 to 4000 ng/ml,
   the therapeutic level range of plasma concentration for tesofensine is between about: 2 ng to 50 ng/ml, 5 to 20 ng/ml, 6.5 to 15 ng/ml, 8 to 12 ng/ml, or about 10 ng/ml,
   the therapeutic level range of plasma concentration for diazoxide is between about: 10 ng/ml to 100 ng/ml, 20 to 80 ng/ml, 30 to 50 ng/ml, or about 40 ng/ml.
17. The method of item 16, wherein the drug, therapeutic combination, or pharmaceutical dosage form, is administered to achieve a therapeutic level range of plasma concentration (optionally human plasma concentration) at a steady state, and wherein:
   (a) the therapeutic level range of plasma concentration for a compound of Formula I, is between about: 250 to 1600 ng/ml; 500 to 1400 ng/ml, 600 to 1300 ng/ml, 800 to 1250 ng/ml, 1000 ng/ml to 1250 ng/ml, 1250 to 1500 ng/ml, 1500 to 2000 ng/ml, 2000 to 2500 ng/ml; or 2500 to 3000 ng/ml;
   (b) the therapeutic level range of plasma concentration for a compound of Formula I, is between about: 600 to 1300 ng/ml, 800 to 1250 ng/ml, 1000 ng/ml to 1250 ng/ml, 1250 to 1500 ng/ml, 1500 to 2000 ng/ml, or 2000 to 2500 ng/ml; or
   (c) the therapeutic level range of plasma concentration for a compound of Formula I, is between about: 600 to 1300 ng/ml, 800 to 1250 ng/ml, 1000 ng/ml to 1250 ng/ml, 1250 to 1500 ng/ml.
18. The method of item 16 to 17, wherein the steady state is achieved after:
   (a) between about 3 to 5 times the elimination half-life (T1_{/2}) of the drug, therapeutic combination, or pharmaceutical dosage form in a subject or a patient, or
   (b) between about 7 to 14 days after periodic or regular administration to a optionally once-a-day dosing of the drug, or therapeutic combination, pharmaceutical dosage form.
19. The method of item 16 to 18, wherein the plasma concentration for the drug, or therapeutic combination, pharmaceutical dosage form is:
   (a) the trough level or trough concentration (C_{trough}), or the lowest concentration reached by the drug, or therapeutic combination or pharmaceutical dosage form before a second or next dose is administered, or
   (b) determined from blood samples taken between about 2 hours to 24 hours, or 4 to 12 hours, or 1, 2, 3, 4, 5, 6, 7, 8, 9, or 10 or more hours, after the last dose or administration of the drug, or therapeutic combination, pharmaceutical dosage form.
20. The method of any of items 14 to 19, or a method of any of the preceding items , wherein each drug or active agent of the therapeutic combination, pharmaceutical dosage form, drug delivery device or product of manufacture is delivered to the individual simultaneously, or separately, wherein optionally one or each or several drug or active agent of the therapeutic combination, pharmaceutical dosage form, drug delivery device or product of manufacture is administered in a chronodosed regimen.
   The method of any of items 14 to 20, or a method of any of the preceding items , wherein the therapeutic combination, pharmaceutical dosage form, drug delivery device or product of manufacture comprises Formula I, or a deuterated derivative of Formula I.
21. Use of a therapeutic combination, pharmaceutical dosage form, drug delivery device or product of manufacture of any of the preceding items for treating, ameliorating, slowing the progress of, reducing the symptoms of, reducing adverse events associated with, or preventing, a disease or condition comprising or associated with:
   hyperphagia (optionally as assessed by HQ-CT),
   moderate to severe binge eating disorder (BED),
   bulimia nervosa,
   management of obesity, optionally further comprising management of weight loss and maintenance of weight loss, or optionally further comprising as an adjunct to a reduced-calorie diet or for increased physical activity for chronic weight management,
   early onset morbid obesity,
   non-Alcoholic Steatohepatitis (NASH), non-alcoholic fatty liver disease (NAFLD),
   Primary sclerosing cholangitis (PSC),
   Primary biliary cholangitis liver (PBC),
   inflammatory bowel disease (IBD), or irritable bowel syndrome (IBS),
   type II diabetes,
   hypothalamic injury-induced obesity,
   obsessive-compulsive disorder (OCD),
   disruptive mood dysregulation disorder (DMDD),
   oppositional defiant disorder (ODD),excoriation,
   trichotillomania,
   intermittent explosive disorder (IED),
   excessive daytime sleepiness (EDS),
   excessive daytime sleepiness associated with narcolepsy and sleep apnea,
   excessive daytime sleepiness associated with narcolepsy,
   excessive daytime sleepiness associated with central or obstructive sleep apnea,
   narcolepsy,
   narcolepsy type 1 (NT1) according to the International Classification of Sleep Disorders-Third Edition (ICSD-3),
   narcolepsy with cataplexy,
   narcolepsy type 2 (NT2) according to ICSD-3,
   narcolepsy without cataplexy,
   cataplexy,
   idiopathic hypersomnia,
   rapid eye movement (REM) sleep behavior disorder,
   seizures,
   epilepsy, an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
   major depressive disorder (MDD),
   treatment resistant depression (TRD),
   negative symptoms of schizophrenia,
   MDD Associated with Parkinson's Disease,
   Parkinson's Disease,
   general anxiety,
   Social Anxiety Disorder (Social Phobia),
   Fibromyalgia (FM),
   diabetic neuropathy.
   lower back pain,
   chronic fatigue syndrome,
   attention deficit hyperactivity disorder (ADHD),
   autism,
   Asperger spectrum, a genetically confirmed disease or syndrome, wherein optionally the genetically confirmed disease or syndrome is or comprises Prader-Willi Syndrome, Bardet Biedl Syndrome, Smith-Magenis Syndrome, 1p36 deletion syndrome, 16p11.2 deletion Syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,
   wherein optionally reducing adverse events comprises reducing psychosis, serotonin syndrome, tachycardia or postural orthostatic tachycardia syndrome.
22. The method of item 21, wherein the therapeutic combination, pharmaceutical dosage form, drug delivery device or product of manufacture comprises Formula I, or a deuterated derivative of Formula I.
23. A therapeutic combination, pharmaceutical dosage form, drug delivery device or product of manufacture of any of the preceding items for use in treating, ameliorating, slowing the progress of, reducing the symptoms of, reducing adverse events associated with, or preventing, a disease or a condition comprising or associated with:
   hyperphagia (optionally as assessed by HQ-CT),
   moderate to severe binge eating disorder (BED),
   bulimia nervosa,
   management of obesity, optionally further comprising management of weight loss and maintenance of weight loss, or optionally further comprising as an adjunct to a reduced-calorie diet or for increased physical activity for chronic weight management,
   early onset morbid obesity,
   non-Alcoholic Steatohepatitis (NASH),
   non-alcoholic fatty liver disease (NAFLD),
   Primary sclerosing cholangitis (PSC),
   Primary biliary cholangitis liver (PBC),
   inflammatory bowel disease (IBD), or irritable bowel syndrome (IBS),
   type II diabetes,
   hypothalamic injury-induced obesity,
   obsessive-compulsive disorder (OCD),
   disruptive mood dysregulation disorder (DMDD),
   oppositional defiant disorder (ODD),
   excoriation,
   trichotillomania,
   intermittent explosive disorder (IED),
   excessive daytime sleepiness (EDS),
   excessive daytime sleepiness associated with narcolepsy and sleep apnea,
   excessive daytime sleepiness associated with narcolepsy,
   excessive daytime sleepiness associated with central or obstructive sleep apnea,
   narcolepsy,
   narcolepsy type 1 (NT1) according to the International Classification of Sleep Disorders-Third Edition (ICSD-3),
   narcolepsy with cataplexy,
   narcolepsy type 2 (NT2) according to ICSD-3,
   narcolepsy without cataplexy,
   cataplexy,
   idiopathic hypersomnia,
   rapid eye movement (REM) sleep behavior disorder,
   seizures,
   epilepsy,
   an addiction, or an addictive disorder or behavior, wherein optionally the addiction, addictive disorder or addictive behavior is or comprises Internet Gaming Disorder (IGD) or a drug addiction, and optionally the drug addiction is or comprises cocaine dependence or alcohol dependence,
   major depressive disorder (MDD),
   treatment resistant depression (TRD),
   negative symptoms of schizophrenia,
   MDD Associated with Parkinson's Disease,
   Parkinson's Disease,
   general anxiety,
   Social Anxiety Disorder (Social Phobia),
   Fibromyalgia (FM), diabetic neuropathy.
   lower back pain,
   chronic fatigue syndrome,
   attention deficit hyperactivity disorder (ADHD),
   autism,
   Asperger spectrum, a genetically confirmed disease or syndrome, wherein optionally the genetically confirmed disease or syndrome is or comprises Prader-Willi Syndrome, Bardet Biedl Syndrome, Smith-Magenis Syndrome, 1p36 deletion syndrome, 16p11.2 deletion syndrome, fragile X syndrome, proopiomelanocortin (POMC) deficiency obesity, leptin receptor (LEPR) deficiency obesity, Melanocortin 4 Receptor (MC4R) Pathway Heterozygous Obesity, trisomy 21, Rett syndrome, cyclin-dependent kinase-like 5 (CDKL-5) X-linked genetic disorder, Angelman's Syndrome, Schaff-Yang Syndrome, Albright Hereditary Osteodystroph, Silver-Russell Syndrome, Maternal Disomy 14, Alström Syndrome, Wilms' Tumor, Aniridia, Genitourinary Anomalies, Mental Retardation or WAGR or WAGRO Syndrome; Dravet Syndrome, Lennox-Gastaut syndrome, Gillespie syndrome or cerebellar ataxia, or Doose Syndrome or myoclonic atonic epilepsy (MAE), or Niemann-Pick type C disease, or Norrie disease, or Coffin-Lowry disease,
   wherein optionally reducing adverse events comprises reducing psychosis, serotonin syndrome, tachycardia or postural orthostatic tachycardia syndrome.
24. The method of item 23, wherein the therapeutic combination, pharmaceutical dosage form, drug delivery device or product of manufacture comprises Formula I, or a deuterated derivative of Formula I.
25. A method for administering:
   (i) a triple monoamine reuptake inhibitor (TRI),
   (ii) a melanin concentrating hormone receptor 1 (MCHR1) antagonist,
   (iii) a diazoxide or diazoxide formulation,
   (iv) a therapeutic combination of any of (i) to (iii),
   (iv) a therapeutic combination as used in a method of any of the preceding items , or a therapeutic combination or as used in any of items 13 to 17, or
   (v) a pharmaceutical composition, drug or formulation as set forth in any of items 10 to 13,
      wherein the dosaging is determined by:
      (a) an empirical method for safe and predictable titration and to determine the initial therapeutic dose, or to determine the lowest therapeutic dose, or to determine an optimal effective dose; the method comprising:
         1. administrating a test dose (for example, a no adverse events observed dose amount or a minimal adverse events observed dose amount or a safe dose amount) daily until a patient achieves a steady state plasma drug concentration,
         2. prior to the next scheduled dose, drawing a blood sample and assessing the test plasma drug concentration,
         3. calculating the initial therapeutic dose/day by:
            A. for drugs with a linear dose to plasma concentration, divide the initial target therapeutic plasma drug concentration by the test plasma drug concentration, and then multiply the dividend by the test dose amount, and
            B. for drugs with a non-linear dose to plasma concentration, divide the initial target therapeutic plasma drug concentration by the test plasma drug concentration, then multiply the dividend by the test dose amount and then multiply the product by a non-linear index factor, optionally, the initial target therapeutic plasma drug concentration is the concentration corresponding to: (i) certain pharmacodynamic efficacy marker levels, such as occupancy of serotonin transporter (SERT or 5-HTT), dopamine transporter (DAT) or norepinephrine transporter (NET) determined by imaging method such as positron emission tomography (PET); or, (ii) the lowest effective therapeutic dose determined by a clinical study.
         4. optionally comprising:
            i. administrating the initial therapeutic dose, daily, until a patient achieves a steady state plasma drug concentration,
            ii. prior to the next scheduled dose, drawing a blood sample and assessing the current therapeutic plasma drug concentration,
            iii. Calculating the next therapeutic target dose/day by:
               A. For drugs with a linear dose to plasma concentration, divide the next target therapeutic plasma drug concentration by the current therapeutic plasma drug concentration and then multiply the dividend by the current therapeutic dose amount, and
               B. For drugs with a non-linear dose to plasma concentration, divide next target therapeutic plasma drug concentration by the current therapeutic plasma drug concentration, then multiply the dividend by the current therapeutic dose amount and then multiply the product by a non-linear index factor, and
            iv. continuing the process until the lowest therapeutic dose or optimal effective dose is achieved; or
      (b) a model-based method for safe and predictable titration, and to determine the initial therapeutic dose and to determine the lowest therapeutic dose, or to determine an optimal effective dose, and method comprising:
         1. Administrating a test dose amount daily for between about 1 to 28 days, 1 to 21 days, 1 to 14 days, 1 to 10 days, 1 to 7 days, 1 to 3 days, or about 1 day,
         2. Drawing a first blood sample at between about 1 hour to 3 days, 6 hours to 2 days or about 1 day after administering the first test dose,
         3. Drawing a second or subsequent blood samples at between about 1 hour to 28 days, 6 hours to 14 days, 1 day to 7 days, or at about 2 days, after drawing of the first blood samples or subsequent blood samples,
         4. calculating the initial therapeutic starting dose/day by:
            i. using the initial target therapeutic plasma drug concentration, the test dose amount, the time at the administration of the test dose(s), the plasma concentration of the blood samples and the time the draws of the blood samples and a pharmacometric model,
            ii. and optionally, using the initial target therapeutic plasma drug concentration, the test dose amount, the time at the administration of the test dose(s), the plasma concentration of the blood samples, the time of the draws of the blood samples, human PK data and a Bayesian pharmacometric model,
            iii. and optionally, using the initial target therapeutic plasma drug concentration, the test dose amount, the time at the administration of the test dose, the plasma concentration of the blood samples, the time of the draws of the blood samples, human PK data, a patient's genetic information, a patient's non-heritable host factors, a patient's other medications, and a Bayesian pharmacometric model,
         5. and optionally comprising:
            i. administrating the initial therapeutic starting dose, daily until a patient achieves a steady state plasma drug concentration,
            ii. Prior to the next scheduled dose, drawing a blood sample and assessing the current therapeutic plasma drug concentration,
            iii. calculating the next therapeutic target dose/day:
               A. For drugs with a linear dose to plasma concentration, divide the next target therapeutic plasma drug concentration by the current therapeutic plasma drug concentration and then multiply the dividend by the current therapeutic dose amount, and
               B. For drugs with a non-linear dose to plasma concentration, divide he next target therapeutic plasma drug concentration by the current therapeutic plasma drug concentration and then multiply the dividend by the current therapeutic dose amount and then multiply the product by a non-linear index factor, and
            iv. Continuing the process until the lowest therapeutic dose or optimal effective dose is achieved.
26. The method item 25, wherein the therapeutic combination, pharmaceutical dosage form, drug delivery device or product of manufacture comprises Formula I, or a deuterated derivative of Formula I.
27. A method for delivering or administering a triple monoamine reuptake inhibitor (TRI) to an individual in need thereof, wherein the TRI comprises Formula I, or a deuterated derivative of Formula I.
28. The method of item 27, wherein the TRI comprises tesofensine.
29. A method for administering and maintain an effective dose of:
   (i) a triple monoamine reuptake inhibitor (TRI),
   (ii) a melanin concentrating hormone receptor 1 (MCHR1) antagonist,
   (iii) a diazoxide or diazoxide formulation,
   (iv) a therapeutic combination of any of (i) to (iii), or
   (iv) a therapeutic combination as used in a method of any of the preceding items , or a therapeutic combination or as used in any of items 13 to 17, or
   (v) a pharmaceutical composition, drug or formulation as set forth in any of items 10 to 13,
      wherein the dosaging is determined by a method comprising: (a) recording the maintenance dose amount and the maintenance therapeutic plasma drug concentration associated with the lowest effective therapeutic dose or optimal therapeutic dose, and
      (b) at the early of between about 1 to 12 months, 3 to 9 month 4 to 8 months or 6 months or change in body weight, medication or health status then:
      prior to the next scheduled dose, draw a blood sample and assess the current plasma drug concentration,
      and optionally if the current plasma drug concentration is different from the maintenance therapeutic plasma drug concentration by more than about 20%, optionally more than about 50%, then calculate a new therapeutic dose/day by a method comprising:
         i. For drugs with a linear dose to plasma concentration, divide the maintenance therapeutic plasma drug concentration by the current plasma drug concentration, and then and multiple the dividend by the maintenance dose amount, and
         ii. For drugs with a non-linear dose to plasma concentration, divide the maintenance therapeutic plasma drug concentration by the current plasma drug concentration, then multiply the dividend by the maintenance dose amount, and then multiply the product by a non-linear index, or
         iii. optionally, reaching steady state after two subsequent measured plasma drug concentration results at least one week apart and within about 20% of the maintenance therapeutic plasma concentration, or
         iv. optionally, continuing the process until a maintenance therapeutic dose at steady state is achieved.
30. The method of item 29, wherein the TRI comprises Formula I, or a deuterated derivative of Formula I.
31. The method of item 30, wherein the TRI comprises tesofensine.
32. A method for treating hyperphagia, hyperphagia in PWS, Disruptive Mood Dysregulation Disorder (DMDD), Oppositional Defiant Disorder (ODD), obesity in hypothalamic-injury induced obesity, or binge eating disorder (BED) comprising administering to an individual in need thereof Formula I, or a deuterated derivative of Formula I.
33. A method for administering Formula I, or a deuterated derivative of Formula I, for treating hyperphagia, hyperphagia in PWS, Disruptive Mood Dysregulation Disorder (DMDD), Oppositional Defiant Disorder (ODD), obesity in hypothalamic-injury induced obesity, or binge eating disorder (BED).
34. A method for treating hyperphagia, hyperphagia in PWS, Disruptive Mood Dysregulation Disorder (DMDD), Oppositional Defiant Disorder (ODD), obesity in hypothalamic-injury induced obesity, and binge eating disorder (BED) with an oral dosage form of Formula I, or an deuterated derivative of Formula I of the proceeding items , the method comprises administering an oral dosage form that provides trough plasma concentration, a 24-hour time-averaged plasma concentration, or a daytime 12-hour time-averaged plasma concentration between 250 ng/mL to 500 ng/ml, 500 to 1000 ng/ml, 1000 to 1500 ng/ml, 1500 to 2000 ng/ml, or 1500 to 2500 ng/ml of Formula I, or an deuterated derivative of Formula I of the proceeding items , when administered once daily, or twice daily, and measured at about one week, about two weeks, or steady state.

## Claims

1. A melanin concentrating hormone receptor 1 (MCHR1) antagonist or a pharmaceutically acceptable salt thereof for use in a method for treating, ameliorating, slowing the progress of, reducing the symptoms of, reducing adverse events associated with, or preventing, a disease or condition comprising or associated with:
hyperphagia,
management of obesity,
early onset morbid obesity,
hypothalamic injury-induced obesity,
disruptive mood dysregulation disorder (DMDD),
excessive daytime sleepiness (EDS),
excessive daytime sleepiness associated with narcolepsy,
sleep apnea,
excessive daytime sleepiness associated with central or obstructive sleep apnea,
narcolepsy,
narcolepsy type 1 (NT1) according to the International Classification of Sleep Disorders-Third Edition (ICSD-3),
narcolepsy with cataplexy,
narcolepsy type 2 (NT2) according to ICSD-3,
narcolepsy without cataplexy,
cataplexy,
idiopathic hypersomnia,
rapid eye movement (REM) sleep behavior disorder,
addiction,
an addictive disorder or behavior,
major depressive disorder (MDD),
treatment resistant depression (TRD),
Prader-Willi Syndrome,
Smith-Magenis Syndrome,
Angelman's Syndrome, or
Schaff-Yang Syndrome.

2. The MCHR1 antagonist for use according to claim 1, wherein the MCHR1 antagonist is selected from:
(a) a compound having the formula 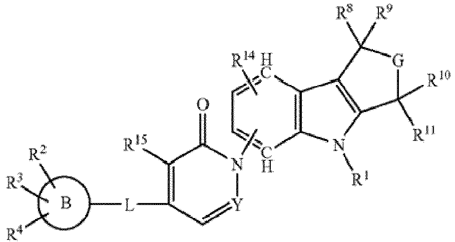 or a pharmaceutically acceptable salt thereof, wherein
R¹ is H or optionally substituted alkyl;
R², R³, and R⁴ are each independently selected from H, -O-alkyl, -S-alkyl, alkyl, halo, -CF₃, and -CN;
G is -CR¹²R¹³-NR⁵- or -NR⁵-CR¹²R¹³;
R⁵ is H, optionally substituted alkyl, optionally substituted heterocycle, -C(=O)-R⁶, -C(=O)-O-R⁷, or -C(=O)-NR¹⁹R²⁰;
R⁶ and R⁷ are each optionally substituted alkyl or optionally substituted heterocycle;
R⁸, R⁹, R¹⁰, R¹¹, R¹², R¹³, R¹⁹ and R²⁰ are each independently selected from H or optionally substituted alkyl;
R¹⁴ and R¹⁵ are each independently H or halogen;
Y is CH;
L is -CH₂-O-, -CH₂CH₂-, -CH=CH-, or a bond; and
B is aryl, heteroaryl, or cycloalkyl; with the proviso that, when L is a direct bond, B cannot be unsubstituted heteroaryl or heteroaryl monosubstituted with fluorine;
(b) the compound of Formula II: 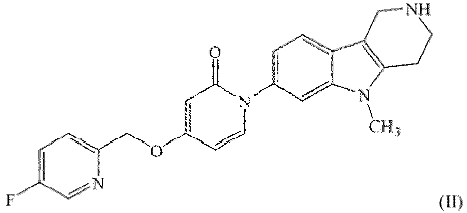 or a pharmaceutically acceptable salt thereof;
(c) a (1-azinone)-substituted pyridoindole as described in USPN 8,716,308;
(d) GW8564649, AZD-1979, AMG-076, BMS-830216, ATC-0065, ATC-0175, GW-803430, GW-3430, NGD-4715, SNAP-7941, T-226, or T-296, or any combination thereof; or
(e) a deuterated form of (a), (b), (c), or (d).

3. The MCHR1 antagonist for use according to claim 1, wherein the MCHR1 antagonist is a compound of structure: 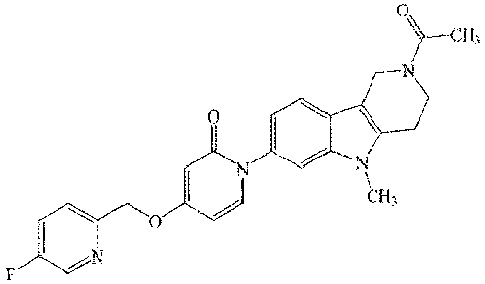 or a pharmaceutically acceptable salt thereof.

4. The MCHR1 antagonist for use according to any one of claims 1 to 3, wherein the method further comprises administering a N-acetyltransferase 2 (NAT2) inhibitor, an arylamine N-acetyltransferase inhibitor, acetaminophen, N-acetyl-para-aminophenol (APAP), or paracetamol (or TYLENOL^{™} or PANADOL^{™}).

5. The MCHR1 antagonist for use according to any one of claims 1, 2, or 4, wherein the MCHR1 antagonist is of formula: 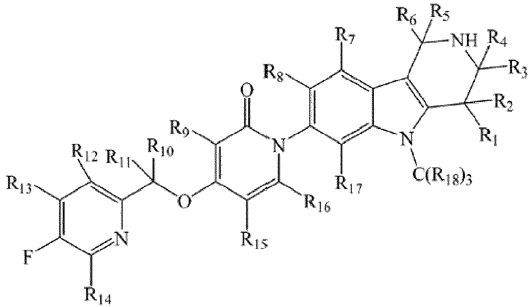 wherein at least one of R¹ through R¹⁸ is -D (deuterium) and the rest of R¹-R¹⁸ are hydrogen, or all of R¹ through R¹⁸ is -D; or a pharmaceutically acceptable salt thereof.

6. The MCHR1 antagonist for use according to any one of claims 1, 2, 4, or 5, wherein the MCHR1 antagonist is : 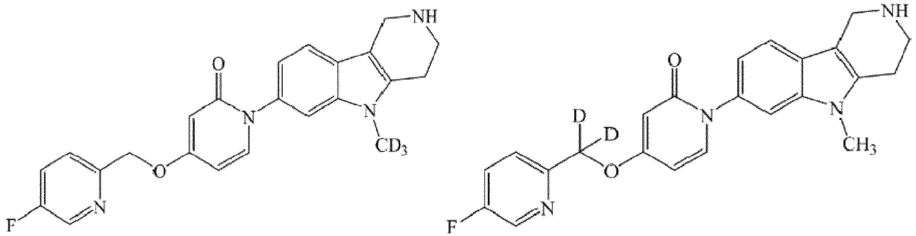 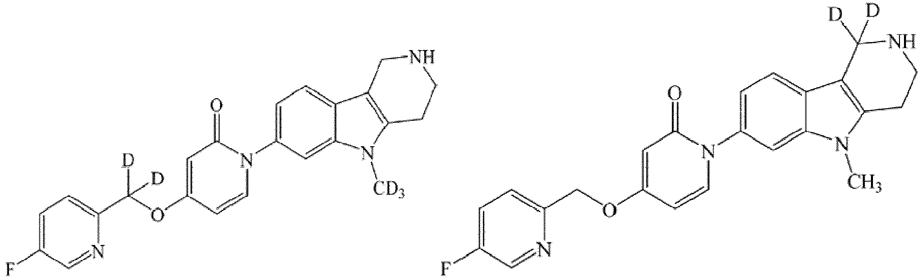 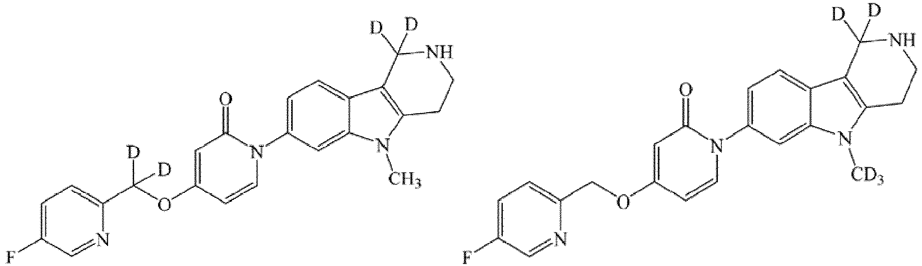 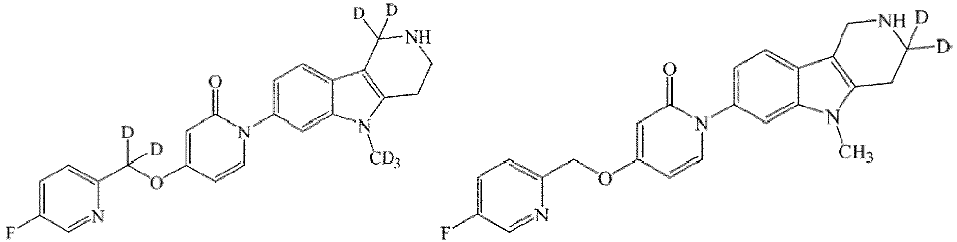 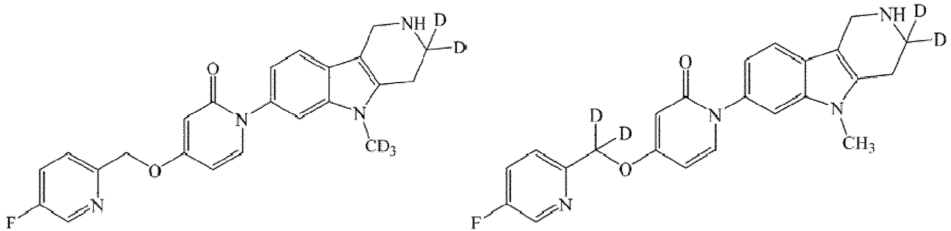 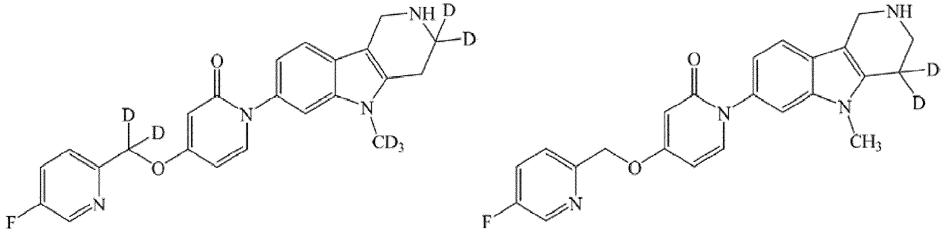 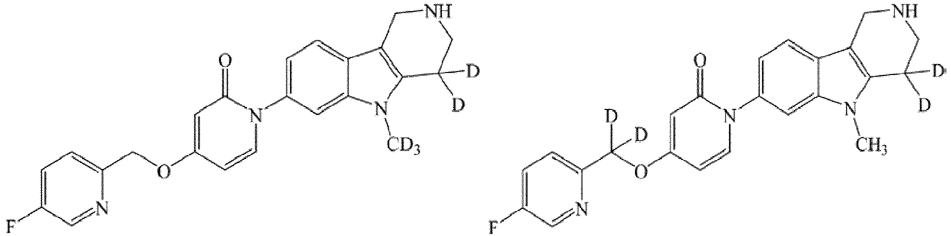 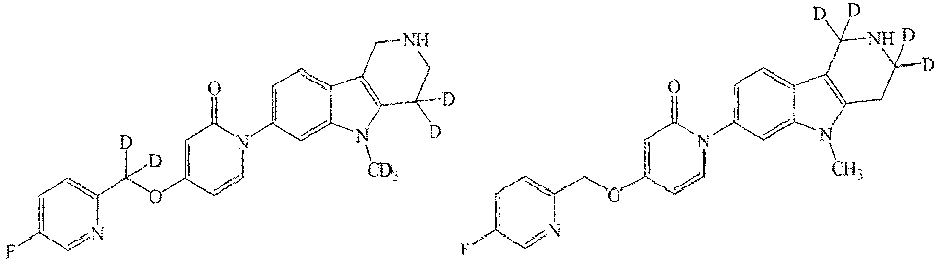 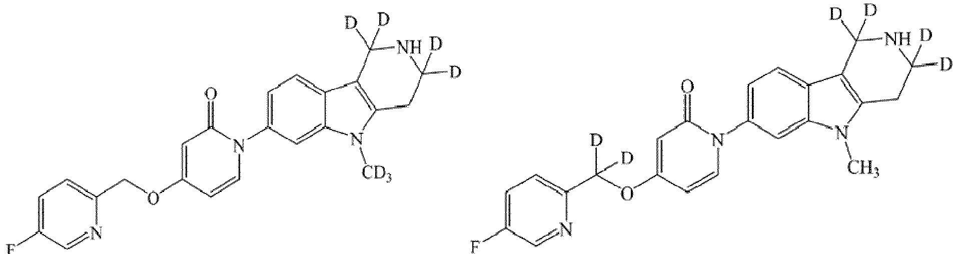 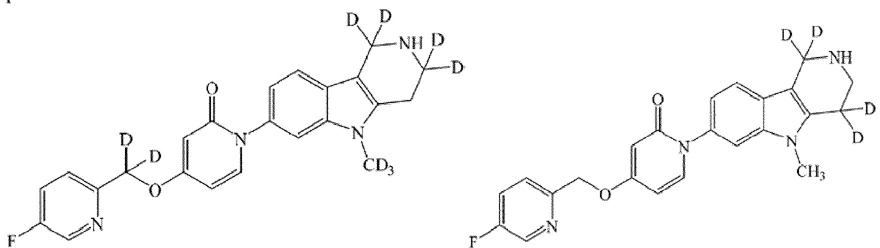 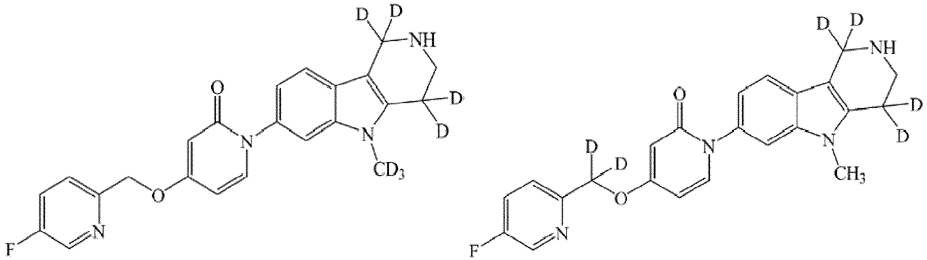 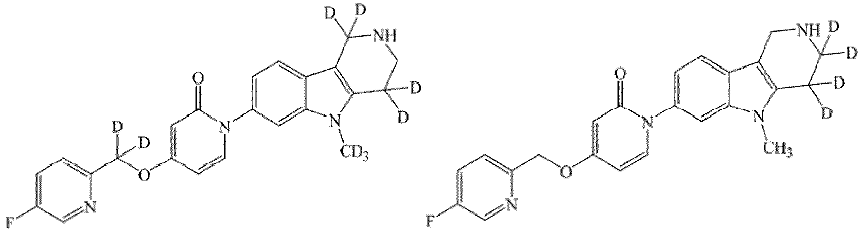 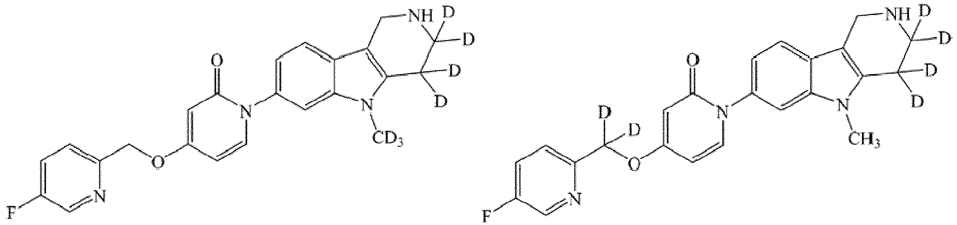 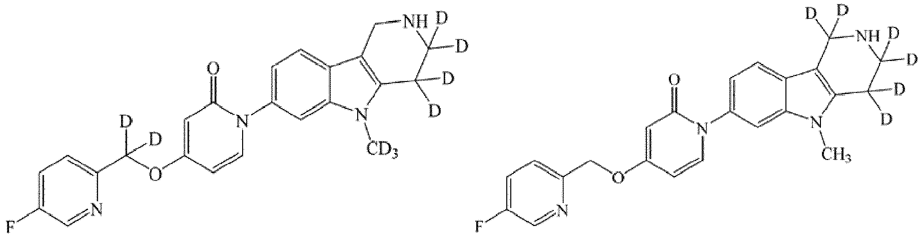 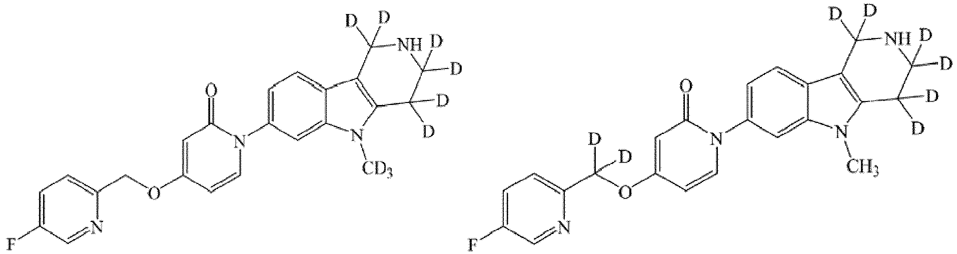 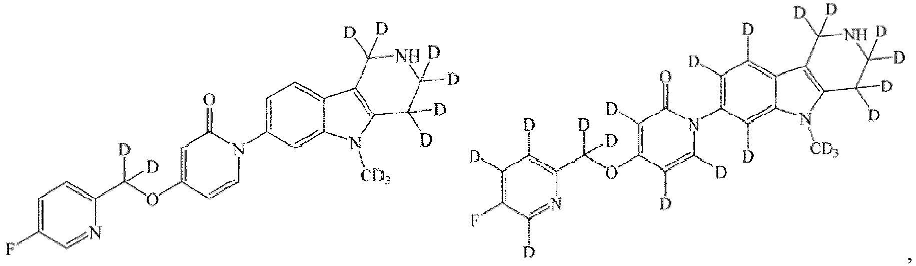 or a pharmaceutically acceptable salt thereof.

7. The MCHR1 antagonist for use according to any one of claims 1 to 6, wherein
the MCHR1 antagonist is administered to achieve a therapeutic level range of plasma concentration at a steady state, and wherein: the therapeutic level range of plasma concentration for the (1-azinone)-substituted pyridoindole, or a compound of Formula II, is between about: 5 to 4000 ng/ml, 10 to 1000 ng/ml, 10 to 500 ng/ml, 25 to 500 ng/ml, 25 to 250 ng/ml, 50 to 500 ng/ml, 50 to 1000 ng/ml, 50 to 500 ng/ml, 100 to 1000 ng/ml, 100 to 500 ng/ml, 200 to 1000 ng/ml, 500 to 1000 ng/ml, 1000 to 2000 ng/ml, 2000 to 3000 ng/ml, or 3000 to 4000 ng/ml.

8. The MCHR1 antagonist for use according to claim 7, wherein the steady state is achieved after:
(a) between about 3 to 5 times the elimination half-life (T1/2) of the active agent(s) in a subject or patient, or
(b) between about 7 to 14 days after periodic or regular administration, optionally once-a-day dosing, of the MCHR1 antagonist.

9. The MCHR1 antagonist for use according to claim 7 or 8, wherein the plasma concentration for the MCHR1 antagonist is:
(a) the trough level or trough concentration (Ctrough), or the lowest concentration reached by the MCHR1 antagonist before a second or next dose is administered, or
(b) determined from blood samples taken between about 2 hours to 24 hours, or 4 to 12 hours, or 1, 2, 3, 4, 5, 6, 7, 8, 9, or 10 or more hours, after the last dose or administration of the MCHR1 antagonist.

10. The MCHR1 antagonist for use according to any one of claims 1, 2, or 4 to 9, wherein the MCHR1 antagonist is Formula II, or a deuterated derivative of Formula II.

11. The MCHR1 antagonist for use according to any one of claims 1 to 10, wherein the disorder is narcolepsy type 1.

12. The MCHR1 antagonist for use according to any one of claims 1 to 11, wherein the disorder is Prader-Willi Syndrome.

13. A compound as defined in claim 5 or 6, or a pharmaceutically acceptable salt thereof.

14. A pharmaceutical composition comprising the compound of claim 13, and a pharmaceutically acceptable carrier.
